# EUROPEAN PATENT SPECIFICATION

(11) **EP 2 471 949 B1**
(45) Date of publication and mention of the grant of the patent: **25.12.2013**
(21) Application number: 10197481.4
(22) Date of filing: 31.12.2010
(51) Int. Cl.: C12Q 1/68

(54) **Method for the identification by molecular techniques of genetic variants that encode no D antigen (D-) and altered C antigen (C+W)**
Verfahren zur Identifizierung durch Molekulartechniken von genetischen Varianten, die kein D-Antigen (D-) und das veränderte C-Antigen (C+W) codieren
Procédé pour l'identification par des techniques moléculaires de variantes génétiques ne codant pas d'antigène D (D-) et qui codent l'antigène C modifié (C+W)

(43) Date of publication of application: 04.07.2012
(73) Proprietor: Progenika Biopharma, S.A., 48160 Derio, Vizcaya (ES)
(72) Inventor: Ochoa, Jorge, 48160 Derio, Vizcaya (ES); Lopez, Monica, 48160 Derio, Vizcaya (ES); Tejedor, Diego, 48160 Derio, Vizcaya (ES); Martinez, Antonio, 48160 Derio, Vizcaya (ES); Simon, Laureano, 48160 Derio, Vizcaya (ES)
(74) Representative: Casley, Christopher Stuart

(56) References cited:
- WO-A2-2006/032897
- WO-A2-2006/075254
- WO-A2-2011/003921
- DE-A1- 10 049 363
- AVENT N D ET AL: "The bloodgen project of the European Union, 2003-2009", TRANSFUSION MEDICINE AND HEMOTHERAPY 2009 S. KARGER AG CHE LNKD- DOI:10.1159/000218192, vol. 36, no. 3, June 2009 (2009-06), pages 162-167, XP002633276, ISSN: 1660-3796
- WESTHOFF CONNIE M ET AL: "DIIIa and DIII Type 5 are encoded by the same allele and are associated with altered RHCE*ce alleles: clinical implications", TRANSFUSION (MALDEN), vol. 50, no. 6, June 2010 (2010-06), pages 1303-1311, XP002633277, ISSN: 0041-1132
- PHAM BACH-NGA ET AL: "Heterogeneous molecular background of the weak C, VS+, hr(B)-, Hr(B)- phenotype in black persons", TRANSFUSION (MALDEN), vol. 49, no. 3, March 2009 (2009-03), pages 495-504, XP002633278, ISSN: 0041-1132
- FAAS B H W ET AL: "Rh E/e genotyping by allele-specific primer amplification", BLOOD, AMERICAN SOCIETY OF HEMATOLOGY, US, vol. 85, no. 3, 1 January 1995 (1995-01-01), pages 829-832, XP002614101, ISSN: 0006-4971
- MAASKANT-VAN WIJK P A ET AL: "GENOTYPING OR RHD BY MULTIPLEX POLYMERASE CHAIN REACTIONS ANALYSIS OF SIX RHD-SPECIFIC EXONS", TRANSFUSION, AMERICAN ASSOCIATION OF BLOOD BANKS, BETHESDA, MD, US, vol. 11, no. 38, 1 November 1998 (1998-11-01), pages 1015-1021, XP008005129, ISSN: 0041-1132, DOI: DOI:10.1046/J.1537-2995.1998.3811129905630 9.X

### SEQUENCE LISTING

<110> Progenika Biopharma, S.A.
<120> Method for the Identification by Molecular Techniques of Genetic Variants that Encode no D Antigen (D-) and Altered C Antigen (C+W)
<130> CSC/FP6712525
<160> 42
<170> PatentIn version 3.3
<210> 1
   <211> 1254
   <212> DNA
   <213> Homo sapiens
<400> 1
   atgagctcta agtacccgcg gtctgtccgg cgctgcctgc ccctctgggc cctaacactg      60
   gaagcagctc tcattctcct cttctatttt tttacccact atgacgcttc cttagaggat     120
   caaaaggggc tcgtggcatc ctatcaagtt ggccaagatc tgaccgtgat ggcggccatt     180
   ggcttgggct tcctcacctc gagtttccgg agacacagct ggagcagtgt ggccttcaac     240
   ctcttcatgc tggcgcttgg tgtgcagtgg gcaatcctgc tggacggctt cctgagccag     300
   ttcccttctg ggaaggtggt catcacactg ttcagtattc ggctggccac catgagtgct     360
   ttgtcggtgc tgatctcagt ggatgctgtc ttggggaagg tcaacttggc gcagttggtg     420
   gtgatggtgc tggtggaggt gacagcttta ggcaacctga ggatggtcat cagtaatatc     480
   ttcaacacag actaccacat gaacatgatg cacatctacg tgttcgcagc ctattttggg     540
   ctgtctgtgg cctggtgcct gccaaagcct ctacccgagg gaacggagga taaagatcag     600
   acagcaacga tacccagttt gtctgccatg ctgggcgccc tcttcttgtg gatgttctgg     660
   ccaagtttca actctgctct gctgagaagt ccaatcgaaa ggaagaatgc cgtgttcaac     720
   acctactatg ctgtagcagt cagcgtggtg acagccatct cagggtcatc cttggctcac     780
   ccccaaggga agatcagcaa gacttatgtg cacagtgcgg tgttggcagg aggcgtggct     840
   gtgggtacct cgtgtcacct gatcccttct ccgtggcttg ccatggtgct gggtcttgtg     900
   gctgggctga tctccgtcgg gggagccaag tacctgccgg ggtgttgtaa ccgagtgctg     960
   gggattcccc acagctccat catgggctac aacttcagct tgctgggtct gcttggagag    1020
   atcatctaca ttgtgctgct ggtgcttgat accgtcggag ccggcaatgg catgattggc    1080
   ttccaggtcc tcctcagcat tggggaactc agcttggcca tcgtgatagc tctcacgtct    1140
   ggtctcctga caggtttgct cctaaatctt aaaatatgga aagcacctca tgaggctaaa    1200
   tattttgatg accaagtttt ctggaagttt cctcatttgg ctgttggatt ttaa          1254
<210> 2
   <211> 1254
   <212> DNA
   <213> Homo sapiens
<400> 2
   atgagctcta agtacccgcg gtctgtccgg cgctgcctgc ccctctgggc cctaacactg     60
   gaagcagctc tcattctcct cttctatttt tttacccact atgacgcttc cttagaggat    120
   caaaaggggc tcgtggcatc ctatcaagtc ggccaagatc tgaccgtgat ggcggccctt    180
   ggcttgggct tcctcacctc aaatttccgg agacacagct ggagcagtgt ggccttcaac    240
   ctcttcatgc tggcgcttgg tgtgcagtgg gcaatcctgc tggacggctt cctgagccag    300
   ttccctcctg ggaaggtggt catcacactg ttcagtattc ggctggccac catgagtgct    360
   atgtcggtgc tgatctcagc gggtgctgtc ttggggaagg tcaacttggc gcagttggtg    420
   gtgatggtgc tggtggaggt gacagcttta ggcaccctga ggatggtcat cagtaatatc    480
   ttcaacacag actaccacat gaacctgagg cacttctacg tgttcgcagc ctattttggg    540
   ctgactgtgg cctggtgcct gccaaagcct ctacccaagg gaacggagga taatgatcag    600
   agagcaacga tacccagttt gtctgccatg ctgggcgccc tcttcttgtg gatgttctgg    660
   ccaagtgtca actctgctct gctgagaagt ccaatccaaa ggaagaatgc catgttcaac    720
   acctactatg ctctagcagt cagtgtggtg acagccatct cagggtcatc cttggctcac    780
   ccccaaagga agatcagcat gacttatgtg cacagtgcgg tgttggcagg aggcgtggct    840
   gtgggtacct cgtgtcacct gatcccttct ccgtggcttg ccatggtgct gggtcttgtg    900
   gctgggctga tctccatcgg gggagccaag tgcctgccgg tgtgttgtaa ccgagtgctg    960
   gggattcacc acatctccgt catgcactcc atcttcagct tgctgggtct gcttggagag   1020
   atcacctaca ttgtgctgct ggtgcttcat actgtctgga acggcaatgg catgattggc   1080
   ttccaggtcc tcctcagcat tggggaactc agcttggcca tcgtgatagc tctcacgtct   1140
   ggtctcctga caggtttgct cctaaatctc aaaatatgga aagcacctca tgtggctaaa   1200
   tattttgatg accaagtttt ctggaagttt cctcatttgg ctgttggatt ttaa          1254
<210> 3
   <211> 17
   <212> DNA
   <213> Artificial sequence
<220>
   <221> source
   <222> 1..17
   <223> /note= "Description of artificial sequence: Primer"
<400> 3
   ggccaccacc atttgaa 17
<210> 4
   <211> 20
   <212> DNA
   <213> Artificial sequence
<220>
   <221> source
   <222> 1..20
   <223> /note= "Description of artificial sequence: Primer"
<400> 4
   ccatgaacat gccacttcac 20
<210> 5
   <211> 23
   <212> DNA
   <213> Artificial sequence
<220>
   <221> source
   <222> 1..23
   <223> /note= "Description of artificial sequence: Probe"
<400> 5
   ttttacagac gcctgctacc atg 23
<210> 6
   <211> 23
   <212> DNA
   <213> Artificial sequence
<220>
   <221> source
   <222> 1..23
   <223> /note= "Description of artificial sequence: Probe"
<400> 6
   catggtagca ggcgtctgta aaa 23
<210> 7
   <211> 23
   <212> DNA
   <213> Artificial sequence
<220>
   <221> source
   <222> 1..23
   <223> /note= "Description of artificial sequence: Probe"
<400> 7
   ttttacagac gtctgctacc atg 23
<210> 8
   <211> 23
   <212> DNA
   <213> Artificial sequence
<220>
   <221> source
   <222> 1..23
   <223> /note= "Description of artificial sequence: Probe"
<400> 8
   catggtagca gacgtctgta aaa 23
<210> 9
   <211> 18
   <212> DNA
   <213> Artificial sequence
<220>
   <221> source
   <222> 1..18
   <223> /note= "Description of artificial sequence: Primer"
<400> 9
   tcctggctct ccctctct 18
<210> 10
   <211> 18
   <212> DNA
   <213> Artificial sequence
<220>
   <221> source
   <222> 1..18
   <223> /note= "Description of artificial sequence: Primer"
<400> 10
   ttttcaaaac cccggaag 18
<210> 11
   <211> 23
   <212> DNA
   <213> Artificial sequence
<220>
   <221> source
   <222> 1..23
   <223> /note= "Description of artificial sequence: Probe"
<400> 11
   ggtcaacttg gcgcagttgg tgg 23
<210> 12
   <211> 21
   <212> DNA
   <213> Artificial sequence
<220>
   <221> source
   <222> 1..21
   <223> /note= "Description of artificial sequence: Probe"
<400> 12
   gtcaacttgg cgcagttggt g 21
<210> 13
   <211> 23
   <212> DNA
   <213> Artificial sequence
<220>
   <221> source
   <222> 1..23
   <223> /note= "Description of artificial sequence: Probe"
<400> 13
   ggtcaacttg gtgcagttgg tgg 23
<210> 14
   <211> 21
   <212> DNA
   <213> Artificial sequence
<220>
   <221> source
   <222> 1..21
   <223> /note= "Description of artificial sequence: Probe"
<400> 14
   gtcaacttgg tgcagttggt g 21
<210> 15
   <211> 18
   <212> DNA
   <213> Artificial sequence
<220>
   <221> source
   <222> 1..18
   <223> /note= "Description of artificial sequence: Primer"
<400> 15
   tcctggctct ccctctct 18
<210> 16
   <211> 24
   <212> DNA
   <213> Artificial sequence
<220>
   <221> source
   <222> 1..24
   <223> /note= "Description of artificial sequence: Primer"
<400> 16
   gttgtcttta tttttcaaaa ccct 24
<210> 17
   <211> 23
   <212> DNA
   <213> Artificial sequence
<220>
   <221> source
   <222> 1..23
   <223> /note= "Description of artificial sequence: Primer"
<400> 17
   gctctgaact ttctccaagg act 23
<210> 18
   <211> 20
   <212> DNA
   <213> Artificial sequence
<220>
   <221> source
   <222> 1..20
   <223> /note= "Description of artificial sequence: Primer"
<400> 18
   attctgctca gcccaagtag 20
<210> 19
   <211> 24
   <212> DNA
   <213> Artificial sequence
<220>
   <221> source
   <222> 1..24
   <223> /note= "Description of artificial sequence: Primer"
<400> 19
   ttgaattaag cacttcacag agca 24
<210> 20
   <211> 18
   <212> DNA
   <213> Artificial sequence
<220>
   <221> source
   <222> 1..18
   <223> /note= "Description of artificial sequence: Primer"
<400> 20
   caccttgctg atcttccc 18
<210> 21
   <211> 20
   <212> DNA
   <213> Artificial sequence
<220>
   <221> source
   <222> 1..20
   <223> /note= "Description of artificial sequence: Primer"
<400> 21
   agtagtgagc tggcccatca 20
<210> 22
   <211> 20
   <212> DNA
   <213> Artificial sequence
<220>
   <221> source
   <222> 1..20
   <223> /note= "Description of artificial sequence: Primer"
<400> 22
   cttcagccaa agcagaggag 20
<210> 23
   <211> 25
   <212> DNA
   <213> Artificial sequence
<220>
   <221> source
   <222> 1..25
   <223> /note= "Description of artificial sequence: Probe"
<400> 23
   ataaagatca gacagcaacg atacc 25
<210> 24
   <211> 23
   <212> DNA
   <213> Artificial sequence
<220>
   <221> source
   <222> 1..23
   <223> /note= "Description of artificial sequence: Probe"
<400> 24
   taaagatcag acagcaacga tac 23
<210> 25
   <211> 25
   <212> DNA
   <213> Artificial sequence
<220>
   <221> source
   <222> 1..25
   <223> /note= "Description of artificial sequence: Probe"
<400> 25
   ataaagatca gagagcaacg atacc 25
<210> 26
   <211> 23
   <212> DNA
   <213> Artificial sequence
<220>
   <221> source
   <222> 1..23
   <223> /note= "Description of artificial sequence: Probe"
<400> 26
   taaagatcag agagcaacga tac 23
<210> 27
   <211> 21
   <212> DNA
   <213> Artificial sequence
<220>
   <221> source
   <222> 1..21
   <223> /note= "Description of artificial sequence: Probe"
<400> 27
   ctggccaagt ttcaactctg c 21
<210> 28
   <211> 19
   <212> DNA
   <213> Artificial sequence
<220>
   <221> source
   <222> 1..19
   <223> /note= "Description of artificial sequence: Probe"
<400> 28
   tggccaagtt tcaactctg 19
<210> 29
   <211> 21
   <212> DNA
   <213> Artificial sequence
<220>
   <221> source
   <222> 1..21
   <223> /note= "Description of artificial sequence: Probe"
<400> 29
   ctggccaagt gtcaactctg c 21
<210> 30
   <211> 19
   <212> DNA
   <213> Artificial sequence
<220>
   <221> source
   <222> 1..19
   <223> /note= "Description of artificial sequence: Probe"
<400> 30
   tggccaagtg tcaactctg 19
<210> 31
   <211> 23
   <212> DNA
   <213> Artificial sequence
<220>
   <221> source
   <222> 1..23
   <223> /note= "Description of artificial sequence: Probe"
<400> 31
   gtgcacagtg cggtgttggc agg 23
<210> 32
   <211> 21
   <212> DNA
   <213> Artificial sequence
<220>
   <221> source
   <222> 1..21
   <223> /note= "Description of artificial sequence: Probe"
<400> 32
   tgcacagtgc ggtgttggca g 21
<210> 33
   <211> 23
   <212> DNA
   <213> Artificial sequence
<220>
   <221> source
   <222> 1..23
   <223> /note= "Description of artificial sequence: Probe"
<400> 33
   gtgcacagtg cagtgttggc agg 23
<210> 34
   <211> 21
   <212> DNA
   <213> Artificial sequence
<220>
   <221> source
   <222> 1..21
   <223> /note= "Description of artificial sequence: Probe"
<400> 34
   tgcacagtgc agtgttggca g 21
<210> 35
   <211> 24
   <212> DNA
   <213> Artificial sequence
<220>
   <221> source
   <222> 1..24
   <223> /note= "Description of artificial sequence: Primer"
<400> 35
   acaaactccc cgatgatgtg agtg 24
<210> 36
   <211> 22
   <212> DNA
   <213> Artificial sequence
<220>
   <221> source
   <222> 1..22
   <223> /note= "Description of artificial sequence: Primer"
<400> 36
   gaggctgaga aaggttaagc ca 22
<210> 37
   <211> 23
   <212> DNA
   <213> Artificial sequence
<220>
   <221> source
   <222> 1..23
   <223> /note= "Description of artificial sequence: Probe"
<400> 37
   tgctggtgct tgataccgtc gga 23
<210> 38
   <211> 21
   <212> DNA
   <213> Artificial sequence
<220>
   <221> source
   <222> 1..21
   <223> /note= "Description of artificial sequence: Probe"
<400> 38
   gctggtgctt gataccgtcg g 21
<210> 39
   <211> 23
   <212> DNA
   <213> Artificial sequence
<220>
   <221> source
   <222> 1..23
   <223> /note= "Description of artificial sequence: Probe"
<400> 39
   tgctggtgct tcataccgtc gga 23
<210> 40
   <211> 21
   <212> DNA
   <213> Artificial sequence
<220>
   <221> source
   <222> 1..21
   <223> /note= "Description of artificial sequence: Probe"
<400> 40
   gctggtgctt cataccgtcg g 21
<210> 41
   <211> 64956
   <212> DNA
   <213> Homo sapiens
<400> 41
<210> 42
   <211> 65624
   <212> DNA
   <213> Homo sapiens
<400> 42
   acccttggcg tggacacatt tccagggagg gaccggagga cctcctacct cattggtcac 60
   tgccagtgac tgagcttgac tcaggtagga gggcatggca ggtattctca gggagtctgg 120
   tgtttacaga aaagtcatga ttacacgtga aagctgtggg ctccctggct tgattcacca 180
   cacctgcagg aagcctggct gctcagacca gcacgccgtg gacatagcac cacttgctca 240
   gcttcatttc cgtaactcag gctgccaggc ctgctgacaa attttcacgt ttgtaataac 300
   cctgtgagga gaccagagta catcttactt gactcataag gaaattgaga ctgggtgatt 360
   tagtaacttg ggaggcagaa ttgcaaagtg attagcaaca caagccatgg tgtcagatgg 420
   atctgggtta ggtcccacct ctgccgttta ttagctgtgt ggctttgggt actcacgcca 480
   cctctctgag cagcagtttc ctcttttgta agcgtaatga tgcctacact cacaggcttg 540
   agaggaagat ccgatgaaat agcatatgca aaatgattgg ttccgtgctt ggcattccag 600
   aaatggtagc tgttattcag ccaacaaata tttattgagc acctactatg gacttccctg 660
   gtgctgagga tacaacagca accacagcag tcaaaagtcc ctgtctttat gttgctcaga 720
   ttctcatagg ggaaagcaga taatgaacaa atacacggcc agacgcagtg gctcacgcct 780
   gtaatcccag tactttgcga ggccaaggtg ggcaagtcac ctgaggtcag gagttcgaga 840
   ccagcctagc caacatggtg aaaccctgtc actactaaaa atacaaaaat tagcgcagtg 900
   tggtggctca tgcctgtagt cccagctact tgggaggctg aggaaggaga atcgcttgaa 960
   cctaaaaggc agaagttgca atgagccaag atcgtgccac tgcattccag cctgggtgac 1020
   agagtactcc atctaaaaaa aaaacctaaa tacacaagta aaaatataga cttcgtcaga 1080
   tgctagtaag tgctgtgaag gaaactaaaa ggggaacaca aggaaccctt gtcaagggga 1140
   gcagaaaggg gagttgatgc tgtcctttta aatagggcaa tcagaggcca ggcacagtgg 1200
   ctcacactta taatcccagc actttgggag ttcgaggcag gtggatcact tgaggtcagg 1260
   agttcaagac cagccaggcc aatgtggtga aaccctgtct ctactaaaac tacaaaaact 1320
   agccaggtgt ggtatcgcgt gcctataatc ccagctactc gggaggctga ggcgggagaa 1380
   tcgcttgaac ctgggaggcg gaggttgcag tgagccgaga ttgtgccatt gcagtccagc 1440
   ctgggcaaca agagcaaaac ttcatctaaa aaaaaaacac agcaaaaaag ggcagtcagg 1500
   gaaaacttcc ctgagaaggg gatggtggag tacagatcca gggaggtgag gtggggagca 1560
   agccagtaca gttgttcctt gactttcgat gaggttatgt cctgataaag ccatggtaag 1620
   taggaaatat tgtaagtcaa aaatgcattt aatacaccta acctacggaa catcatagct 1680
   tagtgtcacc taccttaaac atgcttagaa cgcttacatt agcctacggt tgggcaaaat 1740
   catctaacac aaagcctatt ttatgataaa gtattgaata tctcatgtaa tgtactgagt 1800
   actgtacgga aagtgaaaga cggagtggtg ggatgggaac tctaagcacg gcttccactg 1860
   catgtgtgtt gctttcgcgc catcataaag ttgaaaagcg ttaagtcaaa ccaccgtacg 1920
   tcggaggcca tctgtatctg gtaggaggag tgtttcagac agagagaaca gcaggtgcaa 1980
   tagagtgctt ttttcccagc attttattat gaaaaatttc aaacatctac caaaaaaagt 2040
   tgaaagactt gtacggtgaa aagccataca tctcacagct agaatcaaca attaacattt 2100
   tactgtattt ggtttttgac ttatctatcc tagatccctt gtgctttctg tagcaggtga 2160
   cctgccttga agatttaaag acagaatatc gggaaatgta gtcagaaaat ggggcctttt 2220
   ataagagtca gaggggaaga gcaaaaactc tgctttcgag aaatctgtcg ggagaggcca 2280
   actgcaggga tacctccctt ttttaatgaa agcatttctg ttctgcgagg agcgggatcc 2340
   tcttgtcaag cagtcagtcc ctgctgcttc cttactgggg caggatcagg acgcacaggg 2400
   atttggagtg ccttggaacc aaccaccacc cacgctgttt gccagctggt aaacatgcct 2460
   gtcaggtcta ggggttggca ttgcctggaa atctttagtg ttcatcttgc tgacatctgg 2520
   tgccctcggg taggtaggtg cagttggctg cctggtttac agagcttgta ctgggcccag 2580
   gttagcaggg gtcacatccc tttatcccac tgtgcagggg agttccttct caggaaaccc 2640
   agtttataag aagtactgac tgccagaaat agagcagaga tcagaaccag gaggcaattg 2700
   tgagaggaat ggagacttct gacctctggg gattggggta ccctccccct taattgctgt 2760
   tggggtagca gagggcttag aagcccatgt tcctagactt ttagaattgg aagaagactt 2820
   agaagtaatc taggctgggg gtccccaacc cccaggctgt ggcccgttag gaacctgacc 2880
   gcacagcatg aggggtaggc cagcgagcac taccgcctga gctccgcctc ctgtcagatc 2940
   agcagcggca ttagattctc acaggggcac aaaccctatt gggaaccgcg catgagaggg 3000
   atctaggttg cgtgctcctt aggagaatct aactaatgcc tgatgatctg aggtggaaca 3060
   gtttcatccc cacaccatcc ctccaacctc accccggtcc atggaaaaat tgtcttctac 3120
   aaaacccgtc cctggtgcca aataggttgg ggacccctga tctaggctac agttaagtgg 3180
   tcaaacaccc aggtcctgaa gttaggctgc ctgggtttaa atcccagctc tactgcttac 3240
   tagccctgtg accttgagca agtcacttag tttttctgtg cctcagttta ctcatttgta 3300
   ataaaagctt aatagtaccc atcccagtgt catgaactaa gttcatatat gtaaagtgct 3360
   tagaatggtg cctagcaagt acttagtaac agttagctct gaaaatgtat aaagcaaaat 3420
   taaccaatgt tttagtggtt tgcagccaac ttttttctat gcgtgtgcta acatattatt 3480
   ttataagagt gggaatatat tgtacatgct gttatataac ttgctttttc actaaacagt 3540
   ctatcctctg tgtcagtttt gataaaagcg ttttcctctt gcttttcctg catatgttca 3600
   gaaccatcat attggtagca agtttcatgt cctgcagttt tcttaaccaa ccccctgcta 3660
   gcggacattt aggttagtct cagttttttc cttctgtaaa taaagctgca ctgagcaaga 3720
   agtgaccgat gccaagtgac tagatgacct taggtatgac ctctctgggt cttggtttct 3780
   tggtctaaaa acaaaatgac aggattcgac tgggtgatta aaatctcctc tgatctacat 3840
   aggaattgtt ttcaagacat ttctgcattc ctctagtgac agggtgctca ctacctcatg 3900
   agtatttcag tggacaactg taatggtcaa taaagtatcc actttccacc ttccacttcc 3960
   ctgtagctcc tggccctggc tttattctct ggggctccac acattcagtt tacactcagt 4020
   ggccagtggc tggggccatt gtagaaaatg aggaaactcc aattccttcc ttcttttctt 4080
   cctctttcat cccttcctcc ctccctacat ccctctctct cttccttcct tccttgacac 4140
   ttaccatgta ccagaccttc tgccaggcac atggatggga gcacagttcc gggaagttgg 4200
   ctgcagggtt agaactaagt cccaagcccc gtaaagctca tgccagggga ctggactgtc 4260
   cagtactgag ggatggggat gctgaggctg gtggccttcc tcagatgcac tgtagtgccc 4320
   caggcagagt cctgggctgc cctgtgagga ggtgaccaga ggtagagcaa cttcacccta 4380
   aggctggatc aggatcccct ccaggttttt actagagcca aacccacatc tcctttctct 4440
   tctgccaccc ccccttaaaa tgcttagaaa cacatagatt taaatacaag ttcaaatgta 4500
   agtaatttca actgtgtaac tatgaggagt caattctacg tgggtcctat ctgtatcctc 4560
   cccagggctc agctccattc tttgctttca ttcattctca ttcaatacat tgttgttaag 4620
   agctcactgg gtgccctctc tgtcatgtag taaggtttta aaaagaaagc ctcttctgag 4680
   cttcagtttc cttatttata aaataggagt attgatccgt tccttgcttt tcttacaagg 4740
   atatgctgaa gatgactgaa gtacagagta aagaaggatt atgtttgggt gtcaaaggaa 4800
   tagaatgccc tctttcaaac tgagcacagc aggaacctgt aacaggaaca cagcaacttg 4860
   ttgaatgaat gacaatattg gaaaacatac atttcctccc ctccccatca tagtccctct 4920
   gcttccgtgt taactccata gacaggccag cacagccagc cttgcagcct gagataaggc 4980
   ctttggcggg tgtctcccct atcgctccct caagccctca agtaggtgtt ggagagaggg 5040
   gtgatgcctg gtgctggtgg aacccctgca cagagacgga cacaggatga gctctaagta 5100
   cccgcggtct gtccggcgct gcctgcccct ctgggcccta acactggaag cagctctcat 5160
   tctcctcttc tattttttta cccactatga cgcttcctta gaggatcaaa aggggctcgt 5220
   ggcatcctat caaggtgaga gttcattgga acagtggtca caggagcaaa tagcaggggc 5280
   aggggcgggg gaggcctatg gttctccagg ggcacagatg ttcctttcta caaaatcccg 5340
   aggaaaagat tcccccatct tcttccgtag attgcaccga aattcagtca acaatgtaag 5400
   ctttccttta gaagcagcct gggcatgccc tcttctgtga agcctgcctt gatttttcag 5460
   cacagtgaga ggcatcctct ttggtgttcc tcaaattccc tctaccaaat ggtcttcata 5520
   attctctgct tctctgcttc cccttctctc tccttagtgg caaggatttt ttttattttt 5580
   atagatttag gggatacaag tgcagctagc ttatgcaagc aatttcatgt tgttgggttt 5640
   tcgggttttg tttccttttt gtggcctctc gctcatttct tatttctttt tgagacaggg 5700
   tctcactctg ttgcccaggc tgaagtgcag tggcatgatc atggttcact gcagccttga 5760
   cctcctagtc tcaagcaatc ttcccacctc agcctcccaa gaagctggga ccacaggagg 5820
   gcaccaccat gcctggctaa attttttttt ttttttggta gagatgtggg tctccctgtg 5880
   tttcccagac tggtctcaaa ctcctggaca caagcgatcc tccagcctca gtctcccaaa 5940
   gtgctggaat tacaggcgtg aagcactgtg cccagctctc ttgctcatat ctatactagt 6000
   tttcttttgg aagcttcagc ctgttgctac cccccacccc cacccccacc gaccccagct 6060
   ttcttctcac ttaggggctg ggaagtctgc atgctgtcta taaatccaga accagaaggt 6120
   atggctgaag gggagggtag gatgatggtt attttatatt cagctaaaaa tattcccaga 6180
   ctgtgatgag acaactgtaa ataagacaga tgtccacaat ggtgtgactt tgctttttta 6240
   aaaatattga aatgagtttc aggcatctca gtgggctgat aggttgttga taatggacag 6300
   ggcctccttg aagaatgtcc ctgagacaaa gttgaagctt gagcctggtt gagtgcttgc 6360
   ttgttcctag gttgatatga acggctagtt aactggaagc aaagagaagt catcctgggg 6420
   gccatggcag tgacaagtag gacttaggga gggaagccct tataccattt aaggtgctgg 6480
   cccagagagg agccttcagt gacagacaaa caagagctgg cacaatttta attcatttca 6540
   atttacttta attcatttca atccaataca attcaatgca ttccattcat tcaaccatgt 6600
   atgacatcca atgtgggatc cagacacatg atgattagaa ctgatattta tgagcactta 6660
   ctatgtacca ggcactattc tacatgcttt acattgaacc ctcacaataa cccaatgagg 6720
   tgggtactat tatgatcttc gtttttcata tgaggaaact aggcatatgg atgttgagta 6780
   atttgcccaa ggtcgctcag ctagcaatag cacagcgtat ttaaatttag ccaccctgga 6840
   tttagtttcc ttacacttaa ccattatgca tcatggcccc attttacagt ggcgttgagt 6900
   catttgtcat ataacccagt aggtgtagca gccactattc caaccctgta gattgactct 6960
   agggtccatg ttctttaccc ctgcaccgtg ctactaacgt aggtacaaaa tgtcctcaga 7020
   aactcacttt atatggaagc tcagaggagg gtccacaacc caggcagggg agacgatggt 7080
   gtcaggggag gcttctggag ggaggtgcct gcccagccag ctcttgaagg ctcagtagga 7140
   attacctgtg ggacaaaggc gggtcatcca agtgagggca cagtgggtgc cattgcgtgt 7200
   gcacacacta gagcagactg agcttgggct taacattgca ttgccctgta gcctaaaaag 7260
   agaagcaagg ggctgggcga ggtagctgac acctgtaatc ccagcacttt gggaggccaa 7320
   ggctggagaa tcacctgagg ttaggagttc aagaccagcc tggccaacat ggcaaaaccc 7380
   catctctact aaaattataa aaactagccg ggtgtggtgg cacacgtctg taatcccagc 7440
   tacttgggag gccattacac tccagcctgg gcgacagagc aagacttcat ctcaaaaaac 7500
   caaacaaaaa caacaacaac aacaaaaaac aaagaggaga gcagggactg ggtgtggtgg 7560
   ctcatgcctg taatcccaaa cactttggga ggccaaggcg ggcagatcac ctgaggtcag 7620
   gagttcgaga ccagcctggc ccatatggtg aaaccctgtc tctactaaaa atacaaaaat 7680
   tagccggatg tggtggcacg tgcctgtagt cccagctgct tgggaagctg agggaggaga 7740
   attgcttgaa cccaggaggt agaggtagct gagctgagaa tacgccactg cactccagcc 7800
   tgggtgacag agtgggactc tgtctgaaaa aaataatagt aataaataaa aataaacagg 7860
   gaagcagtgg gtggtagact cactgggctg catacggagt ttggcttcag tctgaggtcc 7920
   gaatagtaaa caggagcgcg acaagtttgg gtttgggtca tggcggatgc catgccaggg 7980
   ctggtgttgg gcacagggga aggggcatgg cttgagacac aagaccagcg tggaggctgt 8040
   agtgtagtat tgacccgagg gcttcaacct tctgatggtg tacacaccat tttttgagca 8100
   tgtaccatgg ttatatgtta cactttaagt attactacat taatatattt tgtatgttat 8160
   aataaataca tacaaattag gaaaattgaa agagatcaga atgaaatata taatattttc 8220
   aaattactaa tcataatggt gtcaatctcc aggcagggtc cattgctaca gttgacgata 8280
   gtggatgaaa attcactcct cagagtcttc ttgataattt gaaattgtct tgattgactt 8340
   gtcagatctg attagatcga cattttttaa atctcgaatg tgactgacag cttgtacaag 8400
   gagaagtttc actctgcctt tcctttttgt tcacttgact gccattattt ctctgcttcc 8460
   aatctgtgtt tttctgcacg agttggttaa gccattactt cattttgtga aagtttgttg 8520
   agttaaactt aggtaactta atctgtcaat ccacttaatt gaattcagtc ctggtaaact 8580
   ataatagatt attcaaacct gccaattcta aaaagacatt ttgagacaat caggaaatct 8640
   gaatatagca tgaatatctt acgatataca aggattattg ttaattttgt taggtatgat 8700
   aaaagcatgg tgggtttttt ttttgttttt gttttttaag gctctatctg ttagagaggc 8760
   acattgaaat ggcatgatat ctggggtttg ctttcatacc agaaaaaaga aaaagtagag 8820
   aaggattata gaaacaagat tggtctcatg tgacaatcat cagagtttgg agatgggcac 8880
   gtagggtcat cgtgctgttc tctctgtttt catatatgct ttgaaagttc tgtaatagtt 8940
   aattaaaaaa aaaaaaaaca ccctggctga gcacttaggg aggccaagtg gggaggattg 9000
   cttaaaccaa gaagttcaag accagcctag gaaacatagg gagacccccc cccgccatct 9060
   ctaaaaaaaa aaaaaaaaaa ctgtaaaatt taacccagtg tggtggcaca tgcctgtagt 9120
   cccagctact cagtaggctg aggtgagagg cttgcttgag cctgggagct tgaggctgca 9180
   gtgggacggg attgtaccac ttcactccag catgggcgac agagcaagac cctgtctcaa 9240
   aaaaaaatga aaatatttga ggtgaagcga gactgtaata acaaatttaa aaatataaat 9300
   aaaacataaa ggctgggtgc ggtggctcac gcctgtaatc ccagcacttt gggaggccaa 9360
   ggcaggcaga tcacgaggtc tggagatgga gaccatcctg gctaacatga tgaaacccca 9420
   tctctactaa aaatacaaaa aattagctgg gtatggtggc gggtgcctgt agtcccagct 9480
   acttgggagg ctgaggcagg agaatggcgt gaacccagga ggcggagctt tcagtgagct 9540
   gagattacac cactgcactc cagcctgggc aacagggcga gactccatct caaaaaaaaa 9600
   atgaaaataa aaataaataa aacataaaac cctgccatta gttgcaacat gaagaatata 9660
   gagaaatgcg tatcaaatcc ttctcattgg accaatattc ccttagggca ccttccaaag 9720
   ctaggagact caaggctgta tgacatcctg agcaagtgag gggtggcttc tgggtgaatc 9780
   tgaatattaa atatttgcag aattgaaaac ttcacaaagt acctttagag atagaatagc 9840
   ctagatccat gtttctcaaa gtgtggtccc cagacctgct gcctcagcat ctcctggaaa 9900
   tttagtagaa atgcagattc tcaggcccta ggccagacct actgatcaga agctctgggc 9960
   ctggggccca gcaatctgtg ttttcacaag ccctctgggt gattcttctg tgcgtgaaag 10020
   ttcgagaatt cctggagcta gactgattca aatcttgcct ctgtatctta gagaccttgg 10080
   gcagattagt caacctcttt ctgcctctgt ttctacttct gtcagaggat gatagtactt 10140
   gtttcattaa gttgttgaaa ggataaatga attgacacac ataaagagta ttagctttta 10200
   ttatcaaaag cttttttttt ttgagacaga gttttgctct tattgcccag gggagtgcag 10260
   tggtgcgatc ttggctcacc gcaacctccg cctcccaggt tcaagtaatt ctcctgcctc 10320
   agcctcccga gtagctggga ttacaggcat gcgccaccac gcccggctaa ttttgtattt 10380
   ttagtagaga cggggtttct ccatgttggt caggctggtc tcgaactccc aacctcaggt 10440
   gatccacccg cctcggcctc ccaaagtgct gggattacag gcgtgagcca ccatgcctgg 10500
   cccaaaagct ttaatttctt aattttttaa ataaaataaa taaaactaga attgcttgtt 10560
   ttcttccagc taccctggtg attgtattga gcattttctg gggtgtgtgt tctttgctgt 10620
   aatgactact ggtctggatg acctgtgatg agaccagatg ggcaggggca gtggaggaga 10680
   ttctagagat atttaggaga taaagtcagc tgtacttgat gaaaagagtg gggagttaag 10740
   actggctgca gatgtatgat ttggcataga gaggtgccag ttcctgaggt gagagacaga 10800
   aggggaggga caggttgtga ggatgaatga acaatgatat gttcattctg ggcttggagt 10860
   taaggggcct atgatatgct taggggaagc agagagtatc aattacctat tgctgcataa 10920
   cagccacccc aaacttagtg gcttaaaata gcaacctttt aatttactca tgatcatgat 10980
   tctgtggtgc aacaactggg ctgggttcag ctgggcagtt cttctgttag tttcacccag 11040
   ggtcattcat gcatctgcag tttggggtgg gatggcctca gatgacctca ttcacatgtt 11100
   tggcaattgg tgattcactg ggggccatta ctgtaacaat cgcctaccag gcagagcttc 11160
   cctaaggcta ccaaactggg agactatcct gggtcctgtg ctgtggatac cactcagtcc 11220
   cccatcccca ccccatactc ctcaaaggca gagagagggg ctactagaag acagaggagt 11280
   tttcccagtg acatgtaaac actccaaacc ctggcacctt ccacactgca gctttggtct 11340
   gcccctttgg gaaatctctg tttttcttcc caggctgctg gaggggtgag agtcgccggt 11400
   agagtagagg ctgtgggcga ggaggtggcg gcctcctgag gctgcagtgg tctttccagg 11460
   cagcagtggg agcacagggt ggaggtcaac cctagagcct gggggagtga agctggttct 11520
   gccttcagag ctcttggtgc tgaagtttct gcaggccaga gggaggggca agagtgggag 11580
   ggggtgcaga tccagaatca cagaggcagc tgaccggagg aggcagctgc ccaaggggat 11640
   ggactcagaa ggccaaagtg ctgttatcca aacgaactct ttgcaagtgg tctctttgca 11700
   acaggcctgg gggagagcag tcttgcctaa agtcacaccg ctaatcagcg gccggcacgg 11760
   ggtaacagtt actaacactc actacgtacc caatgctggg caaagtgact tgcatgagcc 11820
   agcgagctca atgctcatgg caatcctctg agcagctggc attgtttcat ctcaatttta 11880
   cagctcagga agctgggaca cagaggaaga gccaggctct gaacactgac aacctgattg 11940
   agagacccac actgttcatc accgttacgc tatatatgct gtatagaaag gcaggatggc 12000
   ataatggtta aacctaggta ggtagggttt gaatcctcct gctaccattt actagctctg 12060
   tgacttggac tagttatagc acctctctgt gcctcccttt ccccctctct aaaatgggga 12120
   taataaatcg tacctcctac ctgaggctgt tgtgggctaa gtctgtaagg cacgtagaac 12180
   agtgcctgga acgtggggta ctgtctatct gtgtgcctgc tgttacaaca atggtgagta 12240
   ttgccttatc tctcgctgct gaactaccag gttagacttc tttctgcaag tcatgaggct 12300
   ttcataaact tttcctgaag gctttccgta gaatgtacaa ttcccctctg ggcccaggca 12360
   tgggcgcccg ggtaggacat ccacttctta tcacccctga acaccttaga gcccatcagc 12420
   ttatcaaacc agcagctgat gtgagtgcag agcagactgt gagaggtgga ggctgatacc 12480
   agtgaggatg ctccaagctg ggacccagcc ctgaagcggg agcccagata atggacgggt 12540
   ggaaatgggc ctggagccca agagaggtgg gaggatgagg gggcaggggg aggagaagcc 12600
   tgaaatcaaa tgttatttcc tgaccagttt ggggtgcatg agctctgtca acagctcatg 12660
   gaaactgctg ccctaatttc atcttgttgg ctgaggcaca attcctctct cagggacagt 12720
   gtagagcctt ggggaggaag gccctgagcg catatacctg gaatcaggga atcgggatca 12780
   ggggcagcag ctgtgcccga taaagccccc acccaggatc ctctgacttc ctcatctctc 12840
   tttttttttg agccggagtc tcactctgtc atccaggctg gagtacagtg gtgcgatctc 12900
   ggctcactgc aacctcagcc ttctgggttc aagcgattct cctgcctcag cctcctgagt 12960
   agctgggatt acaggcatgc gccaccatgc caggctaatt ttgtattttt agtagagacg 13020
   ggatttcacc atgttggcca ggctggtctc aaactcctga cttcaagtga tctgcccacc 13080
   tcagcctccc aaagtgctag gattacaggc ataagccact gtgcccggcc tttttttttt 13140
   tttttttttt tttttttaaa aaaagggtct ccctctgtcg cctaggctgc tggagtatag 13200
   tgatgtgatc gtggctcact gcagccttaa ccttctaggc acaagccatc ctcccacctc 13260
   accctcctga gtagctggga ctacaggcac ttgccaccac gcccaagtaa ttttgtattt 13320
   tttgtagaga caaggtcttg ctatgttgcc taggctggtc ttgaactcct cagctcaagc 13380
   aatcctcctt ccttggcctc ccaaagtgct gggattacag gtgtgagcca ccacacctgg 13440
   tctgacttcc taatctttag ggccccaact ctgcccttat ccaggcaact ctcctctccc 13500
   catcttccac taacttcttt ggaatattcc agagctgtaa aagccttaga gagtatcaag 13560
   tccaactcct atgtgttaca gacagggaaa ctgaggccta aagagggtaa tggacttgcc 13620
   taagatcgct tagtgaggtg agagaagaaa gagctagaga cagcctagcc tgtgcaagga 13680
   catagttcca ggcattcaga gctgcgctct gctgccggca tgtttggggc ctggtagtta 13740
   gttcactgct gaactaccag gttagatttt ctttctccaa gttgtggggc tttcataaac 13800
   ttttcctgaa ggtcttcctt acaatgtaca attctcctct gggcccggtc atgagcgccc 13860
   ctcacaggct ctctctggtc cccttctgta aaatgagagg aaaatggaag aattgctcta 13920
   ctcatggaat cttcaataag tctggaccct atgcatatag cattgctaca aaatggcaga 13980
   tgcactttaa caatcgtgtt taataaaagg ttggatttgc atatctgaag tggggcatgc 14040
   agtctccaac tgaacacaag cctcactgct cccacatgtg cactgcacct tcatatacat 14100
   atttcctgct tggctcctga gggaatttga gtaatcccaa gaggaacccc tgtagaaaat 14160
   gtcccctggt cacacacccc cattcctaag gatgcaagca ggagatagaa acattccctg 14220
   cacctccctc cttgctgtca gaagaagtgc aaagagttga atccttccta atgcccactt 14280
   ctcacccacg ccccaaatcc ccaggtcccg tggaggtcct tgggggtctc ctatatcctg 14340
   gtggtgtcag gttgatttgg aaatgtcagt gtcctccctt gtcctctctg gcagaccctg 14400
   ggtgtgtgta cgtttcaatg gaagtgaatt taaatgtact ttataaatca aagacttttt 14460
   ctgagacttt ggagagttcc agtaatgaga gcttctcatt gttatcaaag ccagggctgg 14520
   agaccagtgg caggtgagtt cctattgctg tgattgtcat gatgatgttg atgaacagcc 14580
   actatttatt gagtgttctc catgtgctag gcactgtact aaacattatt tccttcggat 14640
   gtcccagaaa cctctcaggt ggctctaatt acccttattc tgttgataag gaaagtaagc 14700
   aacttagaag accacagggc tatgaagttg aaacacgtaa attgatattt tattttattt 14760
   atttatttat ttatttattt tgagacagag tctcactgtg tcgcccaggc tggagtgcag 14820
   tggtgcggtc tcagctcact gcaacctccg cctcctgggt tcaagcgatt ctcctgcctc 14880
   agcctcccga gtagctggga ttacaggtgc ccgccaccac atccagctaa tttttttgta 14940
   attttagtag agacggggtt tcaccatgtt ggccaggcta gtctcgaact gctgacctca 15000
   tgatctgccc acctcatcct cctaaattgg tatttttata tgtccaaaag agtcaactgg 15060
   tggcaattta gtgaggttta atctaatagg aaatgataga gctgggatcg aacagagcta 15120
   tgtgaactca aaacctatgc ttccccttcc accttttcga aaaacattgt ctaggctggg 15180
   cacggtggct catgcctgta atcccagcac tttgggagac ggaggtgggt ggattacatg 15240
   aggtcaggag ttcgagacca gcttggccaa aaattagcca ggcgtggtgg tgcgcgcctg 15300
   tggttcccac tgaagcacag gaggctgaag cacaagaatc acttgaaccc gggaggcaga 15360
   ggttgcagca aaccgagatc gcaccactgc actccaacct gggtaacaga gagactctgt 15420
   ctcgaaaaaa aaaaaattgt ctacatgctg gttgcagaaa atttaaacac taaaactaaa 15480
   aaagtaaaac atctcccaaa gttagagaca atattcatga tgggaaaaaa aaaattcttc 15540
   aagatttctc tctctccagt catttattca tgtgcgaaaa cagttggtga ttattgataa 15600
   gaagagggag ggcagatggt gtggtagtcc aaggcacagg ctccagcaga ttatctaggt 15660
   ttaaatcttg gctgtaggcc aggccctgtg gctcatgtct gtaatcccat cactttggga 15720
   aaccgaggtg ggcagatcac ttgaggtcag gagtttgaga ccagcttggc caacatagtg 15780
   aaaccccttc tctattaaaa atacaaaaat tagccgggca cggtggtggg cacctgtaat 15840
   cccagctact tgggaggctg atgcaggaga atcacttgaa cccaggaggc agaggttgca 15900
   gtgagccaag atctcgccac tgtactccag cctgggtgac aagagtgaaa ctctatctca 15960
   aaattaaaaa aaaaaaatct tagctctacc caccggggca agttacataa cgcctctgtg 16020
   ccttggtttt catatctgta aaatggtgac agtaacagca cccatgtcaa agtgtggttg 16080
   tgagaacgaa acaagatagt ctatgtaaag tgattaaaac agcgtaggca catggtaaac 16140
   gcttaggaaa tgtaggctgt tataaagctc agagatgtta agtaactaga tcaagaccac 16200
   acagttagag agtgccacag tcttgatttg aacccaaatt tgtctcgttc tggagctcaa 16260
   gctgctaacc ctttttcaaa actggaatta aaccaaagtg ctcaccctcc gctttgctgg 16320
   gcccctccct gccctcaggt gcatctcttc cactcacctg ccacagcagc ctctgctcag 16380
   ggtctgagac tgggaaaggt gagggctacc caggtggccc tgatgttttc tgccagccag 16440
   ctcaccaggt ccctcgcagc aggcggcaaa gggagggagg tttgctgtga agattatgtg 16500
   gttcccaaca acaagagcac tgggcctatc tctgccctct cttttctgtg tgtcctggga 16560
   caagtcactt ggcttctgtg gctttatttt ctcatgtgcc cagccagggg gttggccctc 16620
   atatgcaata acagcagcaa tgacctttac tgagtgtcca tgtgcatcaa gcacgtgtac 16680
   tttacacttg ttcttattat taggtttaat aatagaataa ttgccacatt tactgagcac 16740
   tcattatggg ccaggccctg ccctaagtgc ttaattagct ttagctcctc taatccttac 16800
   cttatcccca cacggcatgt tatgttatcc ccattattca gttgagaaca ttgaggctca 16860
   aagaggcaaa gtaacttgac caaatacttg taaacgatct tgcatgcccc ttccagctgc 16920
   catttagtaa gactctaatt tcataccacc ctaaatctcg tctgcttccc cctcctcctt 16980
   ctcaccatct ccccaccgag cagtcggcca agatctgacc gtgatggcgg cccttggctt 17040
   gggcttcctc acctcaaatt tccggagaca cagctggagc agtgtggcct tcaacctctt 17100
   catgctggcg cttggtgtgc agtgggcaat cctgctggac ggcttcctga gccagttccc 17160
   tcctgggaag gtggtcatca cactgttcag gtattgggat ggtggctgga tcacttctgg 17220
   gtcatagagg gaatggaccc cgaaaggaca ggttccagaa gatctgggat attgccccct 17280
   ctctgtctag caccagtgct gtgcaatatt taggacatcc ttatgctaaa agattattca 17340
   ttgtttaaaa ttcaaattta actgggcatc ctgtatttta ctggacagcc ctactctgtg 17400
   tatcacaagg aatccaggcc tacattcctc ctgcatcctt tctttcctgt tattgtcgat 17460
   tatgattttg taaagttaca taatcagtat aagtttatgg aaaacgtaag aaggaaacac 17520
   gttagacaga gagaaataga catgccacac ctagagagac attctatttt ttttttttct 17580
   tttttgagac ggagtttcgc ttttgttgcc caggctggag tgcaatggcg ctatctcggc 17640
   acaccacaac ctcagccttc tgggttcaag cgattctcct gcctcagcct cctgagtagc 17700
   tgggattata ggcatgtgcc accacacctg gctgattttg tatttttagt agagataggg 17760
   tttctctgtg ttggtcaggc tagtctcaaa ctcctgacct caggtgaccg gcctgcctcg 17820
   gcctcccaaa gtgctgggat tacaggcatg agccaccgcg tccagcctga gagacattct 17880
   cttgaaaaga aaggactttc agccccctaa agctactaga caagaaatag ccatgccttt 17940
   attttcatta aattacctgt gctttgttta gatgcctttg tgtgaaatgc taagaaccat 18000
   cacaactaat gtatggtgcc agaagtcaga atagtggtta cctgggcagg aggtggatat 18060
   tgattaggaa ggaacacaaa atagccccat ggggtgcaga aaatgttctc tgtgttcacc 18120
   tgggtgatga ttacacatca agctatacac attttaaaag ggcattggca cttaatagaa 18180
   ggaactaggc taaatttttt cctgaaacat tgttttgttt tgttcaaacc tctgaatctc 18240
   tcagctcccc agatgatggt aaacgtcatc ctaggcatct tagggacctc tcaaggcctc 18300
   tcaaggccat tccagcctcc ccttctaaga ccctgctaaa cctctgggca ctgctgttaa 18360
   acatttctct atgagccagg aactgtgctg agcactccac aaatattatt ttgtttaact 18420
   cttccaggta gggatctaac ctggtataca ggtaaggaag tggaagctca gagagggcaa 18480
   ggcacttgcc tagggccaca cagctaagtg gtggagatgg ctctaacttt tttttataac 18540
   cttttccaca tgctccagag tggtcagaac atgaaacaca gtctagccag ctcctgactg 18600
   gccctagagg aaaaaaactg tatgtatttt tcttttttaa aaggtttaga ggctgggcat 18660
   ggtggttcac gcctgtaatc ccagtacttt tgggagctga ggtgggcaga tcacttgagc 18720
   ccaggagttt gagaccagcc tgagcaacgc agtgagaccc tgtctctgca gaaaatagaa 18780
   aaatcagcta ggcgtggtgg tgtgcaccca cagtcccagc tacttgggag gctgaggcag 18840
   gaggatcacc tgaacccagt gaggctgagg ctgagtgagc catgatcgtg ccactttact 18900
   ccagcctgga caacagagtg agaccctgtc tcaaaaaaca gttttagggg ccgggcgcgg 18960
   tggctcatgc ctgtaatccc agcactttgg gaggtggggg tgggcagatc atgaggtcag 19020
   gagatggaga ccatcctggc taactcggag aaaccctgtc tcgactaaac atacaaaaaa 19080
   ttagctgggc gtggtggcgg gcgcctgtag tcccagccac tcgggaggct gaggcaggag 19140
   aatggcgtga accttggagg cggagtttgc agtgagccga gatcgtgcca ctgcactcta 19200
   gcctgggcga cagagcgaga ctctgtctca aaaaaaaaaa aaccaaaaac aacagtttta 19260
   ggccaggcgc ggtggttcat gcctgtaatc ctagtacttt aggaggccta gacagatgga 19320
   ttacctgagg tcaggagttc gagaccgacc tgagcaacat ggtgaaatcc tgtctctact 19380
   aaaaacacaa aaattagctg ggcattgtgg caggcacctg taatcccagc tacttgggag 19440
   gctgaggcag gcgaatcact tgaacccggg aggcggaggc tatagtgagc cgagatcgcg 19500
   ccattgcact gtagcctggg cgacagagtg aggctccgtc tcaaaaacaa aacaaaacaa 19560
   aaaccatctt agagttaatt cccaccggga ttcaatacac acacacacac acacacacac 19620
   acgcacgcac gcacgcacgc ccgcatacac acactgcatc cacctggaaa gtgacaaagg 19680
   gcaccctggg gggaattcaa atggtggtgg ccctggtttg gtgttgctgc cttagcttaa 19740
   ggtcacacca gccttcagcc tcctgcccca cagtctaggg ctgctccctt catctgatgt 19800
   ccacagggac ctgttcattc ttgactcaat ccaggaagat gagaagggag agaagtcact 19860
   cgcagcctga gtgaactccc ttgctccacc cctgactgct tggatccccc taggggtgac 19920
   ccctgctgaa actggctcct tcctgaccgg ttcccgtcag ggctgtgctg atgggtggtg 19980
   cccaggcctg cccctgggga cggggtactc tcccttggca acactccagc ttgtgccact 20040
   tgacttggga ctgatttggt tctgttttga gtcccttcag gggaggggcc tatcttattc 20100
   aacgttgttg tttgttttcc tcacatactg ataacttagc aaatggctat tggaacaaaa 20160
   atgaaaataa atggaaccct gaagtgggat gttttaaatt tttatttatt atttttttag 20220
   agacagggtc ttgctctgtt gcccagtctg gagtgcagtg gtacaatcat agctcactgc 20280
   agcctctgcc tcctgggctc aagtgatcct cccacctcag cctcctgagt taaatttttt 20340
   tacagacgcc tgctaccatg cccggctaat ttttgtgttt ttagtagaga cggggtttca 20400
   ccaggtgggt caggttggtc tcgaactcct gacctcaagt gatccacccg cctaggcctc 20460
   ccaaagtact gggattacag gcgtgagcca ctgtgcccgg cctaaaactg tgtttgagac 20520
   agggtctcac tctgttgtcc aggctggagt gaagtggcat gttcatggct cactcagcct 20580
   cagcctcact gggttcaggt gatcctcctg cctcagcctc ctaagtagct gggactatgg 20640
   gtgcacacca ccacgcctag ctgatttttc tgtcttctgc agagacagga cctcactgtg 20700
   ttgctcaggc tggtctcaaa ctcctgggct caagtgatct gcccacctcg gctccgaaaa 20760
   gtactggaat tacagcctcc tgagtagctg agaccacagg cacacaccac cacgcctagc 20820
   tttttttttt ttttttttgc tttttgtaga gatggagtct cactatgttg cccaggctgg 20880
   tctcaaactc caggccttaa gcaatcctcc cacctcagcc tcccaaagtg ctaagattac 20940
   aggtgtgagc caccattcct ggccttaaaa gtgtgatatt tttaatgtat tttgaaatct 21000
   gcaggactct ccctagaaga taatagcaat aaccaactcc tttattgtgc ttgacgtata 21060
   tcaactcact ttgcccttac cgtggctcca gaggcattgg gtccacctta taaatggagg 21120
   caccaaggca cagagtgatt aaataagttg cccaggatca cacagccaga aagtgtctga 21180
   gtcaagattc cagcccaggc agcctagacc tgagagcacg ctcctaacca ctgcacatca 21240
   ctgtcttagc acctcctcag cacaaactgg cccttgagga atgaaatacc gccgccggca 21300
   cacacgctcc tgagttaagc ctttgtcaat gaaatgaaca cccacttaaa aggaataacc 21360
   tgtccaggca cgatggaaca ttgaataacc ccttattcta aattcctggt ccctgtaaga 21420
   ctccttcccc atgcccttgc ccttttatga ccttccccta aagtccttga ggcttaagcg 21480
   ggcatagtct gcagcaaaca ctggggaagc tgagtccaga cttcagagca caggctttgg 21540
   atctaggcca gctggatttg aacctcacat ttgtgatcag ctggcatgac tgtttccaaa 21600
   aagtccattt taatcctcta cgtgaccctc tgtaaaatgg ggatactgaa cggtgagcta 21660
   gcacgatttt acagagagtg aatttttttt tttttttttt tttgtgagac agagtcttac 21720
   tctgtcgccc aggctggagt gcagtggtgc aatctcggct gactgcaacc tctgcctccc 21780
   gggttcaagc gactgccatg cctcagcctc gagagtggct gggattacaa gcatgcacca 21840
   ccatgcccgg gtaatttttg tatttttagt tgagacagag tttcaccatg ttggccaggc 21900
   cactcttgaa cccctggcct caagtgatcc acctgccttg gcctcccaaa gtgctgggag 21960
   tacaggcatg agccactgcg cccagcctta tagggttaaa atttaaaaga ggtgatgctg 22020
   ttacaagcct gttttacaaa atgctcttat aataaatcat tatcatcact gttgctgtgg 22080
   ttgtagcatc atcatcatta actcccagag ggaggaggga gtctcagagc aagctgctca 22140
   ggggagactg gatgtccatg gattgtccag ctcagtacca cttcctccag gaagtcctcc 22200
   ctgataagtc cagtcagcat caccctctcc ttccaatgaa ccccactagc cttgtgatat 22260
   cacagatatt cttagttgac aggctcatgg tgtatgtagc ctgtctagat cataagtaca 22320
   tttttttttt ttttggatca taagaacctt caagaccaaa ataattttct cctcctgagc 22380
   atgctcattg gtcaagggaa ggaaggaatc gtaatagtgt taataaggct agtgtctttt 22440
   caggagttgg ttctttgtgc cagtcttggt gctagacaca ccgataggaa gaatactcct 22500
   tcacatcccc aggacaccaa catgggatac gtttgatcat cattcttaat ttgcagaagg 22560
   agaaataggc tcagtgagat gaaatagcca ctccagtggc aaggctggga ctggaagccg 22620
   ggcttgtcct gattccaaat ccagtttctt tccactgcca cggagaggga gagaagggac 22680
   agtggcccca gatgaggatg gggtgactgg atgtgggcag gcctgcgggg gaagagtgcc 22740
   ctctgttgag catccgaatg atggcagcag aaaagaagac tgggcagaat cccagttatc 22800
   agatcccctg agggaacagt caccccgatc accctcagtc agatgagtgt gtgtagatca 22860
   atgcctcata gatgaaggca ctgaggcaca gagtggttaa gtcatctgcc agaccacatg 22920
   gctcagggtg cagaggccac cttaacggga gaagagatgg tcactccact ctgcagcatc 22980
   agcgcccagg tgggtagaaa tcttgtcttc tatttccaca gaaagtaagg tgcccaacag 23040
   tgtttgttga atgaatgaat gaatgaatga atgagtgaga ggcatccttc cttctcagtc 23100
   atcctggctc tccttctcac ccccagtatt cggctggcca ccatgagtgc tatgtcggtg 23160
   ctgatctcag cgggtgctgt cttggggaag gtcaacttgg cgcagttggt ggtgatggtg 23220
   ctggtggagg tgacagcttt aggcaccctg aggatggtca tcagtaatat cttcaacgtg 23280
   agtcatggtg ctgggaggag ggacctggga gaaaagggcc aaaagctcca tttggtgggg 23340
   cttccggggt tttgaaaaat aaagacaacc tgtaatccca gctacttggg aggttgagga 23400
   gggaagatca cttgaggcca ggagtttgag acccgcctgg gcatcatagc aagatcctca 23460
   tctctaaaaa gtaatttttt ctaaattatc cagttgtggt ggcatgcacc tgtagtgtca 23520
   gttactcagg aggctgaggt gtgagttgga aggattgctt gagcccagga gttagagatg 23580
   aacctgggca atatagcaag acctcatctc taaataaata ggtaggtgga tagatagata 23640
   gatagataga tagatagata gatagacaga cagacagaca gacagacaga cagacaggct 23700
   gggtacagtg gctcacacct gtaatcccag cactttggga ggccaaggag ggcagatcac 23760
   ctgaggtcag gagttcaaga ccagcctggt caacatgggg gaacctcatc tctactaaaa 23820
   atacaaaatt tagctgcgca tggtggcagg tgcctgtaat cccagctact caggaggctg 23880
   aggcaagaga atcgcttgaa cccggagggt ggaggttgca gtgaactgag atcgcgccat 23940
   tgcactgcag cctgggggac aagagcaaga cttcatctcc aataaaaaaa aaagaaaaaa 24000
   gaaaagaaaa gattgataga tagatagata cccaaatgag gttacaaaag tgtggtctgt 24060
   gcaaatgttt aaacacaaca aaccagtgcc tttaactact acagtataat cctgtaggat 24120
   tgtgctattc atgatgtaat tatggttgta taaaagtaat taattctcag agcctcacca 24180
   gcagtgggtc cagcaagttt gtacagccag catcttcttt cagtcagtgc gtgtcagtaa 24240
   ctgcacatgt cctctcattg ggagagcctg tcgaaagtct aagtttgaag gcagctgtga 24300
   aggtaaggcc aatccaaatg gctctcccag ctcctctgct gtaaccctga ccctgagtga 24360
   ggacatagcc aaccttccca tctcataggt gagaaggctg atgcctggag aggggaaggg 24420
   actgcccaag atcacatagc aagatagtgg cagaacccaa gcgagaaccc acagttccag 24480
   cctggcttag aagaaagtgc actggacttg gagtcaaagg ctggggtgtg catcccagct 24540
   ctgccataaa tccctgtgtg actctgggca atttaacctc ttagagcttt agtttcttcg 24600
   tctgtaatat gagggtagca gtactaccac atagggtttt gagggagtaa ttgaattaat 24660
   cacatgaaat gatgcacgtt tacaaaaaaa agcatgaagc ccctttactg tgcctcagta 24720
   tcccaaagga ctttggattt actctgagaa atacagggag aactagggag tgttgggcag 24780
   aggagagcta tgatctgact tatgttttaa gatactctgg cttctgggtt cagaaaagac 24840
   tgaaggggca agagaggaag caggtggaga ccagagcagc agtgatggcc atcatccaga 24900
   ctcagactag gacaatagct gtgagggtgg tgggaagtga ttggatcctg actatatttt 24960
   aatagcagaa ttgacaggat ttgctgatag actgcacgtg gggtgggaga gggtcaagat 25020
   gacttcaagg ttctcatctg gcacaactca gcagctgctg gtgccattta ctgagatggg 25080
   gaacattggg gtgggataga tctgggaggg aaaacccaga gttcagtgtc gaatgtggta 25140
   gcgttagggt taaggttggg gcgggtagag atgtgtatga aacatcccag tggagacact 25200
   gaatggagat gtacaagtct gaagcttagt ggaaaggtta gggctaggga tataaatttg 25260
   ggagttgtta caatacagat ggtgtttaaa gccatgagac ccaaggagat cactcaggag 25320
   tgaggataaa gagagatggg aagaagtctg aggactgagt cctagaacac cctgcatttt 25380
   agagggggga catgtgtaag agccagcaaa ggagacagaa ttgtgcttgg agaggcagga 25440
   ggaagcccag gagagcgtga ggtcctggaa ggcaaggaaa gagagggccc caggtgggct 25500
   gaatgctgct gagaggtcaa gtcggatgag ggctgggaag tagccattgg atttgacaag 25560
   gagaccttgg catgcatggt tgtagaggag gatgaaggca aaagcctggc ttgactgatt 25620
   caagagcagg agatgagaaa gtggagacag catgcagggg cagccctgcc aaggactttg 25680
   ctctaaaggg gaacagagaa atggaggaga agcaggaggg caataatccg atagagagga 25740
   aaaatctgat gatacagaag agagatgaac tgcaagagtc aagcctttga gttggaaagc 25800
   aggagtggga ttttgagcac tgataccttt aggccgatgc agggacagtt catctttttt 25860
   aaaattatta ttattataca acattttatt taaaaattta ttttcacaga atacattttc 25920
   acattagaga ttcccattgt gcgaaaataa caatttatta cttatagttt tatatttgtg 25980
   gacagattgt tttagaacaa gtagaataca tttgagaatt aaatctcagt ttacaatggg 26040
   taatattttg atacgtctat ggggaaactt gcccttaaat ggaacttctg tatcttcaga 26100
   agcactccaa gcgtttcttc ctaggattta gaaatttata atatgagata tcagcatttc 26160
   ctaattttaa aatttcccta gtatatgtaa ccatcggtag gtggtatcta ccgactagag 26220
   agggaagttt ttgaaaatta aacactgtct aattttctgc aaagttttta ttcatgaatt 26280
   aagagtattt cccttagtcc attattccca aggcaaatat ggaagtttga tcatatgcta 26340
   atcatactaa agctggattc tctttaagag attgagaaat taaaaggcaa aagctgatat 26400
   atcatgttta gttatactgt gagtcttata agaagctggg aggcaacccc attaactcac 26460
   cagaatacag aactcagtct cacaacttaa atataattcc tctcaaacct tttcctcaaa 26520
   gttaaattct gaaaataatc ttgtgattaa gagaagaagg ctgtccacca atggacttat 26580
   ctgttatttc ttccttattg tgagcttaat ggcatgacaa agcagaggca aagaggcata 26640
   catcaattct tcaaagtagg aagtcaaaaa ggtcagagct tccacagcat ggcaacagct 26700
   ttgcagatgc ccacatcgtg atagttgaaa tagcaaagcc cagcaaaggt taaagctgaa 26760
   aatgccaaaa gccctgcctt ggcagctttc tgcgaggcat ccccatgaac atagtcagta 26820
   acaacttgtc caaggcccca gtgaccatga agagtgaggg ctgcagccag ggaatagtcc 26880
   gtcgcagagc aaggattcaa ataagcagcc ggaagcagac ccgggagcaa aacactgaca 26940
   accctctcgc tagtccagtg gagagatgca gccttggagc cagaatggtg gctcggtgac 27000
   aagtgtatgt gctgcactcc acaccattct gggataggtc ggtcctgaag aaatgctgag 27060
   atatgagcag gtctgaccac tggagttcgc agcaacagag ctcggcctcc ttgggcaccg 27120
   caaacggcac tcagcctcca gagaaccgcc atctcgttcc tgaggcggag agttcatctt 27180
   aacgagagaa atggcaggga ctgtgaatag gccggcagat ttggtggcgg gtgccacagg 27240
   ttcagtctcc tgcagggaga ggagaaaatg ccttactaat tccttgtatt ttctcagaga 27300
   aacaagaggc accgtcatca gcctcatgtg agggtgggaa ggagggatgg ggtttgcgga 27360
   gagggaaagt gtggtatggt catctgtggg agtggaagag agtgagaggg ctgcaggggt 27420
   gcagcgggac tgcaggctgg caccagggtc cctagggctt gtagttggtg gaaagtgcat 27480
   cagtgaccag ggctgtgtgc agctgctcca ggcaggtgtg gaagaagcag agttgaactt 27540
   gcccagcctg gagtgctgcc cagagtgagc ccaaagccca agggagacca gagatggggc 27600
   tgtttgcaaa ggaggaagta taacagtagc ccacaaaatc tgagctggtt aagaaaggag 27660
   agagagtgaa aatggggagc ccagcctggc agcctgggta cacatctcag ctcaacccac 27720
   actagctgaa tccatttggg ccccttcgtt gacctctctg tgcctcagtt tccctatcta 27780
   tagaatgggg ataagaataa ggctacttcc tagggctgtt gtgaggattg aacaagtgac 27840
   cgaacacttg ttcaattttg aatactgttc taaagcattt aggacagtgc ctggcatggg 27900
   gtaagtgttg cggcagtgct gttattttca tcatcaccat tgttctcagg ctgcgttgat 27960
   tggagctgct gaagggaggc aatttaagga agtgagccgg acagatagga ggtggtggtg 28020
   gttatcaggt gcgatgcttg aaactgaggc ttcggaggca acagttactg gtaatgacaa 28080
   ggtctaaggc ttgacagtgg gtggcagaag tgtaacgcag ggaaagagac gagcggtcaa 28140
   ggagccgaga gggaaggagt tgggtggact aagatcattt gtggaagaat gatggagaga 28200
   aaggctgaag ggcaggaact gacatcatca gtgaccaagg ggcggccagg aggctgagac 28260
   cgcagcaaga aagggagagt gtgatggcat cttcttcaag ggagctgggg atgtttgggg 28320
   tggaaaaaag aacaatggtc tgggagggaa tatgggaagt tttttttttt tttttcagat 28380
   ggagtttcgc tgttgtcacc caggctggat ggcaatgttg caatctcggc tcactgcaac 28440
   ctctgccttc caggttcaag tgattctcct gtctcagctt cccgagtagc tgagattaca 28500
   ggcacacacc accacgcctg gcttactttt gtatttttag tagagacgga gttttgccat 28560
   gttggccagg ctggtctcaa actcctgacc tcaggtgatc cacccgcctt ggcctcccaa 28620
   agtgctggga ttagaggtgt gagccaccgc gcccagcctg gaagtttgta tttattaatt 28680
   tttggttgtc ttcatctgtg tatgtgactt taacccctaa atacttcagt gtacatttct 28740
   tttttttttt tttttttttt tgagacagag tcttgctcca tcacccaggc tggagtgcag 28800
   tggtgtgatc tcggctcact gcaacctccg cctcctggat tcaagcaatt cttgtgcctc 28860
   accctcccga gtagctggga ttaggggcat gccaccatgc ccagttaatt tttgtatttt 28920
   tagtagagat ggagtttcac catattggcc aggctggtct tgagctcctg gcctcagttg 28980
   atccacctgt ctcagcctcc caaattgctg agattacagg cgtgggccac cataaccggc 29040
   ctcagtgtat atttctgatg cagttgggtt ctgtatcccc ctccaatctc atctcgaatt 29100
   gtaatctcca cgtgttgagg gcaggacctt gtgggaggtg atgggatcac aggggtggtt 29160
   tcccccatgc tgttcttgtg acagtgagtg ggttttcagg agagctgatg gtttgaaagt 29220
   gtggcacttc ctctctctct ttctctctct ctctcacctg ccaccacgta agatgtgcct 29280
   tgcttccctt tcaccttcca ccatgattgt aagtttcctg aggcctctcc ggccatgcca 29340
   aactgtgagt caattcagcc tcttttgttt ataaattacg cagtctcagg aagtatcttt 29400
   atagcagtgt gaaaacagac taacacaatt tcctaaaaca aggggacatt ctcttacata 29460
   accattgttc agttaacaaa aatgagaaat tgacattgat atattatgat taccttattc 29520
   tcatttcacc aattttttca ataatatcct ttctagaaaa aaatacatat tttttgtggt 29580
   cgaggattac atcttgcatt tagttctcat gtcttattaa attccatcaa tctggaacag 29640
   tttcttcatc tttctttatc tttcatgacc ttgacatgtt ttgaagtttc gagccagttc 29700
   ttttgtagaa tgtgggtttg tctgctgttc ctcatgatta gattgtgggt atgcattttt 29760
   ggtaggaatt ctccaagagc cgtgtgtgcc cttcttagta tatcatatca gaagacatgc 29820
   tatcaatttg ccccattact gggtgtgtta actgtgatca ttgggttaag atggtacctg 29880
   ccaggatctt ccactgcaaa gttactattt tcccctttgt aattaataaa catcttgtga 29940
   ggagataatt tcctatagaa atcctgttga tcatccaact ttcacccact gattttagtg 30000
   ttcattgatt cttccctgaa taaattagta ctataataat tgccaatggt ggttttctaa 30060
   ttccatcttt ccttcaatag ttggcattct cctgtaagga aaagctttcg cttctctgtt 30120
   catccactca tctatgtatt tgtttatatt accatggact cctggattcc ggtttacaca 30180
   cttccatttt ctgccttttc tctctgctta atataaggat taatgagaac tccctgattc 30240
   ccaggaagaa aatgtcacca gagctttctt aggtggaatg aagagaattc agtgtaagaa 30300
   ccataaaggt gtatctgtgt agtatggaca gttttaaaaa acaaacaaac aaaaagaacc 30360
   tccaagggca ggaagtgctg ccagactcag gagggcacta gaactgacta tgagaagcca 30420
   ctgagatccc aggtagtctg tgctctccat cttttggctc tgattctctc tgtacatcta 30480
   acatctctgt acaccagctt tctctttagc gaaaaacgtg tcccctccac ccacccatcc 30540
   acctccactt gttcctgcat ttctatgtcc cagatcctgc agaaaacaac tcttttctct 30600
   cagttagtct caattctgta gtccagggag agagaatctg atcagtcccc tgggtcattt 30660
   ttccactctg gtccaagcag ctacagctgg catgggaaat agttcacaca gtaaaaacat 30720
   ggctgtcaag aagaggagta aatttcagag gcagaacact ccctgtgagc ccgaacctct 30780
   tcctgctttg ttgcagtctt cataacgatt gctttaaaag actgcattga tataacatca 30840
   tctctcttct ctgcatcttt gacttgctag cttaactggt ctagaggagg gcttagcact 30900
   gattttcagt attcattttc ctcaaaactt caattcagcc tgggtttctt cagcaggagg 30960
   gctcggggga accagagcca gggaccagag tcatttcagt gcaccagctc aagaaatgaa 31020
   tattccaggc caagaatccc caagtgttct ttctgaagtc cttcctggtg gagctcaaag 31080
   agatgaaaaa cgcaagcccg cttttcagtt cttatcagga aactgcatag actttcctct 31140
   ttatgtatga ctgagggctt tttaccatca tttgttcact tcacagatat ttatttggta 31200
   tttactatat accaggcact cttgtggcag tggaaaatac aactctcgtg gaacatctgt 31260
   tccagaagga aagactgcca ataagcaata aaataggcaa aagatatagc atgttagaga 31320
   gtggtaagta ccacagagaa aaataaaatg gagaaaagaa acacgaaaag ttggggagag 31380
   aggacaactg tttgaggggg tggccagggg cagcttcatc tcatcaaggg ggtgattttt 31440
   tttgagtaca gacctgaagg taacgagtgc acaagccaca tgggtacctg agaacagcgg 31500
   cagaacaatg gcagggtgct gggagggcta tttaccaccc atgctgttta gaattgtcag 31560
   cacatggtga taaaaaaaaa aataggctgg gtgcggtggc tcatgcctgt aatcccagcg 31620
   ctttgggagg ccaaggcgga tggatcactt gaggtcagga gttcgagacc aggctgggga 31680
   acatggtgaa accccgtctc tactaaaaat acaaaaatta gccgggcaca gtggtgggcg 31740
   cctgtaatcc cagctacatg ggaggctgaa gcaggagaat cgcttgaacc cagtgggtga 31800
   agtttgcagt gagccaagat ggcaccactg cactccagcc tggcgacaga gcgagactcc 31860
   gtctcaaaaa taaataaata aataaataaa aataaaaagc agacagactt tttagttggc 31920
   tttagaattg ttagacaccc tctgcagaca aggcaccccg attgcttgca cccagggtgg 31980
   actactccct ccatcctgcc cttgttacac cctggctggg ggtcagcatt tcaggcagct 32040
   gaatgaccca aagtgggaac acgctagtgg gtttgaggat gagcaagtgg aggagtgcaa 32100
   taggaggtga cgcccgagag gtcaggtgag agtggatcct gcagggtcgt ggcaagaacc 32160
   tggaccttga ctttgagtga catgggagcc gctggaggct tctgagcaga ggagtaacat 32220
   gatctgactt gcattttatt ttatttattt atttgacgca gtctcactct gtcgccgaag 32280
   ctggagtgca gtggcgccat ctcagctcac tacagcctct gcctcccagg ttccagtgaa 32340
   tctcctgcct cagcctccca ggtagatggg attacaagca agcatcacca cgcctggcta 32400
   atttttgtat ttttagtaga gacagggttt tgccatgttg gccaggctgg tatcgaactc 32460
   ctgacctcag gtgatccacc cacctcagcc tcccaaagtg ctgagattac aggcttgagc 32520
   caccacgccc ggcctgactt gcattttaac agggtcactc tgtctgctgt gtggagaaca 32580
   gtccgcagga agacaagggt ggaaatgggg agaccagtta ggaggttact gtaacaattt 32640
   ggggtagcgg tgatggtggc ttaaaccaag atggggtcag tgggaaatgg tgctaaaaat 32700
   cctgccaatt ctgggtattt ttagaaagca cagctgacag ctttctccag tagcccacta 32760
   aataagttat gaagcattac taaaatgtga tagtcatgat gcaaaattag aatatatcta 32820
   gaatctcccg aagaccttag tttggtatta caagaagtct ggttgcttca tgttgcaaaa 32880
   tttatatcac tcatcactcc tgcagagtta aaattccgct gagaagtagg aatcagtgaa 32940
   gtgcgtgtcc atgtgggttt ttgccacacc taagtgaacc ttggtcaaaa gcatataaga 33000
   gctactgata ggccgggcgt ggtggctcat gcctgtaatc tcagcacttt gggagggaag 33060
   gatctcttga gcccaggagt tcgagaccag cctgagcaac atagtgagat tccatcttta 33120
   cacaaaattt aaaaattggc caggcatggt tgtgcactcc tgtaatccca gctacttagg 33180
   aggctgaggt gggaggattg cttgagcctg ggagttggag actacagtga gctgtggcca 33240
   caccactgca ctccagcttg agcaatggag caagactctg tctcaaaaaa aaaaaaaaaa 33300
   aaaaaaaaaa gaggccgggc acagtggctc atgcctgtaa tcccagcact ttgggaggcc 33360
   gaggcgggtg gatcgcctga ggtcaggagt ttgagaccag cctggcaaac acggtgaaac 33420
   cccatctcta ctaaaaatac aaaattagcc cagcgtagtg gcgcatgcct gtaatcccag 33480
   ctactaggga agctgaggca ggagaatcgc gtgaacctgg gaggcaaatg ttccagtgag 33540
   ccgagatcgt gccattgcac tccagcctgg gcaaagcctg ctgggttggg ctgggtaagc 33600
   tctgaacacc agtctcgtgg cttcaagtca cacctcctaa gtgaagctct gaactttctc 33660
   caaggaccat cagggctttc ccctgggcag aggatgccga cactcactgc tcttactggg 33720
   ttttattgca gacagactac cacatgaacc tgaggcactt ctacgtgttc gcagcctatt 33780
   ttgggctgac tgtggcctgg tgcctgccaa agcctctacc caagggaacg gaggataatg 33840
   atcagagagc aacgataccc agtttgtctg ccatgctggg taaggacaag gtggggtgag 33900
   tggtctcata cttgggctga gcagaatggc tcagaaaagg ctctggctga aaaaatctcc 33960
   ctcctttacc aacttcccct gggtgtctga agcccttcca tcatgattca cttctttgag 34020
   tagtgtttgc taaattcata cctttgaatt aagcacttcc ttttagggac ctctcttcat 34080
   taatatccac tagaaaggag agactcatta tgtgtgagtt tcaataagtt tatccaatcc 34140
   ctttgttttc aactgaaagg agggaaacgg acaagtgaag aaggtagggc ccaggagtga 34200
   aggaacaagg gtgggaatag taataatgtt gtactttgaa aatctactgg gaaaatgatg 34260
   aacttagact gctgggagag gctaatagaa aatcgggcag tgagcttgat agtaggcaaa 34320
   ggactatcag gccacggggt caagttaaag cagcacattc attaaaaaaa aaaaaataag 34380
   cgtttgggcc aggcgtggtg gctcaagcct gtaatcccag cactttggga ggccaaggtg 34440
   ggtggatcac ctgaggtcag gagttcgaga ccagcctggc caacagggcg aaaccccatc 34500
   tctactaaaa atacaaacaa atcagctggg catggtggtg cacgcctgta atcccagcta 34560
   cttgggaggc tgaggcagga gaatcttttg aatccaggtg gtggaggttg cagtgagcca 34620
   agatcgcgcc actgcactcc agcctgggca acagagcaag agtccatctc aattaaaaag 34680
   aaaaaaaaat taaaataagc atttgaccat cacagagcag gttcaggagg cctggggtat 34740
   gcagatttca accctcttgg cctttgtttc cttgtctgta aaatgtggtt agctggtatc 34800
   agcttgagag ctcggagggg agacgtgact tccccatcta actctaagtg acaaggctga 34860
   gactctccag ccctaggatt ctcatccaaa acccctcgag gctcagacct ttggagcagg 34920
   agtgtgattc tggccaacca ccctctctgg cccccaggcg ccctcttctt gtggatgttc 34980
   tggccaagtg tcaactctgc tctgctgaga agtccaatcc aaaggaagaa tgccatgttc 35040
   aacacctact atgctctagc agtcagtgtg gtgacagcca tctcagggtc atccttggct 35100
   cacccccaaa ggaagatcag catggtgagc agggcgctgc ccttgggcag cacttgggtc 35160
   taacaggact agcacacata tttatgcccc tccccacccc agggccagcg tgggttggga 35220
   gagggcatgc cgggtggtgg agctgtgcct gcctctacag tggagctcta ggaagaatgc 35280
   tgggtggtca caggggccct gggactcagg agactgtcca gtgatcaaag gctttctggg 35340
   gggagtgatt aaatccatcc atgctaacat gaaacagacc tgagtttgaa ccccgtttct 35400
   gctagttgct caagtcagtc accatgagcg agagtcagca gcaacagact agactagaat 35460
   tagccagcct ctctcttccc cccaacaaat ttcaagaatg gaaccatcag aatcagaagt 35520
   agagaagtat gtgacactag ccatgtggct ctggtcaagc cacttcaacg ttttgagtct 35580
   cagtggcctc atctgtaaag tgagaattaa gagatggtgc atgtaaagtg cttaacgggg 35640
   agtaaatggt aggcaaacat tagctgctgc tattagtaca gagagacaat ggtgtgtgtg 35700
   agtcttgtgg gcagagatgg gtgagagggg agacaaaaca agttctcatg atgatggggg 35760
   cagggggtcc agctggtggt gtcggaggga agtctggaca gaccagtggt ggggctcggg 35820
   tgggaggcac tgggggggct ggagtggaaa gaatgtggcc acagatgaca gcttcacagc 35880
   agaattcagt gctaagagga agtgagtggc catgagttcc atggtgacag aaagtctaag 35940
   acacctagca aggcaggagt gggtgtcagc tcagggaagc tcagaggcta aacctaggtg 36000
   agagctgagg gtgtcagata agagcaaggc aaggctccgg ttctggagta gtgaaggaca 36060
   tagcagagct ataacccagg aacaaggccc agcttattgg aactgggacc agtcacacag 36120
   ggtggcacag gcaccaagta gccaataata ataataaaaa caataacaat gatttatgtc 36180
   tattgggcat ttattcatgt tctatgccag acactggact aagagcttta tatgtggaaa 36240
   ctcatttaat ccttacaata accttatgaa gaaggtacat ccaaaacccc attcttctag 36300
   gccaggtgca gtggctcaca cctgtaatcc caatattttg gaaagctgag gcaagaggat 36360
   tggttgaggc caggagttca agaccagccc aggcaacata gcaagaccct gtctctaaaa 36420
   aataaaacaa aaacccattc ttcccgctgt ccagggacac accactaatg agtgtgatgg 36480
   gtgcctagga tgctgagcac ctggacttcc cagctcattc cctaaatgct gcacaatcag 36540
   ggtaactgtg ccctgagcct aagaggcagt agtgagctgg cccaccgtgt ccactgatga 36600
   aggacacgta gccccaacac aggggagagg tggtttcagg atcagcaaag cagggaggat 36660
   gttacagggt tgccttgttc ccagcgtgct ggtcacttgc agcaagatgg tgttctctct 36720
   ctaccttgct tcctttaccc acacgctatt tctttgcaga cttatgtgca cagtgcggtg 36780
   ttggcaggag gcgtggctgt gggtacctcg tgtcacctga tcccttctcc gtggcttgcc 36840
   atggtgctgg gtcttgtggc tgggctgatc tccatcgggg gagccaagtg cctgccggta 36900
   agaaactaga caactaatgc tctctgcttt ggctgaaggc cagcaggacg ctgggacctg 36960
   atgggccact gtgcagtgca cagctgcatt aggcaggtgt tggtgcattc tcttattggc 37020
   ttcaacgcct agcgagggat ccatcctggc tcggtggcac atttgttaag atgctgggga 37080
   gcaggtggca gaacccattt gagcttgctt gggcactggg gagaatttgt taccaggcta 37140
   caggggtgtc acagaactca aggacaggga ctggagtgtt gtggggagcc cagaagcccc 37200
   tgttttactt ctttctttgc ttttcctgaa tatctgcttt attcttactc tatagacctg 37260
   cttcctcctc tttcacccca cattgtgggg tgtagtcttt tgcttcaaga aagcagcctg 37320
   gtggatggaa tctcttggcc ccaatcccaa attctctgga gaaggggctc tttggtttaa 37380
   cttggataat gttgtcttca gctgggggtg ggcacatcgt gcatatgtgg ctgctgccgg 37440
   ggaaccacgt ggatgatgtg agaggagcag cacccagaag agggagtgct gggctgatgg 37500
   tccaggtcgt gtccacttct gattgtttaa ttcttcttct aagtggatgg atctttctcc 37560
   aatactcagc aaatcctgat cgttccagaa tacttcatta tagccaattg gttataatgt 37620
   gcttctctaa gagaaatatt tagggacaac aaatcttcat gggtttgaag acttgatgga 37680
   ggaaaaagga gtagattttc gaaggctgga tttggatgaa caggggctat tcagggagtg 37740
   cattccaacc taaaattagg aaaaactggc tgggcgcagt ggctcacgcg ctttgggagg 37800
   ccgaggcggg cagatggcct gaggtcagga gttcaagacc agcctggcca acatggtgaa 37860
   accatctcta ctaaaagtac aaaaattagc caggcgtggt ggcgggcacc tgtcatctta 37920
   gctactcagg aggctgagat gcgagaatca cttgaacctg ggagacagag cttgcagtga 37980
   gccgaaattg cgccactgca ctccagcctg ggcgacagaa caagactctg tcttaaaaaa 38040
   aaaaaagtgt tttatataca gagtggaata ttatttagcc ataaaaagaa tgaaatcctg 38100
   tcatttgcag caacatggat ggaactggag gtcattaaaa aataaaataa aataaataag 38160
   gaaaaacgta tcaatacttc gattgaccaa aaccagggca aatctgattt tcatctttgc 38220
   aaggggaaca aatttctttt atctcctctg gctttgaaac cctgaaatga aaggaggaag 38280
   ggcagaaaaa agaacacata gcaagttacc atcaggctca gcgcccatcg cattccctga 38340
   gcttgtttcc ttgacttcat cactggcagg actattcaaa aatgattccc tcattcattc 38400
   atatattcat tcattcatca ttccttcatt caacacatac gttttaacac tcatcttgct 38460
   tttcaagcta tagtttagtg agcgaaatgg atacacagaa tacagtgtga gaacagctac 38520
   agggcacatc tgagctagcc tgggatgggt ccggaaatgc ttcctggagc agaggaaacg 38580
   gttgacagcc aagtgttgac agagaagtag tattagccag gcagagacat ggggaatgta 38640
   ttccaggcag aaggcacagt gtgtatgaaa gcttattggt aagaagagtg tgtggcccaa 38700
   ccaggaaaca gacattctga aggcataggg tccacccagg agcatggtga acccagatcc 38760
   ctgaaagatg ggaggtgctc aggcacactt cctgggctag ttgaggggtc tggattttta 38820
   tttacttatt tttttattta ttgagacaga gtctcgttct gtcacccagg ctggagtgca 38880
   gtggtgcaat ctcagctcac tgcaacctcc acctcctggg ttcaagtgat tctcctacct 38940
   cagcctcctg agtagctggg attacaggtg cccaccacca tgcctggcta atttgtgtgt 39000
   gtgtgtgtgt gtgtgtgtgt gtgtgtgtgt tttgttgttg ttgttgttgt tgagacggtg 39060
   tctcgctctt ttgcccaggc tggagtgcag tggcgccatc ttggcttact gcaagctctg 39120
   cctcccgggt tcacaccatt ctcctgcctc agcctcctga gtagctggga ctacaggcgc 39180
   cctccaccac gcccagctaa ttttttgtgt ttttagtaga gacggggttt cgccatgttg 39240
   gccatgctgg tcttgaactc ttgacttcag gtgatccacc cacgttggcc tcccaaagtg 39300
   ctgggattac aggcatgagc caccgtgccc gacctggatt tttattctga agactaatgg 39360
   ggatcctaag gaaggaacca gcctgactga atttgcatat gtgtccacat ctgctggctc 39420
   atggctgtgt gggaggctga gtgatgggga ggaaggatta ctgagtaggg atctagaggt 39480
   gtggcctcat gctttctttc taaccagctg tgttgtcttt gggatggtgc ttaaatttgg 39540
   gctagaccag tgggtcttgg tcacccccca ggggacatct gacaatgtct ggaggcgttc 39600
   ttggttgaca cagtggggtg agggctgcta ctggcagctc gtggggagag accaggaatg 39660
   atgcttaaca tcctacagtg cacagggcag cccccatcac aaggaattat cagctgaaat 39720
   tgtgaatagt gcctacacta gacccttgct actcatagtg tggtccgtag atgagcagca 39780
   ttggcatcac ctgggacctt gttagaaatg ctcttagacc ccaccccaca tccactaaag 39840
   ccagctcttc atttcaacaa actccccatt gatgtgagta cacattcaag tctgagaagg 39900
   gcttctttga ggtgagcctt agtgcccatc cccatttggt ggcgccggat accaagggtg 39960
   tgtgaaaggg gtgggtaggg aatatgggtc tcacctgcca atctgcttat aataacactt 40020
   gtccacaggt gtgttgtaac cgagtgctgg ggattcacca catctccgtc atgcactcca 40080
   tcttcagctt gctgggtctg cttggagaga tcacctacat tgtgctgctg gtgcttcata 40140
   ctgtctggaa cggcaatggc atgtgggtca ctgggcttac cccccatccc cttaacactc 40200
   ccctccaact caggaagaaa tgtgtgcaga gtccttagct ggggcgtgtg cactcggggc 40260
   caggtgctca gtaggcttcg gtgaatattt gttggctgat ttattcagaa attatgtcca 40320
   gcccctacct tggatggatt tatcacctct ccaggccacc tcttctttcc aaataggacc 40380
   acctaggtat agaccaaaga cacgaaatct tctgtgaccc cacaaacaca gagcaggtca 40440
   aataggccca agccaattga gactgtggtt caggtcgtga tgcagagctt tgctgtggac 40500
   gtgctcccac tgcgtactag ctgggcatgc ggcttaacct ttctcagcct cagtcgcccc 40560
   cttgtaaatg gagataagga tactatctcc cctcacaggg ctgttgggat gctactggat 40620
   ttaataagct aatgcaggga catgctaagc acaacccatc cctgaggccc agagaagggt 40680
   gggcctcggc tgaggtctca ctgtgaggtg ggaatgtggg cctccagacc agaggtaggt 40740
   cctgtggccc ctagacagtg gacagcaatg gtcagtttga cacaccagag ccctagccat 40800
   tacttcctgg atgttgtgtg aatattttct ggacatggct tatataaaat gaaaaagtga 40860
   attgggcacg atatagggat agatttttag agatgaactg atagcatgat gataatcata 40920
   ttcactgata acatttacta ctgttattga ctgctttaaa agtgttgggc attgtgctag 40980
   aaaccattat atgcattatc tccttgaatt ctcacaaccg cctactgagg tattctcaga 41040
   ctctaagaaa tgagatttaa gagaagttat ctgcccaagg tcacccggct ggaacctggc 41100
   tgtaaaaatg gctgaagcag gtgatgagga gctgatgtgt ttggacgtgt ctcagagaaa 41160
   tcatggaggc gctggggttc cttccggttc ttggatgcct tctacagaga caaccatagc 41220
   cccaaattat agggatcaca tatcagtggg tgagacatcc ttgcttggga tgaggagggg 41280
   atgagctgtg tgaagcaagg tgcctctgta atgggttcca gtgatgtgtc tgccactgtc 41340
   ttaataactg tgcaattcta agcagaacct ttcctgtctc tgggcctgag agttcccctc 41400
   tgtaagatga ggacttgacc tagcaaggtc ctactcagat gcctgtagag aacaggcagg 41460
   ggaagttaga aaaaaaaaaa gccagtgaag gaagggagct cttcagcttg cacccaccat 41520
   cacagtgcag ggacccaggc tcagtgttgc cagatccaat gacttctcaa gagctcaaaa 41580
   tctagagttt tgcatgtgct ctcccaagta ctggcagaaa attcaagatt gttagtaaca 41640
   ctgtgtggct aaattctgct tgtgggctgc ctagattccc aattctgtga ttctgtggtt 41700
   ctctggaagc attggttctc cacagcacct gcatcacttg gaaacttgtt agaaatgcaa 41760
   gccctaccta cggccccacc ccagacctac ccagttagaa atctgggggt gggacctatc 41820
   agtccatgtt tgaacaagcc ccacaagtgt tctcttgcaa gctcaagttt tagaaccact 41880
   gacctatagc caaaaaagaa aaagccaatc agtggtttgc tggtagagga ttaacttaac 41940
   aactggcttt ccatgaaaat aaagccttga ttggtagcac ttgcaatttc tatggtacaa 42000
   acgcttccca catgactgag ttcaagctat caaggagacg tcactgcaca tggacttggg 42060
   aagagatgag aacaatcagc ccactgagcc tatgggaact ggctccagca catccctgca 42120
   agtcaactct catcagggtg agtgagttga ggaccaagaa gcagttatcc tcttgccttt 42180
   gcaggaccca ggcaaaggga agggcatagt gacagtgatg atctctcttc cggaagtctt 42240
   tggtttgctg agagtaaaag gcgtgggctt caccagtggt gaagccagtc atgcagcctt 42300
   agtcctggta ctcaaactcc ctaaatctca gttttctatc tgtaaaatgg gaaaataagt 42360
   cctatgtcac agggttgctg tgcagattta gcaatagaac atagccccgt tctttatgat 42420
   gactgatgct gcatcagtat ggggacatct ctatgtaatg gaaagatgga gagaggatta 42480
   agtgcaaagt cacagcactt aatgggaact gtggattagc tacttggtgg cattgggcaa 42540
   gtcagttgac tttgcattaa ttccacaaac aatatttccc aatttcctat tcagatgagc 42600
   atatgtgact gagtcagatg ctgtgatcag agccaggatg gagcatttcc cacaaactgt 42660
   gggattttta agtgatggga aggcacactg aaatggcatt gaatcatgca gttgcagata 42720
   ctctttttca attctcagtc ctttgattac atcagggaga aaagaaagtc cccacttggg 42780
   ctgagaatct ctgcaccctt ctagctcttg ttaaccactc ttttgaatag cagagaaaac 42840
   ctcagactgc catatctggg agagatttta gcaacatttt gttttcattg tatctctttt 42900
   tacagctacc tcccatttcc cttctatttc aagctagtaa cacagttttc ttttaaattc 42960
   atttatttaa atgtaaaaat aagtctattt ggagaaaaaa aatttttaat agcatctctg 43020
   gaatgccagt atggctaaat tcatgaatgt tgtcctcaaa tgctgaaatc tgggaagcat 43080
   ctggccaagc tttgtggaca ggccttccta gtttgaatcc caagagccac tcattccgag 43140
   ccacaaaaca ttggaattct tggttcactt ccctaacctg aacttgtcct ctgtgaaata 43200
   gggacattaa tagctcactc acagggctgc tgtgaggaca tgtgttgagc tgagggtctg 43260
   gccaggggag accctgtgca gggagactgt tatcatggtg atggatttct gcttcattca 43320
   tttctttttc cagacagcat catatagaat gagttgtggg gtggcagtca gcaggtttgg 43380
   gtttatcctc tattctgcca cttattactt aaaaaaaaaa acccaactta tatagtataa 43440
   gctatatcca gaaaagtgca aatatcatac aagtaccatt tgatgaatct tctgatatcc 43500
   ccacataacc aacacccaga acctcttctt gtctcattcc aggataacca ctaacctgac 43560
   ttctaacagc atcagtcagt tttgtctgtt tttgtacatt atatatgtga tggtttgaat 43620
   gtgtccccca aatttcatgt gctagaaact taatccttca attcatatgt tgatgctatt 43680
   tggaggaagg gcctttggga agtaattagg attagataag gtcatggggt gaggtatgat 43740
   ggcactggtg acttataaga agagaaagag aaatctgagc tggcatgctc ttgccctctc 43800
   accgtgtgat gacttctcca tgtcatgatg cagcaagaag gccctcacca gatggtggca 43860
   ccatgctttt ggacttccca gcctctagaa ctgtgagcta aatcaattta ttttctttat 43920
   aatcacccag tttgatattt tgtcatagca acagaatatg gacaaagaaa gaaaattaat 43980
   gcaagaagta gagtttttac tgtaacagat tcctgaaaat gtggaagtgg ctttggaact 44040
   gggtgatggg aataggttgg aagagttttg aggagcaggc tagaaaaagc ctgtattgtc 44100
   aagaatggag cattaggcca ggcacggtgg ctcagactta taatcccagc actttgggag 44160
   gccaaagcag gtggatcacc tgaggtcagg agttcgagac cagcctggct aacatggtga 44220
   aacgctgttt ctaccaaaaa tacaaaaaat tagctgggca ctctggcgca cacctgtaat 44280
   cccagctact caggaggctg aagcaggaga atcacttgaa cccaggaggc agaggttgca 44340
   gtgagctgag atcgtgctat tgcactccag cttgggcaac aagagcaaaa ctccaactca 44400
   aaaaaaaaaa aaaaagaaaa agaaaaagaa tggagcatta aagacagttc tacagttctg 44460
   gtgagggctt aaaagaagac cccagaacta gggaaagtct ggaacttctt aatggttact 44520
   gaagtcgttg agatcagaat gctgatagaa atgtggctgg tgaaggccat tctgatgagg 44580
   tctcagatgg aactgaagaa ccacgtgttg gaaactggag caaaggtcat cctttttata 44640
   aagaagcaaa gatcttagct gaactttgtc tgtgccagag tcatttatgg aaagcagaaa 44700
   atccgtaggt cacccatgtt gtagagaatg aaagaacatt ttcagctgag aaaactgaga 44760
   gtgtgaccaa gctaccgatt gataagaaaa ctagtacaca taaattagcc aggcgtggtg 44820
   gtgggcgcct gtagtcccag ctacatggga ggctgaggca ggagaatggc atgaacccgg 44880
   gaggcagagc ttgcagtgag ccgagatcgc gccactgcac tccagcctgg gcgacaaagc 44940
   gagactccat ctcaaaaaaa aaaaaaaaaa aaaggaagaa agaaaattag tacacataga 45000
   acaaagccag aggctgttca tcaggacaag ggagaaaaac tccaaagcca tttcagagat 45060
   cttcaagact gcccctccca ttactggccc agagctctaa gagggcagaa tggtttggaa 45120
   tgaccagctg ctgcccaggg ctgccttggg tctctgctcc ccacatttct ggtgcagcat 45180
   tcctcagcca tcccagctgt ggttcaagtg gccacaggtg tgatgtggaa ggtaaaagtc 45240
   ataaaccttg gcagcataca catggcacta attttgcagg tgtgcagaat gcaaaagctg 45300
   agggggcatg ccttctccca cctacatttc aaagggtgct gtgaacggcc accccagaga 45360
   gcccctagta gagcaaggtc tagtggagct acaagggtgg ggccaccgcc aagaccccag 45420
   aatggtagag ctatcatagt gcaatgccag cttgggagaa ctgcaggcat gagactccaa 45480
   cctgtgcgaa gtgcaacatg ggcagaaccc agcaaaacca caggggcaga gctccccgaa 45540
   gcttcggggg tccaaattcc atagtgtgtc caggaggtgg cacacagagt aaaagatcat 45600
   tctgaaggtt taaggtttaa tgttgttttc tatgttgggt tttgtacttt cctggaacca 45660
   gttacccttt ttcccttgcc tctttttcct tttagaatgg gaatgtctgt cctatgcctg 45720
   ttccactgtt gtattttgga agtcaataac ttgttttgac tttacaggct tacagccaga 45780
   gggaatctcc catagaatga attgtacctt aagtctcacc cacatctgat ttagatgaga 45840
   ccatggactt tggaattttg agttggtgct ggaacaagtt aagactttgg gggttgtcta 45900
   agtgtggtgt ttcatgcctg taatcccagt gatttgggag gctgaggtgg gaggattgct 45960
   tgagcccagg agttcaagac cagcctaggc aacatagtga gacctgtctc tacaaaaata 46020
   aaaataaaaa gttagccagg tattgtggca tgtgcctgta attctagcta ctcaggaggc 46080
   tgaggtgaga ggatcacttg agcccaggag tttgaggctg cagtgagcta tggtcgtgcc 46140
   actgcattcc agccagggca acagagtgag actctgtctc tacaaataag attaaataaa 46200
   cgtagctgga gatggtggca cacgtctgta gtcctagcta ctcaggaggc tgagacagga 46260
   ggattacttg agccaaggag tttgaggctg cagtgagcta tgatcatgcc actgcattcc 46320
   agcctggatg atagagcaaa atcccatctt taaaaaaaaa aaaaaaaaaa aaaaaaatat 46380
   atatatatat atatatatat atatatatat atatatatat actttggtgc tattgggatg 46440
   aattttgcat gtacgaagga catgcatttt gggggctggg gcagaatgct atggtttgaa 46500
   tgcatccctc aaatttcatg tgttggagac ttaatctcca aattcatatg ttgatgaaat 46560
   tggaggtgaa gcctttggga ggtaactagg attagataaa gtcatcaggg tggggcccct 46620
   atgatgagac tggtggctta caagaggaag agagacctga gctgacatgc tcttgccctc 46680
   ttgccatgtg ataccctctg ccatgttatg gcacagcaag aaggtcctca acagatgcca 46740
   gcagcatgct cttagacttc ccagcctcca gaaccatgag ctatatataa ttattttata 46800
   aattacccat tctgtggtat tctgttatag caacagaaag tgaactgaga taatatacat 46860
   ggaatcatac agtaagtctg tgcttttgta tgcttctttt actcaacatt gtagttgtga 46920
   gattcatcca ggttgttaag cattgctgta ctttttttcc actgggatat agtgttctgt 46980
   catgcttggg tcttaattta taaaggtgac tgagtggcat tttcttccag tattattgga 47040
   aggaaagttt tgttgttcac agttcccctg taaaaaagag gcagaacacg tcttgcaggg 47100
   ccacacaaaa ctgtgtcatc cagggaccag gcagcagaaa gagaggggga actgggccta 47160
   tgcctttatg aaaaagagtg gtgggagagt aactgggtga gggcatccac taatgggcag 47220
   gaagtgaaaa cacatatgtt ggaatttgta gctgaggggt ttataatatg agtttcccat 47280
   gcctgagaaa gctgacttgc aagaaaacga gataaacaac tttggccatt agtgtggccc 47340
   tgtcataaat gaatgccgga tagacaaatc gagaatctaa gaaaagatag ttggaacaag 47400
   tgttccattg tgtgaatgca gcagaattta tttatccatt attgaggagg atttgggtag 47460
   tttccagttt ggagctatta tgaatattct agtattgctc ctctgaacat tctagcactt 47520
   ttgtttttgg agcacacgaa tgcacttctg ttgattatat gcctagaagt gaaattgttg 47580
   agttatacag tattcacaca gtcagcctta gtggctactg ctaaacagtt ttctctagta 47640
   gtttgcgcca atctaatcac cagtagtgta tagaagctcc ttttactcca cattttgtta 47700
   acacttggtg ttttccttct ttttgattag tcatttagca gtgaaaccta ttttttacat 47760
   tttgatatct ccaataacta actaaatgga gcacttttaa tatgcttttt ggacagttga 47820
   atatcttttc ttgtgaaatg tctattcaag ttagtttgcc cattttctat tgtggtgttc 47880
   tgtctttttc ttattgattt taggaattcc ttacatatcc tggatatgaa tcccactatg 47940
   tggcttacct ttttccttct ttctttttga aacagagtct ccttctgtca cccaggctgg 48000
   aatgcagtgg cgctatctca gctcactaca acctctgcct cccaggttca agcaattctc 48060
   atacttcagc ctcctgagta gcttagatta caggtgcatg ccaccatgcc caccgaattt 48120
   ttgtatagac aaaataattt ttggtagaga cagggttttg ccatgttggc caggctgatc 48180
   ttgaatccta gcctcaactt tggcccacct tggcctccca aagtgccagg attacaggtg 48240
   tgagccacca tgcccagccc accttttact ttcttaatgg tgtcttttga acaaggaggt 48300
   tcttaatttt aatatagccc aatttatcat tgttcccttt atgcttagtt cttttatgtc 48360
   ctgtttaaga atttttgcag ccagctcggt ggctcacacc tgtaatccca gcactttggg 48420
   aggctgaggc tggcagatca caaggtcaag agatcgagat catcctggcc aacatggtga 48480
   aaccctgtcc ttactaaaaa tacaaaaaat tagctgggcg ttgtggctct tgcctgtagt 48540
   ctcagctact cgggaggctg agatcacgcc actgcactcc agcctggtga cacagcaaga 48600
   ctccatctaa aaaaaaaaga aatttgcaag gtcatgcata tgtccccctg aatttttttc 48660
   taaaaatcac ttaattttag atcaatgaat tgagtaattg actccatttt tcagtcattc 48720
   aacaaacatt tccctgaggt tttgataacc tgaactgtgt ttggagctgg ggaggaagca 48780
   aactattgaa tatatacaaa gatggcaaag atgagggcct ggagcttgcc acacggaagg 48840
   ggggatggct gcctgaatgg ttgggcaggt agttgttgac atctgcactc cctacaagag 48900
   cagcagggtg gcaactcttt ttatcttttt aatttatttt tcttttctct tttttttttg 48960
   agatggagtc ttgctctgtt gcccaggctg gagtgcagtg gcgtgatctc agctcactgc 49020
   aaactccacc tcctgggttc acaccgttct cctgcctcag cctcctgagt agctgggact 49080
   gcaggcacct gccaccactc ccggctaatg ttttgtattt ttagtagaga aggggtttca 49140
   ctgtgttagc caggatggtc tccatctcct gacctcatga tccacccgcc tcggcctccc 49200
   aaagtgcggg gattacaggt gtgagccacc acacccggcc ttaatttatt tttctagtct 49260
   gcaggtaatt ctttttaatt ctctccactc tcctatgatc ttatgaggta gggactgtga 49320
   ttatttctcc cactttataa tgaacaatca gtaaagacag ggaagataac caaatgacat 49380
   acaaggtggg gtccacccca tgaggctgca ggcttggagc tttcctttgt cttaaaaatg 49440
   agaacatgag ctgcccacct gttgagacaa gaaataggaa aggcttaaaa aactggcttg 49500
   ttgtgtacaa ctatccgtgg ggctgcagtg aacgggctgg cagtgcccag gtgcatgctg 49560
   aaccctggga caatcacatt cagcatccag gggcccccgt aatagcttaa tgtttgaatt 49620
   gaacccctgg ggttgccttg aaggagagag atcctggaag tatgttcaag gggtagggat 49680
   gggcagggga gatgggtctg aaagccaagc tctaccccac ccaccttgcc ccaagagaaa 49740
   tagaaccttc atctttaatt gcctaacgag aaaactgggg ctggccagat gtggtggctc 49800
   atgtctgtaa tcccagcaat ttgggaggcc aaggcgggca gatcacttga ggtcaggagt 49860
   tcgagttcag cctggtcaac atggtgaaac cccgtctcta ttaataatac aaaaattatc 49920
   caggtatggt ggcgcatgcc tgtagtccca gctacttgag gcacaagaat cgcttgaacc 49980
   tgggggacag aggttgcagt gagccgacca ctgcactcca gtctggacga cagagtgaga 50040
   ctccatctca caaacaaaaa cagaaaaaaa aaaaaaaaaa agagagagag agaaaactgg 50100
   aggctctgag aggttaaagg acttgcccag ggtcttgcag ctagtaagtg acagagctgg 50160
   gacttgagct tgggttttct gactcctggt ctggttcatt atccatgagg tgctgggaac 50220
   taaaataagc cacaatcttg gaatctccgt cgcctccctc cctcccacat gtctgcgtgg 50280
   ctttttggga aaatgccagg ggaatgtacc agccagggag aggacccttg ttttcctcat 50340
   ggcccttcct ggcaatggca ctactgacac cgacagtcct ttttgtccct gatgacctct 50400
   gctgcctgat gcccaagtga ccacctctgc tttgtcattt ctaggattgg cttccaggtc 50460
   ctcctcagca ttggggaact cagcttggcc atcgtgatag ctctcacgtc tggtctcctg 50520
   acaggtcagt gtgaggccac ctttcttcca ccattgccag gacacagcac ccacgtccag 50580
   agcgcaccct gccgtgtggc tggatgtcta tgtgccccat ctccttccct gaggatcaca 50640
   taatttcaga attggaaagg ttcttagagg tcacctgctg ctaatgtgga ctgtgaggcc 50700
   agggcaggga agggacatcc ctgaggttat aagtagggtg agtggcaacg ttgcagactt 50760
   ttgaacccag ggctggtgat cacactcagt tttgcacaga agcccgagaa aatccttaca 50820
   cccaaaagcc taccttttat ttctgaggac acccataata ctattttatt caacagatat 50880
   ttattcaata tccactatga gccaggcact ggggacacag cagtgagcaa aacaaattcc 50940
   ctgaccccat ggaattgacc ttctagtggg ggaaggtatt agcaataaat agacaaataa 51000
   gtgtctacta cgccagatgg gaagaagtgg ctgtgaagac agagcaaact agagaaacat 51060
   agagtcaatg tgggatgggg tgttctttta ggggggtggt cagggaaagc ttatctgagt 51120
   agttagcttt taagcagaga ccccaatgaa gaggagggag atatgcgatg catttagtta 51180
   ggggaagaac attccatgaa aataggatag caagtgcaaa ggccctgaga cagcagcatg 51240
   ctttgtgtgt tgagggaaca gtaaggagac cagtgtggtt ggtgtgaatg gagtgagaag 51300
   gagcagcagg ggttgagggc agaatggtag tgaggagcag gcccttataa aagatgggaa 51360
   gccactggag atctttcaac aaaggggaaa agtatgtttc tgttcttgca atacaataga 51420
   aaagcaaaaa atctagggga gttgctaatt agccagtttt acttatatgc caggtgaaaa 51480
   tatgtggcta ggtgcagtgg ctcatacctg taattgcagc agtttgggag accgaagtgg 51540
   gcagatcatc tgaggtcagg attcaagacc agcctggcca acatggtgaa accctgtctc 51600
   tactaaaaat taaaaaatta gccaggcgtg gtggtgggca cctgtaatcc cagctacttg 51660
   ggaggctgag gcaggagaat tgcttaaacc cgggaggcag aggttgcagt gggccgagac 51720
   tctgtctaaa aaaaaaagaa aatacacatt caggccaggc acagtggctc acgcctgtaa 51780
   tcccagcact ttgggaggct gaggcaggta gatcacctga ggtcaggagt tcgagaccag 51840
   cctgaccaac atgggaaaac cctgtctctg ccagaaatac aaaaattagc caggcgtggt 51900
   ggtgtgtgcc tgtagtccca gctactcggg aggctgaagt aggggaatgg cttgacccca 51960
   ggaggtggag gttatagtga gccaaggttg caccagccta ggtgacagag tgagactgtc 52020
   tcaaaaaaaa aaaaaagaaa gaaaatatac attccatcca gaacttgtta ttctacaagc 52080
   aaacatcttt tattggttag acacccatat atgtgtccct aagcaggagg tggatgccaa 52140
   ataagagaca aatggcgtaa gacactatga gttgtgtggt gacattgggc atgtcacttc 52200
   actccctctg agccttggtt agcttctctg taaaatgaaa ggattatggt aactaagctg 52260
   gcttccttcc agctttaaca aactgtatgg aggtacattt tggagttact tgggtaattt 52320
   ttgagtgtga gattggctag aattgcttta atataccaat gtctggcctt agcttttggc 52380
   agagtctgtg tgaagaagca gaggcggagt agagttaatt ccgtaagtta acgttcagtt 52440
   cgtggcagct ggcaatccaa ccctgggaaa ggctgccgga tttagcaaaa atgcaaggtg 52500
   tctgttttta aattcgcaat gaattgggta tcctgcattt tatttggcaa ccctgtcctg 52560
   ggactcacac tattcactgt tatcactggt atattcgaag tggtgctgac ttgccctctg 52620
   tcttgcaaag tacccggggg tcttttctta tgcttcactg gagtcaaaaa agagaataga 52680
   ggaaaagaca atcatattgt tcctttaaga gttaagacca acaagctttc ttctttacat 52740
   gttgtttttg acatgagcaa actggtgatt aaaaacaact tgggtggctc atacttgtaa 52800
   tcccagcact ttggaaagct gaggtgggag aatagcttga ggccaggagt tcaagccagg 52860
   gcaatcctat agtgagaccc catctctaca aaagatacaa aaattagcca ggtgtggtgg 52920
   tacacctgta gtcccagctg ctccggaggc tgagatggga ggatcagttg agcttgggag 52980
   gcagaagttg cagtgagctg agatcgtgcc actgcactcc agcctggaca acagagcaag 53040
   accctgtctc aaaaaaggaa acaaaacaac ttggacaatg gaagggggag aaagttcctc 53100
   aagaagccaa aattgcacca aatggactcc cagaagccaa gcatttaact tgttaattga 53160
   gccctctgtg ggcctgtcta tacttattta aggaacaatc ctatcaagca tagttattgg 53220
   gtttctcagc ccaggtagat tagaaatagc agattagagg tgggctaggt ttctagaggt 53280
   aaagtacacc agcagaagtt agaagtgaaa gcaaagagcc taacagagga agagaaattc 53340
   tttttttttt ttttagacgg agttttgctc ttgttgccca ggctggagtg caatggcgct 53400
   atctcggctc aacgcaacct ccgcctcctg ggttcaagtg attctcctgc ctcagcctcc 53460
   tgagtagctg ggattacagg catgcaccac cacgcccggc taattttgta tttttagtag 53520
   agacagggtt tctccatgtt ggtcatgctg gtctcgaact cctgacctca ggtgatccgc 53580
   ccaccttggc ctcccaaagt gctgggatta caggcataag ccactgtgcc cggccaacaa 53640
   attcttaaaa ctggacacaa gaacacaaaa cgcttgggct gctgagagat tagaccaaca 53700
   accctccacg gctacaaacc ttttccacgt tatatggcac gttataagtg ggtgttccta 53760
   gtgatggttc tgattttttt tttaaaaagt ctaaatatgt ttaatgttgt ctcagaagac 53820
   aaaatatatt ttagacagat attcctcagt gatgagtaag cctcagctat ctggaaaatt 53880
   catgcaggcg ccagagatca ttactgagta attcaagcta ataactgcgt catgctggtt 53940
   gtaccctgca tgccaatatc tgctaaaagc agcaccacga aagggaaata cgaatctcac 54000
   taagcactca cccattcttg ttaacgacac tggaactgat catccttaat aatacacaga 54060
   taaatctatc aggagcattt ccttgcttcc tgtgaaagga agtactcatt ccatgtgtcc 54120
   tgtgaaattc agccagcttc gggaagctgg aggaatacat atggccaagc tacctgggca 54180
   gagagtagac agggaatgga ggttgggcac agtggctcac acctgtaatt gcagcccttt 54240
   agaaggcaaa ggcgggcaga tcacttgagc tcaggtgttc aagaccagcc tgggcaacat 54300
   ggctaaaccc cgtctctgca aaaaatacaa aaaaatgagc tgggtatggt agcacacact 54360
   tgtggtccca gctacttggg aggctgaggt gggggggttg cttgtgcctg ggagtttgag 54420
   gctgcaatga gctgtgattg tgccactgca ctccagcctg gataacagaa tgagaccctg 54480
   ttccaaaaat aaaaaataaa atcaaagaca cttaaaaaga tggggaaaag gaaggacagg 54540
   cacttaagca agttataagc tactttccta actacacaag tggaatctta agctgaggtt 54600
   cccaggagtt gactggagcc agagaagaca gacctatagg agcacccaac tggagtcgcc 54660
   ctccatagta gcccatatgt cttacatgga tcagctttcg tggggccctt ctactccgtc 54720
   tggggaaggg cgtcagatct gtggctctca tgtactgctc agtacactgc cattcccagt 54780
   tctttttttc aaaaaaaaaa aaattgttta cagaatcggc cgggtgtggt ggcttatgcc 54840
   tataatacta gcaatttgga aggctgaggt gggtggatca cctgaggtca ggagttcgag 54900
   accagcctgg ccaacatggt gaaaccccat cctactaaaa aaaaaaaaaa aaaaaaatta 54960
   gctggatgtg gtggcaggcg cctataatct tagctacttg ggaggctgag gcaggagaat 55020
   cgcttgaacc tgggaggcag aggctgcagt gagccgagat catgccacgg tactccagcc 55080
   tgggtgatag agtgagactc tgtctcaaaa taaataaaat aaaataaaat aaaataaaat 55140
   aaaataaaat agtctacaga attaagctgg tccaggaatg acagggcgtc catttatttg 55200
   tctttcaatt gtgggagaaa aaggatttct gttgagacac tgtcgttttg acacacacaa 55260
   tattttgatt aatcttgaga ttaaaaatcc tgtgctccaa atcttttaac attaaattat 55320
   gcatttaaac aggtttgctc ctaaatctca aaatatggaa agcacctcat gtggctaaat 55380
   attttgatga ccaagttttc tggaaggtaa gatttttcac ctattaacgt gatagatttt 55440
   gagtgcatga acttaaaaac atacctgggt atatatgttg acttgctgtt tatgagtaaa 55500
   acaaaaacaa aaatggagta aggagcattg caggaggaac tagaggagaa acaaatccat 55560
   gatatgcatg tgtgtggggg agggtggcgg ggaggtggta aaggtcacca tttccctgat 55620
   acctcaaatt cattcagagt cagggatgag acagctttca ctggccacac ttcccctccc 55680
   gctatctgca gtcctcagcg tagccaaata gtttgacatg cgggtgacag aaccccgcaa 55740
   tgcaaaagct ggaagaaacc tcaagccttg gagtccaacc ccttttttga cagatgctaa 55800
   gagtggagac atgacttatc aagatcttac aactggctgg gcacggtggc tgacgcctgt 55860
   aatcccagca ctttgggagg ctgaggtggg gcgatcacct gaggccagga gttcgagacc 55920
   agcctggcca acgtgtcaaa accccatctc tactaaaaat acaaaagtta gctgggcgtg 55980
   gtagcacatg cctgtaatcc cagttactca ggaggctgag gcaagagaat cgcttgaaat 56040
   caggaggcag aggttgcagt gagctgagat tgcgccactg cactccagcc tgggtgacaa 56100
   gagctgacac tctgtctcaa aaaaaaaaaa aaaaaaaaaa aattcttaca gtgtgtgagt 56160
   atccaggctg agtcctgaac acagctcttg ataaatgata acaagcaggc acaaaaaaat 56220
   tgtagtacag gagtctgagg tcacttagca aagggacata aagttcaaac agctcagcag 56280
   ctgctgaggg tcccgtgtta cattgtagca tttgttgttg tgactgggct agaaagaagg 56340
   tgaagaaggt tggagctcac tccctgcctc ccctcccact ctcctccctt tgacctacac 56400
   tcatagttca cgcagcactc tgatgtgtcc ccttaggcca tcctctagtc aatgctgtgg 56460
   gtaggctgga ccagcaggga ccagtattgt cacagcaagt ccaggccaac agtggtcagg 56520
   ctgctgcccg gtgttgtgcc tttgtgagtg gcagatccaa gaccggaacc caggccttct 56580
   gagtcccagg ccaatgcttg ccccacccag catccaagat gttgctcact aaagagacag 56640
   agaagcagcc ttattatggg cctggacacc tgtgcatgag gggtcaagca gagaggacct 56700
   ggggagagac cctgcccctt cttttccttc tccttcctct cctttctctt cttcttcctc 56760
   ttcaaatagc tttttgaggt gtaactggca tacaatcaat tgtacatatt taggctgggt 56820
   atggtggctc acgcctgtaa tcccagcact ttgggaggcc aaggcgggtg gatcacttga 56880
   ggtcaggagt ttgagaccag cctgggcaac ccggtgaaac cccgtctcca ctaaaactac 56940
   aaaaattagc caggcgtggt ggcagctgcc tgtaatcccg gctactcggg aggctgaggc 57000
   aggagaatca cttgaacctg ggaagcgaag tttgcagtga tctgagatca tgccactgca 57060
   ctccagcctg ggtgacagag cgagactttg cctcaaaaac aaaacaaaac aattgtacat 57120
   atttaaagtg ttgtaaccaa gtgagttaca gagaaacacc acactttgag cctaattcag 57180
   gagtccttta ttagccggcg acctagagac gactagtgct caaaattctc tcggccccaa 57240
   agaaggggct agattttctt ttataccttg gtttagaaag gggaggggga attgagctga 57300
   agcaatctta cagaagtaaa acaggcaaaa aagttaaaaa gacaaatggt tacaggaaaa 57360
   caaacagttc caggtgcagg agctttaaag ccatcacaag gtgacaggtg cgggggctct 57420
   gggtgctatc tgccggacac aaacgcaggg gcactagagt actatcaccc gggcaaattc 57480
   ctgggaactg cggacacagc ttgccacagt accttatcag ctaattgcac tctttgatgt 57540
   gctgggagtc agcttgcaca agttaagtcc ttgaggaagg gggtgggtaa ggagccctta 57600
   acgtcttgca aatgaaggag ccgaatggaa tccctccggc tttcttagct aagagagagt 57660
   caatcaagtt aatacaagtt agggtatcac aaaagtatat aatttgatac attttaacgt 57720
   atttatacac tgaagagacc atcaccacca tcaagacaag gagcacaccc atcacttcca 57780
   cacacttcct cctgctcctt tgaaattcct cccttcctac ccacctggtc ccacccaaag 57840
   gcaaccactg aactactttc tgtcactaag gtttgcgttt tctgtaattt ttttgtttga 57900
   gacagggtct cactccgcca cccacaccgt aatgcagtgg caccatcatg actcactgta 57960
   gcctcaacct ccccaggctc aggagatcct cccccctcag cctcctgagt agctaggacc 58020
   acaggtgtag gccaccatgg caggctaatt tttgtatttt tttgtagaga tggggtttca 58080
   ccgtattacc taggctggtc tcgaactcat gggttcaagc aatcctcctg ccttggcctc 58140
   tcaaagtgct gggattatag gcatgagcca ctgtgcccag ccctctgtaa tgttacacaa 58200
   agggaatcat gcagcacgta ctgcccttgg tctggcctct tttgctcagc atgattattc 58260
   tgagaatcat ccgtgttgtt gcgtgtaact gacttcatca gcttctctct gcagctgtca 58320
   gctcttggct tctcccaaca gccaatctct ctttatcccc tgcaagtgtt cttgcctatt 58380
   tagcagaatc aaggtactct atcgaaaaga ctcggaaaat tggtttaatc tattcattca 58440
   ttcctcaggt atttatcgaa taactattct ataccaagta ctatgctaat caaccaagga 58500
   cagcacaaac aggagaaatc tccagctcag tcacttgagt tgcaataaat atttgctgga 58560
   taggtcaggt gcagtggctc acacttgtaa tcccagcact ttggggatta ctgagacggg 58620
   aggatctctt gagcccagga ggccaaggct gcagagaacc atgatcatgc cactgcactc 58680
   cagcctgggt gacagagtga gatcctgtct ctgaaaaaaa atatttgctg gataaattaa 58740
   ggaaatctga cgaaccccat cagtagccat tgcagcaaca ggtaaactag aacgagtgtg 58800
   aatttggaat gaggaaaccc gatgttggcc atcattctgt aatgtcatgt attatgtaat 58860
   gtattatata ttaatgtatg tattatgtag gcaagttcct tgacctctct cactggtaac 58920
   ataagagtag taatctttgt gctacttcac tgggttattt caaagatcaa gtgaggtaat 58980
   aatgtctgta acaacattct gtaaaatgca aaccgccaca tgaatgtgaa agtttattac 59040
   tagggattta gccaaccaca agggaatgtg tgagcataag agctatcata ttgcaagcct 59100
   acagtttctg attttgtgct aggtgctttt ccacattacc tgattttatc ctcacaacag 59160
   tcctgcataa aagtaagtat gtcgcccagg tgcggtggct catgcctata atcccagcac 59220
   tttgggagcc cgaggtgggc aaatcacttg agatcaggag tttgaaacca gcctggtcaa 59280
   cgtggtgcaa ccctgtctct actaaaaata caaaaaaaaa ttagacaggc gtggtggtgg 59340
   atgcctgtaa tcccagctac ttgggaagct gaggcaggag aatggcttga gcccgggaga 59400
   tggagattgc agtgagatga gattgcgcca ctgcactcca gcctgggtga cagagcaagg 59460
   ctatgtctca aaagagaaaa aaaaagtaag tatctcagtc ttgaagatga tgaaatggag 59520
   gcctagagag attaagtaac ttgcccaaaa tgacagaact aatgcataga aaagaagaaa 59580
   tgtgatgtct tttggctcca aagacacccc acatatgcgt tggttacagt tactagagaa 59640
   aagttattcc acccccaccc cacccccaga aatcttctga cttgttttct cgcagttgag 59700
   taggaccatt tattcggcag tgtaccattc tcagcttgca gttgaaagcc aaatatccat 59760
   taaagaggca aggatgcaaa cttgctaagc tgataaatcc aggggtgatt tttttttttt 59820
   ttgcaaacca tccaacaaga cattttaaat actcattgaa tttcatagaa ctgactgcca 59880
   ggattggaaa gacattaaag ccagctcagc cactgcctcg ctggttggcc agaccacgcc 59940
   tggcacttct gggagggagc actcaccacc ccccaagggc acccatctca tcctccgaag 60000
   gtttatgaaa atgcactcat catttgctaa ttcattccac tacgtgtatt acctaatttg 60060
   tgacacgatg tgaagtacca gagagataat tctaaataaa atatagttat gggtctcaag 60120
   gagccagata tgctaatctc ctatcctcct gcagtttaca gtggtcctca ccagatactt 60180
   atttacaaaa attcagttta ttatttattt ttttgagaca gagtcttgct ctatagctca 60240
   ggctagagtg taatggtgtg atctcggctc acttcaacct ctgcctccca ggttcaagtg 60300
   attctcctgc ctcaacctcc caagtagctg ggactacagg cacctgccac cacggctaat 60360
   ttttggagtt ttagtagaga cagggtttca ccacgttggc caggctggcc tcgaactcct 60420
   gacctcaggt gatctgccca catcagcctc ccaaaatgtt gggattacag gcgtgagcca 60480
   ccatgcccgg ccaaaacttc agtttataac acaatctttc acgtgtcttc tgctttcatt 60540
   aaaagaatag acagttccct tctttatttc agtttaataa accatggatt ttatttcatg 60600
   ctttgcaaaa cacaagggct cactgacatg cacttcttaa actaattctg gctggtcgcc 60660
   tgtaattcca gcactttggg aggctgaggc cgacagatca cttcaagtca ggagttcaag 60720
   accagcctgg ccaatatggt gaaaccacgt ctctaccaaa aatataaaaa attagccagg 60780
   tgtggtggtg cgtgactata atcccagcta ctcaggggcc tgaggcagaa aaatcacttg 60840
   aacccgggag gcggaggtta cagtgagctg agatcgcgcc actgcactcc agcctgggcg 60900
   acagagtgag actctgtctc aaaaaataaa taaatacaaa taatgtaaaa tacgaaacaa 60960
   gcaatcctgg cagtagctgc tggaatgaga ggagggagag gtcataggga ggtcggggac 61020
   aatggagcat ggagttgtgt tggatttggc taagcagcag gaagtgcaag gcattccaag 61080
   caagaggagg ggggcaggtg gggagcatct gcaagaacag aagcagcatg agcaacctgg 61140
   ctcggcagtg tgtgaaaagg ctgaaaggtg gctagagcca cttcaatttc atccttcagg 61200
   caaatgggaa attcccaaag gtttgagtgg ggaagcaatg cctacaatga aagtttgaga 61260
   gtgaagcaga gtgatcgaat taagcatgta ggccgagttc tgaaataact gcaatgtgct 61320
   gaagatcatc cattggcttc tgaatgagta tttgcagttt attttttaaa atgattttat 61380
   tgccaagaaa gataaacact actgttttgg tacaaaaaca taacaaaatg tgttgagtcc 61440
   ctcttgctgt tttacgcgaa gttttaaaaa tctactcttg tcacagtggt atcaccccta 61500
   cttctgattt caaataaatg ttctagagac acagtaaggg cccaacaaac gcttgttcaa 61560
   caacacaagg agagccagct tttaaagtag gaaaacaggc cgggcgccgt ggctcacacc 61620
   tgtaatccca acactttggg aggctgaggt gggcagatca cttgaggtca ggagttcaag 61680
   aacagcttgg ccaacatggt gaaaccctgt ctctactaaa aacacaaaca ttagccaggc 61740
   gtggtggtgc acaccagtag tcccagctat tcaggaggct gaggcaggaa aatggcttga 61800
   actggggagg cagtggttgc agtgagccga gatcgtgcca ctgcactcca gcctggggga 61860
   cagagggaga ctccatctca aaataaaaca aaacaaaacc aaatcataca aaaacattag 61920
   ctgggtgtgg tggtgcatac ctgtaatccc agctacttgg gaagctgagg cagaattact 61980
   tgaacccctg gggggaggtt gcagtgagct gagatcttgc cactacactc cagcctgggc 62040
   aacagagtga ggagactctg tctcaaaaaa tatatatatt aaaaaaaaga aaaaaaaaag 62100
   taaactagga aaacacatca gcagcctgcc aacagactcc cctagcctcg gtgagggcca 62160
   gtgttctggg aggcagatct gaattctagt cctagttcac ccactggcag gctggtgccc 62220
   ttgggcaggt cgcttctctg gggctcagtt tcttcctcta taaaatgaga tcaaatccca 62280
   tgttctaaga gtttgtgctc tggagtcaga cagatctggg ttctaccact gccagctctg 62340
   tgatcttgta gcttcagtct cgtcatctga catggagata acagtaactg tctcactgtg 62400
   ttgttagggt ttaaaggaga taatgtatgt gaaatgttag caaacaagtg ttagctaccc 62460
   tgatttccgg tttcagagtt ctgtggtccc agtttatgcc acatgcagtg acgttgtatg 62520
   gtaggctgtg gtgtggcacc acttcagaac tcagcgcatg cacagcttgc agaagagaag 62580
   gccagaggag acctaagaag gctcttcgaa cacttgaaag accggcatgt aggccgggcg 62640
   cagtgactca cgcctgtaat cccagcagtt ttggaggtcg aggcgggtgg atcacctgag 62700
   tttgggagtt tgataccagc ctgaccaaca aggtgaaacc ccgtctctac taaaaaatac 62760
   aaacattagc tgggcatggt ggcgggtgcc tgtaatccca gctactccgg tggttgaggc 62820
   agaattgctt gaacccggga ggcagaggtt gcagtgagct gagattgcat cactgcactc 62880
   cagcctgaga caagagcgaa actccatctc aaacaaaaca aacaaccaac caaacaaaac 62940
   caaaaaaaaa actggcatgt agaagaaaaa tactttttct ctacacttct ccaaagaatt 63000
   taactaggcc caggggaggt gcagtataaa tttctaacaa tctcaactgt ctgccaaatg 63060
   gaatgagcta cttcatatgg cagtagtgag tcctctgtct ttggaggcat tcaaataaaa 63120
   gccagatggc catttatcaa caatccatgt aaaacgttag atgaaataaa acctatatat 63180
   ccaagatctc ttccaattca gattttatga aagaatttct aaggtctttg taatgagaca 63240
   tttaggctgt ttcaagagat caagccaaaa tcagtatgtg ggttcatctg caataaaaat 63300
   gtttgttttg cttttacagt ttcctcattt ggctgttgga ttttaagcaa aagcatccaa 63360
   gaaaaacaag gcctgttcaa aaacaagaca acttcctctc actgttgcct gcatttgtac 63420
   gtgagaaacg ctcatgacag caaagtctcc ttatgtataa tgaaacaagg tcagagacag 63480
   atttgatatt aaaaaattaa agactaaaaa cttagtttaa gagtcaattt aataagttta 63540
   aaataaatgt ttagtttcat taggatgatg ctatcaatat tttcttggtt acagacacat 63600
   tattaaagtt ttgggttaat tttattgaca attcttaaga ttctttctca tgcttaataa 63660
   agcatgctac tcagttaact cttgtctaca tcagcaaagc agataataca aaacaggaaa 63720
   attacaaatc actgatactt agtccttgtg ggaatcatgc ttttctccca gcagttttac 63780
   aaggtggctg gcattccctg agcatattct gaattgcact gtggggaaag aggttgtgct 63840
   cagttgtagg gtggggggat gcactgcctg aggattaaaa aactagttct gtgaccgtga 63900
   ggaagtcgtt taaatttcca tggtctgttc cctcctatgt gaaaagagaa ggtgggcttc 63960
   aacctctaag atcttctcca gttttcacat tttatggact tttgtagaaa aaacatcagg 64020
   agttcatgtg ggatgacagc aagtcatttc tttgaggaga gtcttgatca ccaggcaata 64080
   ttcacagtgt agagactgtc agatgaccat ggctagcatg gaaatgagac ccacacattt 64140
   aaatcaccca gcaaatattc cgaaggctaa ttgtagcaca ttttatgaaa gacatttcaa 64200
   actgtggtcc tgaagagtgt atcccatctt gcagaggtgg ggagcctggg gggacaagag 64260
   ttctgaagag gaagagacaa caagagttcc cagtagctaa tgtttgtcat tctagttgac 64320
   cgtgctggtc tattaggcta gtggttcagt acacagatga aatgcaacat ggaacccagt 64380
   ttattatcag aacaactaca aagaaattgt cccctgtcta agactggagt gtcaagtctc   64440
   tgcccttttt tcctttcctt caatggtgga tgtggagtga ctgtgcatcc caccagaacc   64500
   acgtgtcatg gctgagtcac atcttcctgc ccttggaatg agaggcacag cggaagacct   64560
   tcccatggaa gggacacagg gagcctggtg gctggaccat ggtgcttctc tcttccaaca   64620
   cgtccactca ccccttggga gaccctcaaa agccagttac attacatgtt cacagaattt   64680
   ttggtaaaag taaataccaa ttatagtgag gaagaatttt gaccacggaa tattttaaaa   64740
   actaaaaaat gtttatattt catttaacat ttgacacaga agagaccaca tttgaataaa   64800
   cacattaaat cttcagagca ctttcattgt ggttttggac ctcagatatg acaaatactt   64860
   acattgacaa atccataatt tcttttgtaa tttcttttta tttttacaaa ttataccatg   64920
   ataaaatttg acaaaaatta ttcatgtgaa agtttcctct aacattttat aagttaatca   64980
   agtgcatacc acaatagatt tttggttgtt gtttaggtgt tctcgtgatt ttagtattac   65040
   acaactttaa gctgagacta cactcagaaa taagtttaga aaatggcatt acaaaaggtt   65100
   gggagtgagc agtaaaaaaa caaacaaacc catgcagggc tgttgtgctg tgggaaatca   65160
   gatgtgttca ctgccataag tcttcagtgc ggccaaactt aaaaaccagc cctctgtgaa   65220
   taaaacaaga aatatcacat gactccctga atttgagaaa agagtatgtg agatttcgag   65280
   aatggtgtga aacaaacaac gaagaataat tgatgagttg tagaagaaat tttggtacga   65340
   aatgtatcaa aacagaaact gatcattcta aggtagtgaa ttcttccatt atgttcaact   65400
   gtgctattaa ccaccatatt cccaacaacc ttaactttca agtactgaat acacatgtga   65460
   cttttaaaaa gttaccagtg tttactatgt aaccattata tgtctgattt tttttttttt   65520
   ttttgagaca gagtcttgct ctgtcgccca ggctggagtg cagtggcgtg atctcggctc   65580
   actgcaagct ctgcctcccg ggttcatgcc attctcctgc ctct                    65624

## Description

### Field of the Invention

The invention relates to methods for genotyping and blood cell antigen determination, which in particular may discriminate the *RHD*DIIIa-CE(4-7)-D* or *RHD*DIIIa-CE(4-7)-D*)*-*like blood type variants, which express the C^{+W} antigen and lack a D antigen, from *RHD*DIIIa, RHD*DIV*a-2 and other blood type variants. The invention also relates to products, in particular, probes, primers and kits for use in such methods.

### Background to the Invention

The success of blood transfusion often depends on the degree of compatibility between donor and recipient. The degree of compatibility, in turn, is a function of the similarity in Red Blood Cell (RBC) antigen content between donor and recipient. Most RBC antigens in an individual can be predicted in a simple manner from the analysis of their genomic DNA. Therefore, analysis of donor and/or recipient DNA can be used to predict the degree of compatibility and thus enable proper blood transfusion practice.

Hemolytic reactions are more common in multi-transfused than in singly transfused individuals, not only because of the increased probability of such an event as the number of transfused units increases, but also because of the accumulative immunological memory-driven nature of the immune response in the recipient. An example of a condition whose treatment includes repeated blood transfusions is Sickle Cell Disease (SCD). It therefore follows that a high degree of compatibility with donor blood is often critical for the success of transfusion in SCD patients.

While SCD is more prevalent among individuals of African ancestry, the blood donor population in the USA and other Western countries is largely Caucasian. As a consequence of this disparity, differences in RBC antigens between both racial groups often become responsible for blood transfusion failures in SCD patients.

The genetic variant *RHD*DIIIa-CE(4-7)-D* (also known as *RHD-CE-D^{S}, RHD-CE(4-7)-D, (C)ce^{S}*, or *r'^{S}*) can be found in approximately 5% of the African-American population, but has not been reported in Caucasians. This variant poses a special challenge to blood transfusion because it encodes a rather complex antigen profile, which includes absence of D antigen, altered forms of C (C^{+W}) and e antigens, expression of low-frequency VS antigen, no expression of V antigen, and absence of the high-frequency hr^{B} antigen. The D and C antigenic profiles are the clinically most relevant for determining compatibility between blood donors and transfusion patients.

The antigenic complexity of *RHD***DIIIa-CE(4-7)-D* correlates with its genetic complexity, which includes changes to the *RHD* gene, with a substitution of part of *RHD* exon 3, *RHD* exons 4-7, and the intervening introns by their *RHCE* counterparts, a G>T substitution at position 186 (exon 2), a C>T substitution at position 410 (hybrid exon 3), a C>G substitution at position 733 (exon 5), and a G>T substitution at position 1006 (exon 7). In addition to the changes in the *RHD* gene, *RHD*DIIIa-CE(4-7)-D* occurs in *cis* with an *RHCE* gene that encodes substitutions C>G at position 733 (exon 5) and G>T at position 1006 (exon 7). The C^{+W} phenotype is characterized by weak expression the Rh C antigen.

To add to the antigenic and genetic complexity, our knowledge about the molecular basis of *RHD*DIIIa-CE(4-7)-D* is incomplete. For instance, the precise points of *RHCE*/*RHD* recombination in intron 3 or intron 7 have not been reported to date. Furthermore, two types of *RHD***DIIIa-CE(4-7)-D* variants have been described (Type 1 and Type 2), which differ in both their genetic composition and antigen profiles, although the clinically relevant D and C antigenic profiles are the same. There may be other *RHD***DIIIa-CE(4-7)-D* -like phenotypes, which also have a D- and C^{+W} antigenic profile. Clinically, it is therefore most important to distinguish *RHD*DIIIa-CE(4-7)-D* and other *RHD*DIIIa-CE(4-7)-D* -like phenotypes from phenotypes with different C and D antigenic profiles.

Several publications (Refs. 1-3) have examined the genetic similarity between *RHD*DIIIa-CE(4-7)-D* and other *RHD* variants, in particular *RHD***DIIIa* and *RHD*DIVa-1*/*RHD*DIVa-2* (*RHD***DIVa-2* henceforth). A number of molecular methods for the specific detection of *RHD***DIIIa-CE(4-7)-D* relied on the detection of single nucleotide polymorphisms (SNPs) located in hybrid exon 3. These SNPs are now known to be shared with variants *RHD***DIIIa* and *RHD*DIVa-2.* Consequently, identification of *RHD***DIIIa-CE(4-7)-D* in a sample by DNA analysis requires detection of hybrid exon 3 SNPs and discrimination from *RHD***DIIIa* and *RHD***DIVa-2,* which is difficult with current methods of genotyping. This discrimination is clinically relevant since *RHD***DIIIa* and *RHD*DIVa-2* encode a different antigen profile, which includes expression of partial D and absence of C^{+W} (i.e. partial D, C-). It is also important to distinguish between other genetic variants that may also share these hybrid exon 3 SNPs but encode different combinations of D and C antigens, which may also be clinically relevant.

WO 2006/075254 describes methods and products for *in vitro* genotyping.

Advent et al., 2009, Transfusion Medicine and Hemotherapy, Vol. 36, No. 3, pp. 162-167, describes the bloodgen project of the European Union.

Westhoff et al., 2010, Transfusion, Vol. 50, No. 6, pp. 1303-1311, reports that DIIIa and DIII Type 5 are encoded by the same allele and are associated with altered RHCE*ce alleles.

Pham et al., 2009, Transfusion, Vol. 49, No. 3, pp. 495-504, describes the heterogeneous molecular background of the weak C, VS+, hr(B)-, Hr(B)- phenotype in black persons.

WO 2006/032897 describes gene analysis, particularly of the *ABO* and *RHD* genes.

DE 10049363 describes a diagnostic kit, a microarray and their use for the determination of the Rh factor in a human, for example in prenatal diagnosis.

Faas et al., 1995, BLOOD, Vol. 85, No. 3, pp. 829-832, describes Rh E/e genotyping by allele-specific primer amplification.

Maaskant-van Wijk et al., 1998, Transfusion, Vol. 11, No. 38, pp. 1015-1021, describes genotyping of *RHD* by multiplex polymerase chain reaction analysis of six RHD-specific exons.

WO 2011/003921 describes methods and products for *in vitro* genotyping.

Some of these other *RHD* variants have been identified, for example RHD*DIVb-4, RHD*weakDtype4.0, RHD*weakDtype4.1, RHD*weakDtype14, RHD*weakDtype51, RHD*DAR, RHD*DAR-E, RHD*ex04-ex07del, RHD*ex03del and RHD*ex03-ex04del, which have varied expression of D antigen.

Antibody reagents commonly used to detect C antigen do not discriminate between C^{+W} and C⁺. Therefore, the phenotype is often reported as C⁺. In cases where the antibody reagent does discriminate between C^{+W} and C⁺ but the sample contains a normal *RHCE*C* allele in *trans* to a *RHD***DIIIa-CE(4-7)-D* allele, C^{+W} is obscured by C⁺, resulting in a C⁺ phenotype for the sample.

This makes it difficult to determine the correct phenotype for C^{+W}/C⁻, C^{+W}/C^{+W}, C^{+W}/C⁺ and C⁺/C⁺ antigenic profiles using serology analysis alone. Therefore, *RHCE*C* needs to be tested for and shown absent prior to assignment of a C^{+W} phenotype to a sample, and so current methods of diagnosing a *RHD***DIIIa-CE(4-7)-D* antigenic profile are difficult even when both serology analysis and genetic analysis are performed.

### Summary of the Invention

The inventors have found a method of discriminating the *RHD***DIIIa-CE(4-7)-D* or *RHD***DIIIa-CE(4-7)-D*)-like blood type variants, which express the C^{+W} antigen and lack a D antigen, from *RHD***DIIIa, RHD*DIVa-2* and other blood type variants, by determining at least four genetic markers. The method does not require the use of antibodies, and enables prediction of the D and C antigen phenotypes of a large majority of samples containing *RHD*/*RHCE* hybrid exon 3.

In a first aspect the present invention provides a method of discriminating the *RHD*DIIIa-CE(4-7)-D* or *RHD*DIIIa-CE(4-7)-D-like* blood type variants, which express the C^{+W} antigen and lack a D antigen, from *RHD*DIIIa, RHD*DIVa-2* and other blood type variants, of a subject, the method comprising:
determining at least 4 markers in a sample that has been obtained from the subject, wherein the markers comprise:
   (i) the presence or absence of an RHCE*C allele;
   (ii) the presence or absence of an RHD/RHCE hybrid exon 3 (RHD/CE Hex03) allele;
   (iii) the absence of, or a single nucleotide polymorphism (SNP) variant within, any one of RHD exon 4, *RHD* exon 5, or *RHD* exon 6; and
   (iv) the absence of, or SNP variant within, RHD exon 7,
wherein:
absence of said RHCE*C allele; presence of said RHD/RHCE hybrid exon 3 allele; absence of *RHD* exon 4, *RHD* exon 5 or *RHD* exon 6; and absence of *RHD* exon 7 in combination indicate that the sample contains said *RHD*DIIIa-CE(4-7)-D or said RHD*DIIIa-CE(4-7)-D-*like blood type variant.

In some cases in accordance with the present invention:
a) the SNP variant within *RHD* exon 4 is at position 602 of the *RHD* coding sequence (rs1053355),
b) the SNP variant within *RHD* exon 5 is at position 667 of the *RHD* coding sequence (rs1053356),
c) the SNP variant within *RHD* exon 6 is at position 819 of the *RHD* coding sequence; and/or
d) the SNP variant within *RHD* exon 7 is at position 1048 of the *RHD* coding sequence (rs41307826),
wherein the *RHD* coding sequence is as set forth in SEQ ID NO: 1.

In some cases in accordance with the present invention the markers further comprise:
(v) the presence or absence of an RHD exon 3 allele,
wherein the absence of said *RHD* exon 3 further indicates that the sample contains said *RHD*DIIIa-CE(4-7)-D or said RHD*DIIIa-CE(4-7-D-*like blood type variant.

In some cases in accordance with the present invention:
a) the method further comprises determining the RHD and RHC antigen phenotypes of the subject in accordance with Table 1; and/or
b) the method comprises detecting the presence or absence of a blood type variant selected from: *RHD*DIIIa; RHD***DIVa-2;* or *RHD*DIIIa-CE(4-7)-D* or *RHD*DIIIa-CE(4-7)-D*)*-*like blood type variants, e.g. wherein the method comprises detecting the presence or absence of *RHD*DIIIa-CE(4-7)-D* or *RHD***DIIIa-CE(4-*7)-D)-like blood type variants; and/or
c) marker (iii) is the SNP within RHD exon 4 at position 602 of the RHD coding sequence (rs1053355); and/or
d) the RHCE*C allele is determined by determining the presence or absence of RHCE*C intron 2, or any one of the following positions in the RHCE coding sequence: position 307 (exon 2), position 48 (exon 1), position 150 (exon 2), position 178 (exon 2), position 201 (exon 2) and/or position 203 (exon 2),
wherein the *RHD* coding sequence is as set forth in SEQ ID NO: 1 and wherein the *RCHE* coding sequence is as set forth in SEQ ID NO: 2.

In some cases in accordance with the present invention the sample comprises nucleic acid and the method comprises amplifying the nucleic acid or a portion thereof by PCR using primers, e.g. wherein:
a) the PCR primers for determining the RHCE*C allele are a forward PCR primer specific for RHCE*C, and a non-specific reverse PCR primer, e.g. wherein
   (i) the non-specific reverse primer is shared with RHD, RHC*C and/or RHC*c; and/or
   (ii) the PCR primers comprise:
      Forward: 5'-GGCCACCACCATTTGAA-3' (SEQ ID NO: 3)
      Reverse: 5'-CCATGAACATGCCACTTCAC-3', (SEQ ID NO: 4);
      and/or
b) the PCR primers for determining the RHD/CE Hex03 allele are forward and reverse PCR primers targeting sequences located in introns 2 and 3, or introns 3 and 2, respectively, e.g. wherein
   (i) the PCR primers comprise:
      Forward primer: 5'-TCCTGGCTCTCCCTCTCT-3' (SEQ ID NO: 9)
      Reverse primer: 5'-TTTTCAAAACCCCGGAAG-3 (SEQ ID NO: 10); and/or
c) the PCR primers for determining the SNP within RHD exon 4 at position 602 of the *RHD* coding sequence (rs1053355) are forward and reverse primers targeting sequences located in introns 3 and 4, or introns 4 and 3, respectively, e.g. wherein
   (i) the PCR primers comprise:
      Forward primer: 5'-GCTCTGAACTTTCTCCAAGGACT-3' (SEQ ID NO: 17)
      Reverse primer: 5'-ATTCTGCTCAGCCCAAGTAG-3' (SEQ ID NO: 18); and/or
d) the PCR primers for determining the SNP within RHD exon 5 at position 667 of the *RHD* coding sequence (rs1053356) are forward and reverse primers targeting sequences located in introns 4 and 5, or introns 5 and 4, respectively, e.g. wherein
   (i) the PCR primers comprise:
      Forward primer: 5'-TTGAATTAAGCACTTCACAGAGCA-3' (SEQ ID NO: 19)
      Reverse primer: 5'-CACCTTGCTGATCTTCCC-3' (SEQ ID NO: 20); and/or
e) the PCR primers for determining the SNP within RHD exon 6 at position 819 of the *RHD* coding sequence are forward and reverse primers targeting sequences located in introns 5 and 6, or introns 6 and 5, respectively, e.g. wherein
   (i) the PCR primers comprise:
      Forward primer: 5'-AGTAGTGAGCTGGCCCATCA-3' (SEQ ID NO: 21)
      Reverse primer: 5'-CTTCAGCCAAAGCAGAGGAG-3' (SEQ ID NO: 22); and/or
f) the PCR primers for determining the SNP within RHD exon 7 at position 1048 of the *RHD* coding sequence (rs41307826) are forward and reverse primers targeting sequences located in introns 6 and 7, or introns 7 and 6, respectively, e.g. wherein
   (i) the PCR primers comprise:
      Forward primer: 5'-ACAAACTCCCCGATGATGTGAGTG-3' (SEQ ID NO: 35)
      Reverse primer: 5'-GAGGCTGAGAAAGGTTAAGCCA-3' (SEQ ID NO: 36); and/or
g) as dependent from claim 3, the PCR primers for determining the RHD exon 3 allele are forward and reverse primers targeting sequences located in introns 2 and 3, or introns 3 and 2, respectively, e.g. wherein
   (i) the PCR primers comprise:
      Forward primer: 5'-TCCTGGCTCTCCCTCTCT-3' (SEQ ID NO: 15)
      Reverse primer: 5'-GTTGTCTTTATTTTTCAAAACCCT-3' (SEQ ID NO: 16);
wherein the *RHD* coding sequence is as set forth in SEQ ID NO: 1.

In some cases in accordance with the present invention the amplified nucleic acid comprises a label, e.g. wherein
a) the label comprises a biotinylated nucleotide; and/or
b) the label comprises a fluorescent moiety.

In some cases in accordance with the present invention the sample comprises nucleic acid, and the method comprises amplifying the nucleic acid or a portion thereof by PCR using primers, fragmenting the amplified nucleic acid, and labelling the fragmented nucleic acid with biotinylated ddNTPS using a terminal deoxynucleotidyl transferase (TdT) enzyme.

In some cases in accordance with the present invention determining the presence, absence or SNP variant of a marker comprises contacting nucleic acid containing each marker with one or more probes, e.g. wherein:
a) the probes for determining the presence or absence of RHD/CE Hex03 or RHD exon 3 contact an SNP located in both RHD/CE Hex03 and RHD exon 3, wherein one SNP variant is specific for RHD/CE Hex03, and another SNP variant is specific for RHD exon 3, e.g. wherein
   (i) the SNP is at position 410 of the coding sequence, located within both RHD/CE Hex03 and RHD exon 3; and/or
   (ii) the probes comprise:
      (1) 5'-TTTTACAGACGCCTGCTACCATG-3', (SEQ ID NO: 5)
      (2) 5'-CATGGTAGCAGGCGTCTGTAAAA-3', (SEQ ID NO: 6)
      (3) 5'-TTTTACAGACGTCTGCTACCATG-3', (SEQ ID NO: 7) and
      (4) 5'-CATGGTAGCAGACGTCTGTAAAA-3', (SEQ ID NO: 8); and/or
b) the probes for determining the absence or SNP variant of the SNP at: position 602 of the *RHD* coding sequence located within exon 4 (rs1053355), position 667 of the *RHD* coding sequence located within exon 5 (rs1053356), or position 819 of the *RHD* coding sequence located within exon 6 comprise:
   (i) RHD exon 4:
      (1) 5'-ATAAAGATCAGACAGCAACGATACC-3' (SEQ ID NO: 23)
      (2) 5'-TAAAGATCAGACAGCAACGATAC-3' (SEQ ID NO: 24)
      (3) 5'-ATAAAGATCAGAGAGCAACGATACC-3' (SEQ ID NO: 25)
      (4) 5'-TAAAGATCAGAGAGCAACGATAC-3' (SEQ ID NO: 26);
   (ii) RHD exon 5:
      (1) 5'-CTGGCCAAGTTTCAACTCTGC-3' (SEQ ID NO: 27)
      (2) 5'-TGGCCAAGTTTCAACTCTG-3' (SEQ ID NO: 28)
      (3) 5'-CTGGCCAAGTGTCAACTCTGC-3' (SEQ ID NO: 29)
      (4) 5'-TGGCCAAGTGTCAACTCTG-3' (SEQ ID NO: 30);
   (iii) RHD exon 6:
      (1) 5'-GTGCACAGTGCGGTGTTGGCAGG-3' (SEQ ID NO: 31)
      (2) 5'- TGCACAGTGCGGTGTTGGCAG -3' (SEQ ID NO: 32)
      (3) 5'- GTGCACAGTGCAGTGTTGGCAGG -3' (SEQ ID NO: 33)
      (4) 5'-TGCACAGTGCAGTGTTGGCAG-3' (SEQ ID NO: 34); and/or
c) the probes for determining the SNP variant of the SNP at position 1048 of the *RHD* coding sequence located within exon 7 (rs41307826) comprise:
   (1) 5'-TGCTGGTGCTTGATACCGTCGGA-3' (SEQ ID NO: 37)
   (2) 5'-GCTGGTGCTTGATACCGTCGG-3' (SEQ ID NO: 38)
   (3) 5'-TGCTGGTGCTTCATACCGTCGGA-3' (SEQ ID NO: 39)
   (4) 5'-GCTGGTGCTTCATACCGTCGG-3' (SEQ ID NO: 40);
   wherein the *RHD* coding sequence is as set forth in SEQ ID NO:1.

In some cases in accordance with the present invention
a) one or more of the probes comprise a label, e.g. wherein the label is a fluorescent moiety; and/or
b) one or more of the probes is attached to a solid support or conjugated to one or more particles.

In a further aspect the present invention provides use of a set of primers in a method for discriminating the *RHD*DIIIa-CE(4-7)-D* or *RHD*DIIIa-CE(4-7-D)-*like blood type variants, which express the C^{+W} antigen and lack a D antigen, from *RHD*DIIIa, RHD*DIVa-2* and other blood type variants, by amplifying nucleic acid comprising at least the four markers defined in claim 1, wherein the set of primers comprises at least three primer pairs selected from the primers set forth in:
(i) claim 5(a),
(ii) claim 5(b),
(iii) any one of claims 5(c), 5(d) or 5(e)
(iv) claim 5(f), and
(v) claim 5(g).

In some cases in accordance with the present invention the set of primers comprises at least three primer pairs selected from the primers set forth in:
(i) claim 5(a)(ii),
(ii) claim 5(b)(i),
(iii) any one of claims 5(c)(i), 5(d)(i) or 5(e)(i),
(iv) claim 5(f)(i), and
(v) claim 5(g)(i).

In some cases in accordance with.the present invention at least 50% of the primers in the set are the primer pairs defined in claim 10 or 11.

In yet a further aspect the present invention provides use of a set of probes in a method for discriminating the *RHD***DIIIa-CE(4-7)-D* or *RHD***DIIIa-CE(4-7-D)-*like blood type variants, which express the C^{+W} antigen and lack a D antigen, from *RHD*DIIIa, RHD*DIVa-2* and other blood type variants, by determining the presence, absence or single nucleotide polymorphism (SNP) variant of at least the four markers as defined in claim 1, wherein the set of probes comprises:
(i) the probes of SEQ ID NOs: 5, 6, 7 and 8;
(ii) the probes of SEQ ID NOs: 23, 24, 25 and 26;
(iii) the probes of SEQ ID NOs: 31, 32, 33 and 34
(iv) the probes of SEQ ID NOs: 27, 28, 29 and 30; and
(v) the probes of SEQ ID NOs: 37, 38, 39 and 40.

In some cases in accordance with the present invention:
a) the probes are immobilised on a solid support or conjugated to one or more particles, e.g. wherein the solid support comprises one or more attached labels, e.g. wherein the label is a fluorochrome; and/or
b) one or more probes comprise a label, e.g wherein the label is a fluorescent moiety.

In yet a further aspect the present invention provides use of a kit for genotyping a subject, the kit comprising a set of PCR primers as defined in any one of claims 10 to 12, and a set of probes as defined in any one of claims 13 to 14.

### Description of the Figures

**Figure 1**: Coding sequence of *RHD* (SEQ ID NO: 1), showing the positions of each exon. The nucleotide sequence shown is a consensus sequence.
**Figure 2**: Coding sequence of *RHCE* (SEQ ID NO: 2), showing the positions of each exon. The nucleotide sequence shown is a consensus sequence.

### Detailed Description of the Invention

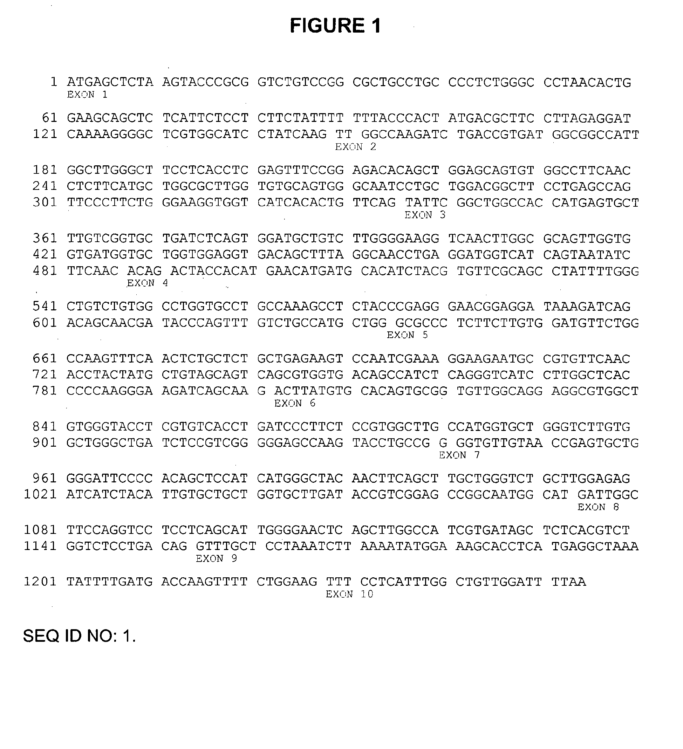

The Rh blood group D antigen is encoded by the *RHD* gene, which comprises 10 exons. The complete *RHD* gene sequence is available at NCBI Reference Sequence: NG_007494.1 No. NG_007494.1, GI:171184448, (SEQ ID NO: 41). The coding sequence, annotated to show the starting position of each exon, is shown in Figure 1(SEQ ID NO: 1).

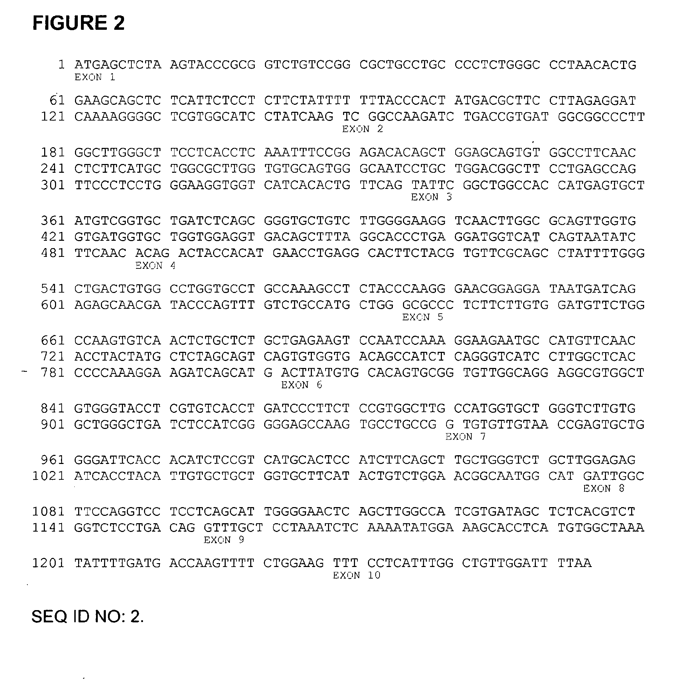

The Rh blood group C antigen is encoded by the *RHCE* gene, which comprises 10 exons. The complete *RHCE* gene sequence is available at NCBI Reference Sequence: NG_009208.2, GI:301336136, (SEQ ID NO: 42). The coding sequence, annotated to show the starting position of each exon, is shown in Figure 2 (SEQ ID NO: 2).

The present invention enables determination of the clinically relevant RHD and RHC antigen phenotypes of a blood sample, based on at least the following four markers: at least one RHCE*C allele; at least one RHD/RHCE hybrid exon 3 (RHD/CE Hex03) allele; the SNP at position 602 of the *RHD* coding sequence, within exon 4, or the SNP at position 667 of the *RHD* coding sequence, within exon 5, or the SNP at position 819 of the *RHD* coding sequence, within exon 6; and the SNP at position 1048 of the *RHD* coding sequence, within exon 7.

Determination of the clinically relevant RHD and RHC antigen phenotypes of a blood sample can be further based on determining a fifth marker: namely, at least one RHD exon 3 allele.

Table 1 demonstrates how the combination of these five markers enables the prediction of the vast majority of RHD and RHC antigen phenotypes. The first three columns show whether at least one allele of RHCE*C, RHD/RHCE hybrid exon 3 (RHD/CE Hex03) allele, and RHD exon 3 are present. The columns entitled RHD 602 and RHD 1048 show the SNP variants at these positions, i.e whether they are absent, heterozygous, or homozygous/one SNP is absent. The presence of at least one allele of RHCE*C determines whether a C⁺ antigen is present, and the next four columns provide information on the D antigen phenotype, and C antigen phenotype in the absence of RHCE*C. Together, these data enable the RHD genetic haplotypes and D and C antigenic phenotypes to be predicted, and different blood types can therefore be distinguished on this basis.

Although the combination of the 4 or 5 markers described herein are able to distinguish *RHD***DIIIa-CE(4-7)-D* or *RHD*DIIIa-CE(4-7)-D*)*-*like blood type variants from *RHD***DIIIa, RHD***DIVa-2* and other blood type variants, they are not intended to unambiguously predict all possible C and D antigenic combinations. Therefore, "Possibly RHD" in the haplotype column is a generic term meant to include RHD as well as RHD variants other than the ones interrogated by the present method. Likewise, "D?" in the phenotype column refers to D, Partial D, Weak D, D- or Dₑₗ phenotypes encoded by RHD variants other than those interrogated by the present method. D+ is the most likely phenotype in this situation, however.

Interpretation of the Predicted Phenotype data in Table 1 is facilitated by Table 2, which provides an exhaustive description of how the haplotype-encoded phenotypes relevant to the present invention (the D antigen phenotype and C antigen phenotype) combine to yield the phenotype of a sample.

In some embodiments, presence/absence of *RHD* exon 3 can be determined by determining the SNP nucleotide sequence at position 410 of the *RHD* coding sequence, within exon 3 (rs number not available).

In some embodiments, determination of the nucleotide sequence at position 602 of the *RHD* coding sequence, within exon 4, could be substituted by the determination of the SNP nucleotide sequence (nucleotide T vs. nucleotide G) at position 667 of the *RHD* coding sequence, within exon 5, or the SNP nucleotide sequence (nucleotide G vs. nucleotide A) at position 819 of the *RHD* coding sequence, within exon 6.

Other combinations of markers that include fewer than the at least 4 or 5 markers described herein would result in a decreased power to determine D- C^{+W} phenotypes.

For instance, without the determination of the presence/absence of *RHCE*C,* it would not be possible to predict a C- phenotype for RHD*DIIIa and/or RHD*DIVa-2 samples or a C^{+W} phenotype for RHD*DIIIa-CE(4-7)-D samples.

Without the determination of the presence/absence of *RHD*/*RHCE* hybrid exon 3, it would not be possible to deduce a RHD*DIIIa-CE(4-7)-D haplotype for any sample, and therefore it would not be possible to predict a C^{+W} phenotype for any sample.

Without the determination of the SNP variant at position 602 of the *RHD* coding sequence, it would not be possible to deduce a RHD*DIIIa haplotype for a sample, and therefore it would not be possible to predict a non-D⁻ phenotype for said sample.

Without the determination of the nucleotide at position 1048 of the *RHD* coding sequence, it would not be possible to deduce a RHD*DIVa-2 haplotype for a sample, and therefore it would not be possible to predict a non-D⁻ phenotype for said sample.

Without the determination of the presence/absence of *RHD* exon 3, it would not be possible to predict a RHD*DIIIa-CE(4-7)-D haplotype for samples with certain novel *RHD* variants, and therefore it would not be possible to predict a C^{+W} phenotype for those samples. For example, when at least one RHCE*C allele is absent, at least one RHD/RHCE hybrid exon 3 (RHD/CE Hex03) allele is present, the SNP at position 602 of the RHD coding sequence is absent, and the SNP at position 1048 of the RHD coding sequence is absent, the presence or absence of at least one RHD exon 3 allele will allow prediction of a C- or a C^{+W} phenotype, respectively (Table 1). Similarly, when at least one RHCE*C allele is absent, at least one RHD/RHCE hybrid exon 3 (RHD/CE Hex03) allele is present, nucleotide sequence at position 602 of the RHD coding sequence is G, and nucleotide sequence at position 1048 of the RHD coding sequence is G, the absence or presence of at least one RHD exon 3 allele will allow prediction of a Partial D or an undetermined (Weak D or Partial D) phenotype, respectively.

A variety of suitable techniques could be used to detect these genetic sequences. The following are presented as non-limiting examples of such techniques.

A suitable technique to detect the herein mentioned genetic sequences is mutation analysis by restriction digestion after a PCR reaction for amplifying the region of interest, if the genetic variant or polymorphism results in the creation or elimination of a restriction site. Sequence analysis, such as direct manual or fluorescent automated sequencing, directly or after selection of the region of interest by PCR, can also be used to detect specific sequences. Allele-specific oligonucleotides, for example, used in a competitive PCR, can also be used to detect genetic variants.

Another technique to detect specific sequences in a sample is testing that sample for the presence of a nucleic acid molecule comprising all or a portion of the region of interest, comprising contacting said sample with a second nucleic acid molecule or probe under conditions for selective hybridization. All or a part of the region of interest can be amplified prior to performing the specific technique used for detection of the genetic variants.

The sample may be any biological sample from a patient, for example tissue, blood, serum or saliva from a patient. The sample may contain cells, be cell-free or consist only of isolated cells.

The methods of the invention make use of the detection of the presence or absence of one or more specific nucleotide sequences within the functional segments.

In certain cases, the method of the invention may be termed Allele-Specific Hybridization, and may make use of synthetic oligonucleotide probes usually 10-50 nucleotides long, preferably 19-27 nucleotides long, the sequences of which are designed to be complementary to the interrogated sequence. Complementarity of sequences enables pairing of genomic DNA and oligonucleotide probe molecules. Specific pairing, i.e. pairing of probes to their complementary sequence and to no other sequence, can be made to occur under appropriate conditions, which include but are not limited to the time of incubation, temperature of incubation, concentration of probe and complementary sequences, stringency of buffers, and mixing. Specific pairing to probes allows detection of sequences in a mix of sequences. Detection or lack of detection of specific sequences, in turn, allows determination of presence versus absence of functional segments.

Synthetic oligonucleotide probes can be used for the detection of particular conserved, non-variant regions and/or allelic variants in an individual's genomic DNA. Often, allelic variants are single nucleotide polymorphisms (SNPs), i.e. nucleotide positions at which the DNA composition may vary across individuals.

In some cases, the synthetic oligonucleotide probes described herein are designed and used to detect the presence or absence of functional nucleic acid segments and also, both to detect allelic variants located within sequences and to determine the presence or absence of functional segments.

Given a particular nucleotide at a particular position of a locus of genomic DNA, synthetic oligonucleotide molecules, or probes, can be designed to detect said nucleotide in a test sample. Probes can be designed in pairs such that one member of the probe pair is complementary to one strand of the sequence, whereas the other member of the probe pair is complementary to the other strand of the sequence. Probes can also be designed in sets so that they have different lengths and be complementary to one strand or the two strands of the sequence of interest.

In accordance with any aspect of the present invention, probes may be attached to a chemically-functionalized solid support. An example of a solid support is a flat glass surface, on which probe molecules are placed by contact deposition. Another example of a solid support is a particle such as a micrometer-size polymer bead, to which probe molecules are attached by conjugation. Another example of a solid support is a nanometer-size particle to which probe molecules are attached.

If the probes are immobilised on a flat glass surface, attachment of a probe to the surface may be performed at multiple individual locations, hereafter referred to as replicate features or "replicates". The number of replicate features for each probe is usually ten, although it may vary. If the probes are immobilised on particles, attachment may be to multiple ensembles of particles.

In accordance with any aspect of the invention described herein, functional segments or their portions containing markers of the invention may be amplified, for example by PCR, using genomic DNA as a template. Amplified functional segments or their portions containing markers can be labeled (e.g. with a biotin and/or fluorescent label) to allow for their detection, and optionally fragmented to facilitate pairing with oligonucleotide probes.

In accordance with any aspect of the invention described herein, labelled and fragmented functional segments or their portions containing markers of the invention may be incubated under conditions that maximize the sensitivity and specificity of pairing with probes attached to the solid support. The presence of probe-paired functional segments or their portions may be determined indirectly from the measurement of a label, usually a fluorochrome, attached to the solid support. This measurement is referred to herein as signal intensity. By way of example, the fluorescence emitted by the fluorochrome may be collected by means of a fluorescence detection device, such as a confocal scanner.

### Determination of the presence or absence of RHCE*C

### Amplification of RHCE*C intron 2 by PCR.

The marker *RHCE**C may be detected by amplifying *RHCE*C* intron 2 using oligonucleotide primers that bind to intron 2 sequences. The target sequence of the forward (upstream) primer may be *RHCE*C-*specific*,* and the target sequence of the reverse (downstream) primer may be non-specific, i.e. shared by *RHD, RHC***C, RHC*c.* The following primers may be used, which yield a PCR product with a size of 357 base pairs :
- Forward primer: 5'**-GGCCACCACCATTTGAA**-3' (SEQ ID NO: 3)
- Reverse primer: 5'-CCATGAACATGCCACTTCAC-3' (SEQ ID NO: 4)

In boldface, *RHCE***C-*specific nucleotides (forward primer).

Alternatively, RHCE*C can be amplified using any oligonucleotide primers that differ from the previously described primers in sequence, length, or any other feature, as long as they enable specific amplification of the *RHCE**C-specific insert located in intron 2. For example, a variant oligonucleotide primer may be used that differs from a primer described herein by 1, 2, 3, 4 or 5 nucleotide sequence alterations.

Alternatively, this step can make use of any oligonucleotide primers that enable the amplification of any other known *RHCE**C-specific sequences, other than the previously described intron 2 insert, for the purpose of establishing the presence or absence of an *RHCE*C* allele in a sample. Such sequences usually contain polymorphic positions in the coding or non-coding regions of the *RHCE* gene. Instances include but are not limited to the following: position 48 (exon 1), position 150 (exon 2), position 178 (exon 2), position 201 (exon 2), position 203 (exon 2), and position 307 (exon 2) of the *RHCE* coding sequence.

The presence or absence of RHCE*C may then be visualized directly, for example by gel electrophoresis, or indirectly, for example by hybridization with a probe as discussed below. Alternatively, the presence or absence of RHCE*C may be determined by probe hybridization alone without prior PCR amplification.

### Hybridization of RHCE*C to oligonucleotide probes.

In some embodiments, the hybridization of the RHCE*C intron 2 amplicon to oligonucleotide probes can make use of 4 oligonucleotide probes: 2 probes would be complementary to a portion of the *RHCE**C-specific insert, each to one of the DNA strands. The other 2 probes would be identical to the above except that they contain an artificial single-nucleotide mismatch at their central position that largely prevents hybridization, thus providing a background signal as a negative control. These probes can only detect presence versus absence of the variant sequence, i.e. they do not allow discrimination between homozygous/hemizygous and heterozygous samples. The following probe sequences can be used for this insert:
*RHCE**C-specific perfect match probe #1: 5'-TTTTACAGACGCCTGCTACCATG-3' (SEQ ID NO: 5)
- *RHCE**C-specific perfect match probe #2: 5'-CATGGTAGCAGGCGTCTGTAAAA-3' (SEQ ID NO: 6)
- Mismatch probe #1: 5'-TTTTACAGACG**T**CTGCTACCATG-3' (SEQ ID NO: 7)
- Mismatch probe #2: 5'-CATGGTAGCAG**A**CGTCTGTAAAA-3' (SEQ ID NO: 8) In boldface, the central position mismatch.

Alternatively, any particular method or set of probes targeting an amplified RHCE*C-specific amplicon, or an unamplified RHCE*C-specific sequence directly, may be used to determine whether RHCE*C is present or absent. Alternatively, a variant oligonucleotide probe may be used that differs from a probe described herein by 1, 2, 3, 4 or 5 nucleotide sequence alterations.

### Determination of the presence or absence of RHD/RHCE hybrid exon 3

### Amplification of RHD/RHCE hybrid exon 3 by PCR.

The marker RHD/RHCE hybrid exon 3 may be detected by PCR amplification using oligonucleotide primers that bind to intronic sequences flanking *RHD*/*RHCE* hybrid exon 3. Specifically, the target sequences of forward (upstream) and reverse (downstream) primers can be located in introns 2 and 3, respectively. The following primers may be used, which yield a PCR product of 256 base pairs:
- Forward primer: 5'-TCCTGGCTCTCCCTCTCT-3'(SEQ ID NO: 9)
- Reverse primer: 5'-TTTTCAAAACCCCGGAAG-3' (SEQ ID NO: 10)
In boldface, *RHD*-specific nucleotides (forward primer) and *RHCE*-specific nucleotides (reverse primer).

The forward primer may also be used for amplification of RHD exon 3, discussed below.

Alternatively, *RHD*/*RHCE* hybrid exon 3 may be amplified using any oligonucleotide primers that differ from the previously described primers in sequence, length, or any other feature, as long as they enable specific amplification of *RHD*/*RHCE* hybrid exon 3. For example, a variant oligonucleotide primer may be used that differs from a primer described herein by 1, 2, 3, 4 or 5 nucleotide sequence alterations.

Alternatively, PCR amplification can make use of any oligonucleotide primers that enable the amplification of any known *RHD*/*RHCE* hybrid exon 3-associated sequences for the purpose of establishing the presence or absence of an *RHD*/*RHCE* hybrid exon 3 in a sample. Such sequences usually contain polymorphic positions in the coding or non-coding regions of the *RHD* gene. Instances include but are not limited to position 178 (exon 2) of the *RHD* coding sequence.

The presence or absence of the *RHD*/*RHCE* hybrid exon 3 may then be visualized directly, for example by gel electrophoresis, or indirectly, for example by hybridization with a probe as discussed below. Alternatively, the presence or absence of the RHD/RHCE hybrid exon 3 may be determined by probe hybridization alone without prior PCR amplification.

### Hybridization of RHD/RHCE hybrid exon 3 or RHD exon 3 amplicon to oligonucleotide probes.

In some embodiments, the hybridization of the *RHD*/*RHCE* hybrid exon 3 amplicon to oligonucleotide probes can make use of 4 oligonucleotide probes. These probes may also be used to detect an SNP at *RHD* exon 3, as discussed below, due to the high similarity between these sequences, as specificity of the hybridization signal from each marker comes mainly from the allele specific PCR amplification step. For example, different fluorescently modified nucleotides may be incorporated during PCR amplification into the *RHD*/*RHCE* hybrid exon 3 and *RHD* exon 3 amplicons, respectively. Alternatively, the two amplicons may be differently labelled after the PCR amplification step, for example by incubation with labelled nucleotides or nucleotide oligos in the presence of a polymerase, ligase or transferase enzyme. For example, the nucleic acid may be incubated with biotinylated ddNTPs in the presence of a terminal deoxynucleotidyl transferase enzyme.

2 probes may be specific for the wild-type sequence of an SNP located within the amplicon, and the other 2 probes may be specific for the hybrid exon 3 variant sequence of the same SNP. These probes can only detect presence versus absence of the variant sequence, i.e. they do not allow discrimination between homozygous and hemizygous samples. For example, the SNP located at position 410 of the coding sequence, with C and T as wild-type and variant nucleotides, respectively, can be used. The following probe sequences can be used for this SNP:
- *RHD, RHCE* wild-type probe #1: 5'-GGTCAACTTGGCGCAGTTGGTGG-3' (SEQ ID NO: 11)
- *RHD, RHCE* wild-type probe #2: 5'-GTCAACTTGGCGCAGTTGGTG-3' (SEQ ID NO: 12)
- Hybrid exon 3 variant probe #1: 5'-GGTCAACTTGGTGCAGTTGGTGG-3' (SEQ ID NO: 13)
- Hybrid exon 3 variant probe #2: 5'-GTCAACTTGGTGCAGTTGGTG-3' (SEQ ID NO: 14) In boldface is the SNP at position 410. The rs number for the SNP at position 410 is not available.

Alternatively, any particular method or set of probes targeting an amplified *RHD*/*RHCE* hybrid exon 3 -specific amplicon, or an unamplified *RHD*/*RHCE* hybrid exon 3 -specific sequence directly, may be used to determine whether *RHD*/*RHCE* hybrid exon 3 is present or absent. Alternatively, a variant oligonucleotide probe may be used that differs from a probe described herein by 1, 2, 3, 4 or 5 nucleotide sequence alterations.

### Determination of the presence or absence of RHD exon 3

### Amplification of RHD exon 3 by PCR.

The marker *RHD* exon 3 may be detected by PCR amplification using oligonucleotide primers that bind to intronic sequences flanking *RHD* exon 3. Specifically, the target sequences of forward and reverse primers may be located in introns 2 and 3, respectively. The following primers may be used, which yield a PCR product of 268 base pairs:
- Forward primer: 5'-TCCTGGCTCTCCCTCTC**T**-3' (SEQ ID NO: 15)
- Reverse primer: 5'-GTTGTCTTTATTTTTCAAAACCC**T**-3' (SEQ ID NO: 16)
In boldface are the *RHD*-specific nucleotides. The forward primer is specific for both *RHD* exon 3 and *RHD*/*RHCE* hybrid exon 3, while the reverse primer is specific for *RHD* exon 3 only.

Alternatively, *RHD* exon 3 may be amplified using any oligonucleotide primers that differ from the previously described primers in sequence, length, or any other feature, as long as they enable specific amplification of *RHD* exon 3.

Alternatively, PCR amplification can make use of any oligonucleotide primers that enable the amplification of publicly-reported *RHD* exon 3-associated sequences for the purpose of establishing the presence or absence of an *RHD* exon 3 in a sample. Such sequences usually contain polymorphic positions in the coding or non-coding regions of the *RHD* gene. Instances include but are not limited to intron sequences flanking *RHD* exon 3. For example, a variant oligonucleotide primer may be used that differs from a primer described herein by 1, 2, 3, 4 or 5 nucleotide sequence alterations.

The presence or absence of the *RHD* exon 3 may then be visualized directly, for example by gel electrophoresis, or indirectly, for example by hybridization with a probe as discussed with reference to *RHD*/*RHCE* hybrid exon 3 above. Alternatively, the presence or absence of the RHD exon 3 may be determined by probe hybridization alone without prior PCR amplification.

### Determination of the SNPs at RHD exon 4 or RHD exon 5 or RHD exon 6

### Amplification of RHD exon 4 or RHD exon 5 or RHD exon 6 by PCR.

In some embodiments, the SNP at *RHD* exon 4 may be detected by PCR amplification using oligonucleotide primers that bind to intronic sequences flanking *RHD* exon 4. Specifically, the target sequences of forward and reverse primers may be located in introns 3 and 4, respectively. The following primers may be used, which yield a PCR product of 281 base pairs:
- Forward primer: 5'-GCTCTGAACTTTCTCCAAGGAC**T**-3' (SEQ ID NO: 17)
- Reverse primer: 5'-ATTCTGCTCAGCCCAAGTA**G**-3' (SEQ ID NO: 18)

In boldface are *RHD*-specific nucleotides.

In another embodiment, the SNP at *RHD* exon 5 may be detected by PCR amplification using oligonucleotide primers that bind to intronic sequences flanking *RHD* exon 5. Specifically, the target sequences of forward and reverse primers can be located in introns 4 and 5, respectively. The following primers may be used, which yield a PCR product of 432 base pairs:
- Forward primer: 5'-TTGAATTAAGCACTTCACAGAGCA-3' (SEQ ID NO: 19)
- Reverse primer: 5'-CACC**TT**GCTGATCTTCC**C**-3' (SEQ ID NO: 20)
The underline indicates the location of the *RHCE*-specific 653-base pair insert exploited to confer *RHD* specificity to the amplification. In boldface, *RHD*-specific nucleotides.

In yet another embodiment, the SNP at *RHD* exon 6 may be detected by PCR amplification using oligonucleotide primers that bind to intronic sequences flanking *RHD* exon 6. Specifically, the target sequences of forward and reverse primers can be located in introns 5 and 6, respectively. The following primers may be used, which yield a PCR product of 371 base pairs:
- Forward primer: 5'-AGTAGTGAGCTGGCCCA**T**C**A**-3' (SEQ ID NO: 21)
- Reverse primer: 5'-CTTCAGCCAAAGCAGAG**GAG**-3'(SEQ ID NO: 22)
In boldface, *RHD*-specific nucleotides.

The presence or absence of these SNPs, and the specific SNP variant, may then be visualized directly, for example by gel electrophoresis after restriction digest as described above, or indirectly, for example by hybridization with a probe as discussed below. Alternatively, the SNP variant may be determined by probe hybridization alone without prior PCR amplification.

A variant oligonucleotide primer may be used that differs from a primer described herein by 1, 2, 3, 4 or 5 nucleotide sequence alterations.

### Hybridization of RHD exon 4 amplicon or RHD exon 5 amplicon or RHD exon 6 amplicon to oligonucleotide probes

In some embodiments, the hybridization of the *RHD* exon 4 amplicon to oligonucleotide probes can make use of 4 oligonucleotide probes: 2 probes are specific for the wild-type sequence of an SNP linked to *RHD* variants and located within the *RHD* exon 4 amplicon. The other 2 probes enable the *RHD***DIIIa* and *RHD***DIIIa-CE(4-7)-D* variants to be distinguished from the *RHD*DIVa-2* variant, as they are specific for the *RHD***DIIIa* and *RHD***DIIIa-CE(4-7)-D* variants relative to *RHD*DIVa-*2*.* However, in some embodiments the probes may not distinguish between other *RHD* variants. In other words, that the individual SNPs by themselves may not uniquely identify a particular RHD variant (for example, due to the existence of other variants RHD variants, in addition to DIIIa, DIVa and RHD*DIIIa-CE(4-7)-D), but this SNP, and its probes, can distinguish one of the three variants Dllla, DIVa and RHD*DIIIa-CE(4-7)-D from the other two.

These probes allow discrimination between homozygous/hemizygous and heterozygous samples. For example, the SNP located at position 602 of the coding sequence, with C and G as wild-type and *RHD* variant nucleotides, respectively, can be used. The following probe sequences can be used for this SNP:
- *RHD* wild-type probe #1: 5'-ATAAAGATCAGA**C**AGCAACGATACC-3' (SEQ ID NO: 23)
- *RHD* wild-type probe #2: 5'-TAAAGATCAGA**C**AGCAACGATAC-3' (SEQ ID NO: 24)
- *RHD* variant probe #1: 5'-ATAAAGATCAGA**G**AGCAACGATACC-3' (SEQ ID NO: 25)
- *RHD* variant probe #2: 5'-TAAAGATCAGAGA**G**CAACGATAC-3' (SEQ ID NO: 26)
In boldface is the SNP.

In another embodiment, the hybridization of the RHD exon 5 amplicon to oligonucleotide probes can make use of 4 oligonucleotide probes: 2 probes are specific for the wild-type sequence (i.e. sequence identical to the conventional *RHD*D* allele) of an SNP linked to *RHD* variants and located within the *RHD* exon 5 amplicon. The other 2 probes enable the *RHD*DIIIa* and *RHD*DIIIa-CE(4-7)-D* variants to be distinguished from the *RHD*DIVa-2* variant, as they are specific for the *RHD*DIIIa* and *RHD*DIIIa-CE(4-7)-D* variants relative to *RHD*DIVa-2.* However, in some embodiments the probes may not distinguish between other *RHD* variants. These probes allow discrimination between homozygous/hemizygous and heterozygous samples. For example, the SNP located at position 667 of the coding sequence, with T and G as wild-type and *RHD* variant nucleotides, respectively, can be used. The following probe sequences can be used for this SNP:
- *RHD* wild-type probe #1: 5'-CTGGCCAAGTTTCAACTCTGC-3' (SEQ ID NO: 27)
- *RHD* wild-type probe #2: 5'-TGGCCAAGTTTCAACTCTG-3' (SEQ ID NO: 28)
- *RHD* variant probe #1: 5'-CTGGCCAAGTGTCAACTCTGC-3' (SEQ ID NO: 29)
- *RHD* variant probe #2: 5'-TGGCCAAGTGTCAACTCTG-3' (SEQ ID NO: 30)
In boldface is the SNP.

In yet another embodiment, the hybridization of the *RHD* exon 6 amplicon to oligonucleotide probes can make use of 4 oligonucleotide probes: 2 probes would be specific for the wild-type sequence (i.e. sequence identical to the conventional *RHD*D* allele) of a SNP located within the *RHD* exon 6 amplicon, and linked to the *RHD*DIIIa* variant. The other 2 probes enable the *RHD*DIIIa* variant to be distinguished from *RHD*DIIIa-CE(4-7)-D* and *RHD*DIVa-2,* as they are completely specific for the *RHD*DIIIa* variant relative to *RHD*DIIIa-CE(4-7)-D* and *RHD*DIVa-2.* However, the probes may only be partially specific for the *RHD*DIIIa* variant sequence of the same SNP versus other *RHD* variants (i.e. *RHD* variants other than Dllla, DIVa and RHD*DIIIa-CE(4-7)-D). These probes allow discrimination between homozygous/hemizygous and heterozygous samples. For example, the SNP located at position 819 of the coding sequence, with G and A as wild-type and *RHD*DIIIa* variant nucleotides, respectively, can be used. The following probe sequences can be used for this SNP:
- *RHD* wild-type probe #1: 5'-GTGCACAGTGC**G**GTGTTGGCAGG-3' (SEQ ID NO: 31)
- *RHD* wild-type probe #2: 5'-TGCACAGTGCG**G**TGTTGGCAG -3' (SEQ ID NO: 32)
- *RHD*DIIIa* variant probe #1: 5'-GTGCACAGTGC**A**GTGTTGGCAGG -3' (SEQ ID NO: 33)
- *RHD*DIIIa* variant probe #2: 5'-TGCACAGTGC**A**GTGTTGGCAG-3' (SEQ ID NO: 34)
In boldface is the SNP. The rs number of the SNP at position 819 is not available.

Alternatively, a variant oligonucleotide probe may be used that differs from a probe described herein by 1, 2, 3, 4 or 5 nucleotide sequence alterations.

### Determination of the SNP at RHD exon 7

### Amplification of RHD exon 7 by PCR

The SNP variant at *RHD* exon 7 may be amplified using oligonucleotide primers that bind to intronic sequences flanking RHD exon 7. Specifically, the target sequences of forward and reverse primers may be located in introns 6 and 7, respectively. The following primers may be used, which yield a PCR product of 695 base pairs:
- Forward primer: 5'-ACAAACTCCCCGATGATGTGAGTG-3' (SEQ ID NO: 35)
- Reverse primer: 5'-GAGGCTGAGAAAGGTTAAGCCA-3' (SEQ ID NO: 36)
In boldface are RHD-specific nucleotides.

The presence or absence of this SNP, and the specific SNP variant, may then be visualized directly, for example by gel electrophoresis after restriction digest as described above, or indirectly, for example by hybridization with a probe as discussed below. Alternatively, the SNP variant may be determined by probe hybridization alone without prior PCR amplification.

A variant oligonucleotide primer may be used that differs from a primer described herein by 1, 2, 3, 4 or 5 nucleotide sequence alterations.

### Hybridization of RHD exon 7 amplicon to oligonucleotide probes.

The hybridization of the RHD exon 7 amplicon to oligonucleotide probes can make use of 4 oligonucleotide probes: 2 probes would be specific for the wild-type sequence (i.e. sequence identical to the conventional RHD*D allele) of a SNP located within the amplicon, and linked to RHD variants. The other 2 probes enable the *RHD*DIVa-2IIIa* variant to be distinguished from *RHD*DIIIa-CE(4-7)-D* and *RHD*DIIIa,* as the presence of the SNP is characteristic for the *RHD*DIVa-2* variant. However, in some embodiments the probes may not distinguish between other RHD variants (i.e. *RHD* variants other than DIIIa, DIVa and RHD*DIIIa-CE(4-7)-D). These probes allow discrimination between homozygous/hemizygous and heterozygous samples. For example, the SNP located at position 1048 of the coding sequence, with G and C as wild-type and *RHD*DIVa-2* variant nucleotides, respectively, can be used. The following probe sequences can be used for this SNP:
- *RHD* wild-type probe #1: 5'-TGCTGGTGCTT**G**ATACCGTCGGA-3' (SEQ ID NO: 37)
- *RHD* wild-type probe #2: 5'-GCTGGTGCTT**G**ATACCGTCGG-3' (SEQ ID NO: 38)
- *RHD*DIVa-2* variant probe #1: 5'-TGCTGGTGCTT**C**ATACCGTCGGA-3' (SEQ ID NO: 39)
- *RHD*DIVa-2* variant probe #2: 5'-GCTGGTGCTT**C**ATACCGTCGG-3' (SEQ ID NO: 40) In boldface is the SNP.

Alternatively, a variant oligonucleotide probe may be used that differs from a probe described herein by 1, 2, 3, 4 or 5 nucleotide sequence alterations.

Determination of binding of amplified sequences to wild-type versus variant probes is typically, but not solely, performed through quantitation of probe-bound fluorescence. Fluorescence and/or fluorescence-capturing moieties are typically, but not solely, introduced into the process at the amplification step in the form of modified nucleotides (see Materials & Methods section).

**Table 1**

| ***RHCE**C** | ***RHD*/*CE* Hex03** | ***RHD ex* 03** | ***RHD 602*** | ***RHD* 1048** | ***RHD* Haplotype 1/*RHD* Haplotype 2** | **RHD, RHC Phenotype** |
|---|---|---|---|---|---|---|
| Absent | Present | Present | C | G | RHD*DIIIa-CE(4-7)-D / Possibly RHD | D?, C^{+w} |
| Absent | Present | Present | c | G/C | RHD*DIVa-2 / Possibly RHD | D?, C- |
| Absent | Present | Present | C | C | RHD*DIVa-2 / RHD*DIVb-4 | Partial D, C⁻ |
| Absent | Present | Present | C | Absent | Not Reported | |
| Absent | Present | Present | C/G | G | RHD*DIIIa / Possibly RHD | D?, C⁻ |
| Absent | Present | Present | C/G | G/C | RHD*DIIIa / RHD*DIVb-4 | Partial D, C⁻ |
| | | | | | RHD*weakDtype4.0 / RHD*DIVa-2 | Weak or Partial D, C⁻ |
| | | | | | RHD*weakDtype4.1 / RHD*DIVa-2 | Weak or Partial D, C⁻ |
| | | | | | RHD*weakDtype14/ RHD*DIVa-2 | Weak or Partial D, C⁻ |
| | | | | | RHD*weakDtype51 / RHD*DIVa-2 | Weak or Partial D, C⁻ |
| | | | | | RHD*DAR / RHD*DIVa-2 | Partial D, C⁻ |
| | | | | | RHD*DAR-E / RHD*DIVa-2 | Partial D, C⁻ |
| Absent | Present | Present | C/G | C | Not Reported | |
| Absent | Present | Present | C/G | Absent | Not Reported | |
| Absent | Present | Present | G | G | RHD*weakDtype4.0 / RHD*DIIIa | Weak or Partial D, C⁻ |
| | | | | | RHD*weakDtype4.1 / RHD*DIIIa | Weak or Partial D, C⁻ |
| | | | | | RHD*weakDtype14 / RHD*DIIIa | Weak or Partial D, C⁻ |
| | | | | | RHD*weakDtype51 / RHD*DIIIa | Weak or Partial D, C⁻ |
| | | | | | RHD*DAR / RHD*DIIIa | Partial D?, C⁻ |
| | | | | | RHD*DAR-E / RHD*DIIIa | Partial D?, C⁻ |
| | | | | | RHD*weakDtype4.0 / RHD*DIIIa-CE(4-7)-D) | Weak D, C^{+W} |
| | | | | | RHD*weakDtype4.1 / RHD*DIIIa-CE(4-7)-D) | Weak D, C^{+W} |
| | | | | | RHD*weakDtype14 / RHD*DIIIa-CE(4-7)-D) | Weak D, C^{+W} |
| | | | | | RHD*weakDtype51 / RHD*DIIIa-CE(4-7)-D) | Weak D, C^{+W} |
| | | | | | RHD*DAR / RHD*DIIIa-CE(4-7)-D) | Partial D, C^{+W} |
| | | | | | RHD*DAR-E / RHD*DIIIa-CE(4-7)-D) | Partial D, C^{+W} |
| Absent | Present | Present | G | G/C | Not Reported | |
| Absent | Present | Present | G | C | Not Reported | |
| Absent | Present | Present | G | Absent | Not Reported | |
| Absent | Present | Present | Absent | G | Not Reported | |
| Absent | Present | Present | Absent | G/C | Not Reported | |
| Absent | Present | Present | Absent | C | Not Reported | |
| Absent | Present | Present | Absent | Absent | RHD*ex04-ex07del | D⁻, C⁻ |
| Absent | Present | Absent | C | G | RHD*DIIIa-CE(4-7)-D / RHD*ex03del | D⁻, C^{+W} |
| Absent | Present | Absent | C | G/C | RHD*DIVa-2 / RHD*ex03del | Partial D, C⁻ |
| Absent | Present | Absent | C | C | RHD*DIVa-2 / No RHD | Partial D, C⁻ |
| | | | | | RHD*DIVa-2 / RHD*DIVa-2 | Partial D, C⁻ |
| | | | | | RHD*DIVa-2 / RHD*DIIIa-CE(4-7)-D | Partial D, C^{+W} |
| Absent | Present | Absent | C | Absent | Not Reported | |
| Absent | Present | Absent | C/G | G | RHD*DIIIa / RHD*ex03del | Partial D, C⁻ |
| Absent | Present | Absent | C/G | G/C | RHD*DIIIa / RHD*DIVa-2 | Partial D, C⁻ |
| Absent | Present | Absent | C/G | C | Not Reported | |
| Absent | Present | Absent | C/G | Absent | Not Reported | |
| Absent | Present | Absent | G | G | RHD*DIIIa / No RHD | Partial D, C⁻ |
| | | | | | RHD*DIIIa / RHD*DIIIa | Partial D, C⁻ |
| | | | | | RHD*DIIIa / RHD*DIIIa-CE(4-7)-D | Partial D, C^{+W} |
| Absent | Present | Absent | G | G/C | RHD*DIIIa / RHD*ex03-ex04del | Partial D, C⁻ |
| Absent | Present | Absent | G | C | Not Reported | |
| Absent | Present | Absent | G | Absent | Not Reported | |
| Absent | Present | Absent | Absent | G | RHD*DIIIa-CE(4-7)-D / RHD*ex03-ex04del | D⁻, C^{+W} |
| Absent | Present | Absent | Absent | G/C | Not Reported | |
| Absent | Present | Absent | Absent | C | Not Reported | |
| Absent | Present | Absent | Absent | Absent | RHD*DIIIa-CE(4-7)-D / No RHD | D⁻, C^{+W} |
| | | | | | RHD*DIIIa-CE(4-7)-D / RHD*DIIIa-CE(4-7)-D | D⁻, C^{+W} |
| Absent | Absent | Present | C | G | Possibly RHD | D?, C⁻ |
| Absent | Absent | Present | C | G/C | RHD*DIVb-4 / Possibly RHD | D?, C⁻ |
| Absent | Absent | Present | C | C | RHD*DIVb-4 / RHD*DIVb-4 | Partial D, C⁻ |
| Absent | Absent | Present | C | Absent | Not Reported | |
| Absent | Absent | Present | C/G | G | RHD*weakDtype4.0 / Possibly RHD | D?, C⁻ |
| | | | | | RHD*weakDtype4.1 / Possibly RHD | D?, C⁻ |
| | | | | | RHD*weakDtype14 / Possibly RHD | D?, C⁻ |
| | | | | | RHD*weakDtype51 / Possibly RHD | D?, C⁻ |
| | | | | | RHD*DAR / Possibly RHD | D?, C⁻ |
| | | | | | RHD*DAR-E / Possibly RHD | D?, C⁻ |
| Absent | Absent | Present | C/G | G/C | RHD*weakDtype4.0/ RHD*DIVb-4 | Weak or Partial D, C⁻ |
| | | | | | RHD*weakDtype4.01 / RHD*DIVb-4 | Weak or Partial D, C⁻ |
| | | | | | RHD*weakDtype4.1 / RHD*DIVb-4 | Weak or Partial D, C⁻ |
| | | | | | RHD*weakDtype14 / RHD*DIVb-4 | Weak or Partial D, C⁻ |
| | | | | | RHD*weakDtype51 / RHD*DIVb-4 | Weak or Partial D, C⁻ |
| | | | | | RHD*DAR / RHD*DIVb-4 | Partial D, C⁻ |
| | | | | | RHD*DAR-E / RHD*DIVb-4 | Partial D, C⁻ |
| Absent | Absent | Present | C/G | C | Not Reported | |
| Absent | Absent | Present | C/G | Absent | Not Reported | |
| Absent | Absent | Present | G | G | RHD*weakDtype4.0 / RHD*weakDtype4.0 | Weak D, C- |
| | | | | | RHD*weakDtype4.1 / RHD*weakDtype4.0 | Weak D, C⁻ |
| | | | | | RHD*weakDtype14 / RHD*weakDtype4.0 | Weak D, C⁻ |
| | | | | | RHD*weakDtype51 / RHD*weakDtype4.0 | Weak D, C⁻ |
| | | | | | RHD*DAR / RHD*weakDtype4.0 | Weak or Partial D, C⁻ |
| | | | | | RHD*DAR-E / RHD*weakDtype4.0 | Weak or Partial D, C⁻ |
| | | | | | RHD*weakDtype4.0 / RHD*weakDtype4.1 | Weak D, C⁻ |
| | | | | | RHD*weakDtype4.1 / RHD*weakDtype4.1 | Weak D, C⁻ |
| | | | | | RHD*weakDtype14 / RHD*weakDtype4.1 | Weak D, C⁻ |
| | | | | | RHD*weakDtype51 / RHD*weakDtype4.1 | Weak D, C⁻ |
| | | | | | RHD*DAR / RHD*weakDtype4.1 | Weak or Partial D, C⁻ |
| | | | | | RHD*DAR-E / RHD*weakDtype4.1 | Weak or Partial D, C⁻ |
| | | | | | RHD*weakDtype4.0/ / RHD*weakDtype14 | Weak D, C⁻ |
| | | | | | RHD*weakDtype4.1 / RHD*weakDtype14 | Weak D, C⁻ |
| | | | | | RHD*weakDtype14 / RHD*weakDtype14 | Weak D, C⁻ |
| | | | | | RHD*weakDtype51 / RHD*weakDtype14 | Weak D, C⁻ |
| | | | | | RHD*DAR / RHD*weakDtype14 | Weak or Partial D, C- |
| | | | | | RHD*DAR-E / RHD*weakDtype14 | Weak or Partial D, C⁻ |
| | | | | | RHD*weakDtype4.0 / RHD*weakDtype51 | Weak D, C⁻ |
| | | | | | RHD*weakDtype4.1 / RHD*weakDtype51 | Weak D, C⁻ |
| | | | | | RHD*weakDtype14 / RHD*weakDtype51 | Weak D, C⁻ |
| | | | | | RHD*weakDtype51 / RHD*weakDtype51 | Weak D, C⁻ |
| | | | | | RHD*DAR / RHD*weakDtype51 | Weak or Partial D, C⁻ |
| | | | | | RHD*DAR-E RHD*weakDtype51 | Weak or Partial D, C⁻ |
| | | | | | RHD*weakDtype4.0 / RHD*DAR | Weak or Partial D, C⁻ |
| | | | | | RHD*weakDtype4.1 / RHD*DAR | Weak or Partial D, C⁻ |
| | | | | | RHD*weakDtype14 / RHD*DAR | Weak or Partial D, C⁻ |
| | | | | | RHD*weakDtype51 / RHD*DAR | Weak or Partial D, C⁻ |
| | | | | | RHD*DAR / RHD*DAR | Partial D, C⁻ |
| | | | | | RHD*DAR-E / RHD*DAR | Partial D, C⁻ |
| | | | | | RHD*weakDtype4.0 / RHD*DAR-E | Weak or Partial D, C⁻ |
| | | | | | RHD*weakDtype4.1 / RHD*DAR-E | Weak or Partial D, C⁻ |
| | | | | | RHD*weakDtype14 / RHD*DAR-E | Weak or Partial D, C⁻ |
| | | | | | RHD*weakDtype51 / RHD*DAR-E | Weak or Partial D, C⁻ |
| | | | | | RHD*DAR / RHD*DAR-E | Partial D, C⁻ |
| | | | | | RHD*DAR-E / RHD*DAR-E | Partial D, C⁻ |
| Absent | Absent | Present | G | G/C | Not Reported | |
| Absent | Absent | Present | G | C | Not Reported | |
| Absent | Absent | Present | G | Absent | Not Reported | |
| Absent | Absent | Present | Absent | G | Not Reported | |
| Absent | Absent | Present | Absent | G/C | Not Reported | |
| Absent | Absent | Present | Absent | C | Not Reported | |
| Absent | Absent | Present | Absent | Absent | RHD*ex04-ex07del / RHD*ex04-ex07del | D⁻, C⁻ |
| | | | | | RHD*ex04-ex07del / No RHD | D⁻, C⁻ |
| Absent | Absent | Absent | C | G | RHD*ex03del/ RHD*ex03del | D⁻, C⁻ |
| | | | | | RHD*ex03del / No RHD | D⁻, C⁻ |
| Absent | Absent | Absent | C | G/C | Not Reported | |
| Absent | Absent | Absent | C | C | Not Reported | |
| Absent | Absent | Absent | C | Absent | Not Reported | |
| Absent | Absent | Absent | C/G | G | Not Reported | |
| Absent | Absent | Absent | C/G | G/C | Not Reported | |
| Absent | Absent | Absent | C/G | C | Not Reported | |
| Absent | Absent | Absent | C/G | Absent | Not Reported | |
| Absent | Absent | Absent | G | G | Not Reported | |
| Absent | Absent | Absent | G | G/C | Not Reported | |
| Absent | Absent | Absent | G | C | Not Reported | |
| Absent | Absent | Absent | G | Absent | Not Reported | |
| Absent | Absent | Absent | Absent | G | RHD*ex03-ex04del / RHD*ex03-ex04del | D⁻, C⁻ |
| | | | | | RHD*ex03-ex04del / No RHD | D⁻, C⁻ |
| Absent | Absent | Absent | Absent | G/C | Not Reported | |
| Absent | Absent | Absent | Absent | C | Not Reported | |
| Absent | Absent | Absent | Absent | Absent | No RHD / No RHD | D⁻, C⁻ |
| Present | Present | Present | C | G | RHD*DIIIa-CE(4-7)-D / Possibly RHD | D?, C⁺ |
| Present | Present | Present | C | G/C | RHD*DIVa-2 / Possibly RHD | D?, C⁺ |
| Present | Present | Present | C | C | RHD*DIVa-2 / RHD*DIVb-4 | Partial D, C⁺ |
| Present | Present | Present | C | Absent | Not Reported | |
| Present | Present | Present | C/G | G | RHD*DIIIa / Possibly RHD | D?, C⁺ |
| Present | Present | Present | C/G | G/C | RHD*DIIIa / RHD*DIVb-4 | Partial D, C⁺ |
| | | | | | RHD*weakDtype4.0 / RHD*DIVa-2 | Weak or Partial D, C⁺ |
| | | | | | RHD*weakDtype4.1 / RHD*DIVa-2 | Weak or Partial D, C⁺ |
| | | | | | RHD*weakDtype14 / RHD*DIVa-2 | Weak or Partial D, C⁺ |
| | | | | | RHD*weakDtype51/ RHD*DIVa-2 | Weak or Partial D, C⁺ |
| | | | | | RHD*DAR / RHD*DIVa-2 | Partial D, C⁺ |
| | | | | | RHD*DAR-E / RHD*DIVa-2 | Partial D, C⁺ |
| Present | Present | Present | C/G | C | Not Reported | |
| Present | Present | Present | C/G | Absent | Not Reported | |
| Present | Present | Present | G | G | RHD*weakDtype4.0 / RHD*DIIIa | Weak or Partial D, C⁺ |
| | | | | | RHD*weakDtype4.1 / RHD*DIIIa | Weak or Partial D, C⁺ |
| | | | | | RHD*weakDtype14 / RHD*DIIIa | Weak or Partial D, C⁺ |
| | | | | | RHD*weakDtype51 / RHD*DIIIa | Weak or Partial D, C⁺ |
| | | | | | RHD*DAR / RHD*DIIIa | Partial D?, C⁺ |
| | | | | | RHD*DAR-E / RHD*DIIIa | Partial D?, C⁺ |
| | | | | | RHD*weakDtype4.0 / RHD*DIIIa-CE(4-7)-D) | Weak D, C⁺ |
| | | | | | RHD*weakDtype4.1 / RHD*DIIIa-CE(4-7)-D) | Weak D, C⁺ |
| | | | | | RHD*weakDtype14 / RHD*DIIIa-CE(4-7)-D) | Weak D, C⁺ |
| | | | | | RHD*weakDtype51 / RHD*DIIIa-CE(4-7)-D) | Weak D, C⁺ |
| | | | | | RHD*DAR / RHD*DIIIa-CE(4-7)-D) | Partial D, C⁺ |
| | | | | | RHD*DAR-E / RHD*DIIIa-CE(4-7)-D) | Partial D, C⁺ |
| Present | Present | Present | G | G/C | Not Reported | |
| Present | Present | Present | G | C | Not Reported | |
| Present | Present | Present | G | Absent | Not Reported | |
| Present | Present | Present | Absent | G | Not Reported | |
| Present | Present | Present | Absent | G/C | Not Reported | |
| Present | Present | Present | Absent | C | Not Reported | |
| Present | Present | Present | Absent | Absent | RHD*ex04-ex07del | D⁻, C⁺ |
| Present | Present | Absent | C | G | RHD*DIIIa-CE(4-7)-D / RHD*ex03del | D⁻, C⁺ |
| Present | Present | Absent | C | G/C | RHD*DIVa-2 / RHD*ex03del | Partial D, C⁺ |
| Present | Present | Absent | C | C | RHD*DIVa-2 / No RHD | Partial D, C⁺ |
| | | | | | RHD*DIVa-2/ RHD*DIVa-2 | Partial D, C⁺ |
| | | | | | RHD*DIVa-2 / RHD*DIIIa-CE(4-7)-D | Partial D, C⁺ |
| Present | Present | Absent | C | Absent | Not Reported | |
| Present | Present | Absent | C/G | G | RHD*DIIIa / RHD*ex03del | Partial D, C⁺ |
| Present | Present | Absent | C/G | G/C | RHD*DIIIa / RHD*DIVa-2 | Partial D, C⁺ |
| Present | Present | Absent | C/G | C | Not Reported | |
| Present | Present | Absent | C/G | Absent | Not Reported | |
| Present | Present | Absent | G | G | RHD*DIIIa / No RHD | Partial D, C⁺ |
| | | | | | RHD*DIIIa / RHD*DIIIa | Partial D, C⁺ |
| | | | | | RHD*DIIIa / RHD*DIIIa-CE(4-7)-D | Partial D, C⁺ |
| Present | Present | Absent | G | G/C | RHD*DIIIa / RHD*ex03-ex04del | Partial D, C⁺ |
| Present | Present | Absent | G | C | Not Reported | |
| Present | Present | Absent | G | Absent | Not Reported | |
| Present | Present | Absent | Absent | G | RHD*DIIIa-CE(4-7)-D / RHD*ex03-ex04del | D⁻, C⁺ |
| Present | Present | Absent | Absent | G/C | Not Reported | |
| Present | Present | Absent | Absent | C | Not Reported | |
| Present | Present | Absent | Absent | Absent | RHD*DIIIa-CE(4-7)-D / No RHD | D⁻, C⁺ |
| | | | | | RHD*DIIIa-CE(4-7)-D / RHD*DIIIa-CE(4-7)-D | D⁻, C⁺ |
| Present | Absent | Present | C | G | Possibly RHD | D?, C⁺ |
| Present | Absent | Present | C | G/C | RHD*DIVb-4 / Possibly RHD | D?, C⁺ |
| Present | Absent | Present | C | C | RHD*DIVb-4 / RHD*DIVb-4 | Partial D, C⁺ |
| Present | Absent | Present | C | Absent | Not Reported | |
| Present | Absent | Present | C/G | G | RHD*weakDtype4.0 / Possibly RHD | D?, C⁺ |
| | | | | | RHD*weakDtype4.1 / Possibly RHD | D?, C⁺ |
| | | | | | RHD*weakDtype14 / Possibly RHD | D?, C⁺ |
| | | | | | RHD*weakDtype51 / Possibly RHD | D?, C⁺ |
| | | | | | RHD*DAR / Possibly RHD | D?, C⁺ |
| | | | | | RHD*DAR-E / Possibly RHD | D?, C⁺ |
| Present | Absent | Present | C/G | G/C | RHD*weakDtype4.0 / RHD*DIVb-4 | Weak or Partial D, C⁺ |
| | | | | | RHD*weakDtype4.01 / RHD*DIVb-4 | Weak or Partial D, C⁺ |
| | | | | | RHD*weakDtype4.1 / RHD*DIVb-4 | Weak or Partial D, C⁺ |
| | | | | | RHD*weakDtype14 / RHD*DIVb-4 | Weak or Partial D, C⁺ |
| | | | | | RHD*weakDtype51 / RHD*DIVb-4 | Weak or Partial D, C⁺ |
| | | | | | RHD*DAR / RHD*DIVb-4 | Partial D, C⁺ |
| | | | | | RHD*DAR-E / RHD*DIVb-4 | Partial D, C⁺ |
| Present | Absent | Present | C/G | C | Not Reported | |
| Present | Absent | Present | C/G | Absent | Not Reported | |
| Present | Absent | Present | G | G | RHD*weakDtype4.0 / RHD*weakDtype4.0 | Weak D, C⁺ |
| | | | | | RHD*weakDtype4.1 / RHD*weakDtype4.0 | Weak D, C⁺ |
| | | | | | RHD*weakDtype14 / RHD*weakDtype4.0 | Weak D, C⁺ |
| | | | | | RHD*weakDtype51 / RHD*weakDtype4.0 | Weak D, C⁺ |
| | | | | | RHD*DAR / RHD*weakDtype4.0 | Weak or Partial D, C⁺ |
| | | | | | RHD*DAR-E / RHD*weakDtype4.0 | Weak or Partial D, C⁺ |
| | | | | | RHD*weakDtype4.0 / RHD*weakDtype4.1 | Weak D, C⁺ |
| | | | | | RHD*weakDtype4.1 / RHD^{*}weakDtype4.1 | Weak D, C⁺ |
| | | | | | RHD*weakDtype14 / RHD^{*}weakDtype4.1 | Weak D, C⁺ |
| | | | | | RHD*weakDtype51 / RHD*weakDtype4.1 | Weak D, C⁺ |
| | | | | | RHD*DAR / RHD*weakDtype4.1 | Weak or Partial D, C⁺ |
| | | | | | RHD*DAR-E / RHD*weakDtype4.1 | Weak or Partial D, C⁺ |
| | | | | | RHD*weakDtype4.01 / RHD*weakDtype14 | Weak D, C⁺ |
| | | | | | RHD*weakDtype4.1 / RHD*weakDtype14 | Weak D, C⁺ |
| | | | | | RHD*weakDtype14 / RHD*weakDtype14 | Weak D, C⁺ |
| | | | | | RHD*weakDtype51 / RHD*weakDtype14 | Weak D, C⁺ |
| | | | | | RHD*DAR / RHD*weakDtype14 | Weak or Partial D, C⁺ |
| | | | | | RHD*DAR-E / RHD*weakDtype14 | Weak or Partial D, C⁺ |
| | | | | | RHD*weakDtype4.01 / RHD*weakDtype51 | Weak D, C⁺ |
| | | | | | RHD*weakDtype4.1 / RHD^{*}weakDtype51 | Weak D, C⁺ |
| | | | | | RHD*weakDtype14 / RHD*weakDtype51 | Weak D, C⁺ |
| | | | | | RHD*weakDtype51 / RHD*weakDtype51 | Weak D, C⁺ |
| | | | | | RHD*DAR / RHD*weakDtype51 | Weak or Partial D, C⁺ |
| | | | | | RHD*DAR-E / RHD*weakDtype51 | Weak or Partial D, C⁺ |
| | | | | | RHD*weakDtype4.0 / RHD*DAR | Weak or Partial D, C⁺ |
| | | | | | RHD*weakDtype4.1 / RHD*DAR | Weak or Partial D, C⁺ |
| | | | | | RHD*weakDtype14 / RHD*DAR | Weak or Partial D, C⁺ |
| | | | | | RHD*weakDtype51 / RHD*DAR | Weak or Partial D, C⁺ |
| | | | | | RHD*DAR / RHD*DAR | Partial D, C⁺ |
| | | | | | RHD*DAR-E / RHD*DAR | Partial D, C⁺ |
| | | | | | RHD*weakDtype4.0 / RHD*DAR-E | Weak or Partial D, C⁺ |
| | | | | | RHD*weakDtype4.1 / RHD*DAR-E | Weak or Partial D, C⁺ |
| | | | | | RHD*weakDtype14 / RHD*DAR-E | Weak or Partial D, C⁺ |
| | | | | | RHD*weakDtype51 / RHD*DAR-E | Weak or Partial D, C⁺ |
| | | | | | RHD*DAR / RHD*DAR-E | Partial D, C⁺ |
| | | | | | RHD*DAR-E / RHD*DAR-E | Partial D, C⁺ |
| Present | Absent | Present | G | G/C | Not Reported | |
| Present | Absent | Present | G | C | Not Reported | |
| Present | Absent | Present | G | Absent | Not Reported | |
| Present | Absent | Present | Absent | G | Not Reported | |
| Present | Absent | Present | Absent | G/C | Not Reported | |
| Present | Absent | Present | Absent | C | Not Reported | |
| Present | Absent | Present | Absent | Absent | RHD*ex04-ex07del / RHD*ex04-ex07del | D⁻, C⁺ |
| | | | | | RHD*ex04-ex07del / No RHD | D⁻, C⁺ |
| Present | Absent | Absent | C | G | RHD*ex03del / RHD*ex03del | D⁻, C⁺ |
| | | | | | RHD*ex03del / No RHD | D⁻, C⁺ |
| Present | Absent | Absent | C | G/C | Not Reported | |
| Present | Absent | Absent | C | C | Not Reported | |
| Present | Absent | Absent | C | Absent | Not Reported | |
| Present | Absent | Absent | C/G | G | Not Reported | |
| Present | Absent | Absent | C/G | G/C | Not Reported | |
| Present | Absent | Absent | C/G | C | Not Reported | |
| Present | Absent | Absent | C/G | Absent | Not Reported | |
| Present | Absent | Absent | G | G | Not Reported | |
| Present | Absent | Absent | G | G/C | Not Reported | |
| Present | Absent | Absent | G | C | Not Reported | |
| Present | Absent | Absent | G | Absent | Not Reported | |
| Present | Absent | Absent | Absent | G | RHD*ex03-ex04del / RHD*ex03-ex04del | D⁻, C⁺ |
| | | | | | RHD*ex03-ex04del / No RHD | D⁻, C⁺ |
| Present | Absent | Absent | Absent | G/C | Not Reported | |
| Present | Absent | Absent | Absent | C | Not Reported | |
| Present | Absent | Absent | Absent | Absent | No RHD / No RHD | D⁻, C⁺ |

**Table 2**

| **Antigen** | **Phenotype 1** | **Phenotype 2** | **Sample Phenotype** |
|---|---|---|---|
| RhD | D⁻ | D⁻ | D⁻ |
| | D⁻ | Weak D | Weak D |
| | D⁻ | Partial D | Partial D |
| | D⁻ | D⁺ | D⁺ |
| | Weak D | Weak D | Weak D |
| | Weak D | Partial D | Weak or Partial D |
| | Weak D | D⁺ | D⁺ |
| | Partial D | Partial D | Partial D |
| | Partial D | D⁺ | D⁺ |
| | D⁺ | D⁺ | D⁺ |
| RhC | C⁻ | C⁻ | C⁻ |
| | C⁻ | C^{+W} | C^{+W} |
| | C⁻ | C⁺ | C⁺ |
| | C^{+W} | C^{+W} | C^{+W} |
| | C^{+W} | C⁺ | C⁺ |
| | C⁺ | C⁺ | C⁺ |

### Examples

### Identification of genetic variants that encode no D antigen (D⁻) and altered C antigen (C^{+W})

The following example relates to a method of identifying *RHD*DIIIa-CE(4-7)-D* or *RHD*DIIIa-CE(4-7)-D)*-like variants. The method described herein has been applied to 58 samples previously known to contain *RHD*/*RHCE* hybrid exon 3. The process described below proceeds from the genotyping of said samples and the subsequent analysis of said samples grouped by genotype and/or predicted phenotype. The serotype assigned to a group corresponds to analysis performed only on a subset of the samples in said group.

### Materials & Methods

Genomic DNA was extracted from nucleated cells in a blood sample by cell lysis. Extracted DNA was purified on an affinity column. Both cell lysis and DNA purification were performed with a QIAamp Blood kit (Qiagen, Germany) by following manufacturer protocols and recommendations. Purity of DNA was determined by spectrophotometry on a Nanodrop instrument (Nanodrop, DE). Only DNA solutions with an OD_{260/280} 1.8±0.2 proceeded to subsequent analysis.

Purified DNA was used as a template for multiplexed Polymerase Chain Reaction (PCR) amplification of the gene segments of interest in a GeneAmp 9700 thermal cycler (Perkin-Elmer, CA). Primer sequences for the different segments are listed in the Technical Description section below. Cycling conditions consisted of a denaturation/polymerase activation step at 95°C for 15 min, followed by 38 cycles of denaturation at 95°C for 45 sec, annealing at 60°C for 60 sec, extension at 72°C for 90 sec, and a final extension step at 72°C for 10 min.

Amplified DNA was enzymatically fragmented by incubation with DNase I (Promega, WI) and alkaline phosphatase (Roche, Germany) at 37°C for 30 min, followed by enzyme inactivation at 95°C for 10 min.

Fragmented DNA was labeled by incubation with TdT enzyme (Roche, Germany) and biotinddUTP (Perkin-Elmer, CA) or Cy5-dCTP (Perkin-Elmer, CA) at at 37°C for 60 min.

Labelled DNA was placed on a Progenika proprietary microarray. The microarray comprised a modified crystal surface to which allele-specific oligonucleotide probes were covalently attached. Probes were designed to interrogate multiple allelic variant positions (i.e. markers) in the amplified genomic segments. Each allelic variant was interrogated by 2 probes, for a total of 4 probes per marker. Each probe was printed 10 times on the microarray, for a total of 40 features (spots) per SNP. Probe sequences are listed in the Technical Description section below. The labelled DNA/microarray interface was placed in an incubation chamber of a HS 4800 Pro station (Tecan, Switzerland) and incubated at 47°C for 30 min and at 45°C for 60 min in buffer containing SSPE, dextran, and deionized formamide to allow for probes to hybridize (bind) to their cognate sequences, when present. Unbound DNA was washed off by incubation at 23°C for various times with buffer containing SSC with or without SDS. A streptavidin-Cy3 conjugate (Invitrogen, CA) diluted in buffer containing PBS and Tween-20 was added to the microarray surface and further incubated at 37°C for 10 min. Unbound conjugate was washed off as before. The microarray was dried by flushing high-pressure liquid nitrogen through the incubation chamber.

Hybridized DNA was fluorescently labelled, either directly with Cy5 (by the transferase reaction above) or via the interaction between streptavidin-Cy3 conjugate and biotin (last steps of the hybridization), and immobilized on the microarray by sequence specific base pairing to their respective probe. Microarray-bound Cy3 and Cy5 fluorescence was detected on an InnoScan 710 confocal scanner (Innopsys, France). This scanner uses two laser beams with appropriate wavelengths for excitation of the Cy3 and Cy5 fluorophores and PMT sensors for the detection of the fluorescence signals generated. The fluorescence signal of each feature was subsequently quantified by *ad hoc* software.

Progenika proprietary software was used to transform fluorescence intensity values for the particular allelic variants detected, singly or in combination, into blood group genotypes, and from genotypes into predicted blood group phenotypes.

Serology analysis may be performed using methods well known to the skilled person. Suitable protocols may be found in, for example, The Blood Group Antigen FactsBook, Second edition. 2004. M. E. Reid and C. Lomas-Francis. Elsevier Ltd., and references cited therein to serology technical manuals.

### Technical description

According to the present example, amplifications and hybridizations for determination of the five genetic sequences were performed as follows:

### Amplification of RHCE*C intron 2 by PCR.

- The following primers were used, which yielded a PCR product with a size of 357 base pairs: Forward primer: 5'-**GGCCACCACCATTTGAA**-3' (SEQ ID NO: 3)
- Reverse primer: 5'-CCATGAACATGCCACTTCAC-3' (SEQ ID NO: 4)
In boldface, *RHCE*C*-specific nucleotides (forward primer).

### Amplification of RHD/RHCE hybrid exon 3 by PCR.

The following primers were used, which yielded a PCR product with a size of 256 base pairs:
- Forward primer: 5'-TCCTGGCTCTCC**C**TCTC**T**-3' (SEQ ID NO: 9)
- Reverse primer: 5'-TTTTCAAAACCC**C**GGAA**G**-3' (SEQ ID NO: 10)
In boldface, *RHD*-specific nucleotides (forward primer) and RHCE-specific nucleotides (reverse primer).

### Amplification of RHD exon 3 by PCR.

The following primers were used, which yielded a PCR product with a size of 268 base pairs:
- Forward primer: 5'-TCCTGGCTCTCCCTCTC**T**-3' (SEQ ID NO: 15)
- Reverse primer: 5'-GTTGTCTTTATTTTTCAAAACCC**T**-3' (SEQ ID NO: 16)
In boldface, *RHD*-specific nucleotides.

### Amplification of RHD exon 4 by PCR.

The following primers were used, which yielded a PCR product with a size of 281 base pairs:
- Forward primer: 5'-GCTCTGAACTTTCTCCAAGGAC**T**-3' (SEQ ID NO: 17)
- Reverse primer: 5'-ATTCTGCTCAGCCCAAGTA**G**-3' (SEQ ID NO: 18)
In boldface, *RHD*-specific nucleotides.

### Amplification of RHD exon 7 by PCR.

The following primers were used, which yielded a PCR product with a size of 695 base pairs:
- Forward primer: 5'-ACAAACTCCCC**GA**TGATGTGAGT**G**-3' (SEQ ID NO: 35)
- Reverse primer: 5'-GAGGCTGAGAAAGGTTAAGCC**A**-3' (SEQ ID NO: 36)
In boldface, *RHD*-specific nucleotides.

### Hybridization of RHCE*C intron 2 amplicon to oligonucleotide probes.

The following probe sequences were used to determine the presence or absence of this amplicon:
- *RHCE*C*-specific perfect match probe #1: 5'-TTTTACAGACGCCTGCTACCATG-3' (SEQ ID NO: 5)
- *RHCE*C*-specific perfect match probe #2: 5'-CATGGTAGCAGGCGTCTGTAAAA-3' (SEQ ID NO: 6)
- Mismatch probe #1: 5'-TTTTACAGACG**T**CTGCTACCATG-3' (SEQ ID NO: 7)
- Mismatch probe #2: 5'-CATGGTAGCAG**A**CGTCTGTAAAA-3' (SEQ ID NO: 8)
In boldface, the central position mismatch.

### Hybridization of RHD exon 3 amplicon or RHD/RHCE hybrid exon 3 amplicon to oligonucleotide probes.

The following probe sequences were used to determine the presence or absence of both amplicons, using the SNP located at position 410 of both RHD and RHD/RHCE exon 3. The two amplicons were distinguished by using different label molecules (Cy5-dCTP or biotinddUTP) in the terminal transferase reaction described above.
- *RHD, RHCE* wild-type probe #1: 5'-GGTCAACTTGG**C**GCAGTTGGTGG-3' (SEQ ID NO: 11)
- RHD, RHCE wild-type probe #2: 5'-GTCAAC**T**TGG**C**GCAGTTGGTG-3' (SEQ ID NO: 12)
- Hybrid exon 3 variant probe #1: 5'-GGTCAACTTGG**T**GCAGTTGGTGG-3' (SEQ ID NO: 13)
- Hybrid exon 3 variant probe #2: 5'-GTCAACTTGG**T**GCAGTTGGTG-3' (SEQ ID NO: 14)
In boldface, the SNP.

### Hybridization of RHD exon 4 amplicon to oligonucleotide probes

The following probe sequences were used to determine the presence, absence and SNP variant (either C or G) for the SNP located at position 602 of the coding sequence:
- *RHD* wild-type probe #1: 5'-ATAAAGATCAGACAGCAACGATACC-3' (SEQ ID NO: 23)
- *RHD* wild-type probe #2: 5'-TAAAGATCAGACAGCAACGATAC-3' (SEQ ID NO: 24)
- *RHD*DIIIa* variant probe #1: 5'-ATAAAGATCAGAGAGCAACGATACC-3' (SEQ ID NO: 25)
- *RHD*DIIIa* variant probe #2: 5'-TAAAGATCAGAGAGCAACGATAC-3' (SEQ ID NO: 26)
In boldface, the SNP.

### Hybridization of RHD exon 7 amplicon to oligonucleotide probes.

The following probe sequences were used to determine the presence, absence and SNP variant (either G or C) for the SNP located at position 1048 of the coding sequence:
- *RHD* wild-type probe #1: 5'-TGCTGGTGCTT**G**ATACCGTCGGA-3' (SEQ ID NO: 37)
- *RHD* wild-type probe #2: 5'-GCTGGTGCTT**G**ATACCGTCGG-3' (SEQ ID NO: 38)
- *RHD*DIVa-2* variant probe #1: 5'-TGCTGGTGCTT**C**ATACCGTCGGA-3' (SEQ ID NO: 39)
- *RHD*DIVa-2 variant* probe #2: 5'-GCTGGTGCTT**C**ATACCGTCGG-3' (SEQ ID NO: 40) In boldface, the SNP.

### Grouping of samples by genotype combination

Analysis by the method described above of 146 samples previously known to contain *RHD*/*RHCE* hybrid exon 3 yielded the results shown below. Samples were grouped by genotype and/or predicted phenotype. Serotype analysis was also performed on a subset of the samples in each group. Serotype analysis is unable to distinguish between C⁺ and C^{+W} antigen phenotypes, demonstrating the inaccurate results that can be obtained using this method. There are also some types of partial, weak and D- phenotypes that cannot be distinguished by serology.

### Group 1

### Number of Samples: 11

Sample IDs: 09-0084, 10-0210, 10-0380, 10-0635, 10-0972, 10-2366, 10-2367, 10-3113, 10-3649, 10-3664, 10-3809

Genotyping data : *RHCE*C* intron 2 present, RHD/CE Hex03 present, *RHD* exon 3 present, *RHD* exon 4 602C, *RHD* exon 7 1048G.
- *RHD* haplotype 1: *RHD*DIIIa-CE(4-7)-D*
   *RHD* haplotype 2: Possibly *RHD*D*
   Predicted RHD phenotype: D?
   Serotype: Not available
- *RHCE* haplotype 1: *RHCE*c*
   *RHCE* haplotype 2: *RHCE*C*
   Predicted RHCE C phenotype: C⁺
   Serotype: C⁺

Determining the markers described herein correctly predicted that the clinical phenotype was not *RHD*DIIIa-CE(4-7)-D, RHD*DIIIa* or *RHD*DIVa-2.*

### Group 2

### Number of Samples: 49

Sample IDs: 09-0216, 09-0294, 10-0056, 10-0097, 10-0118, 10-0280, 10-0367, 10-0371, 10-0373, 10-0376, 10-0389, 10-0396, 10-0428, 10-0461, 10-0476, 10-0575, 10-0598, 10-0654, 10-0752, 10-0773, 10-0790, 10-0849, 10-0867, 10-0933, 10-1391, 10-1423, 10-1500, 10-1591, 10-1599, 10-1643, 10-1653, 10-2038, 10-2153, 10-2155, 10-2212, 10-2321, 10-2347, 10-2758, 10-3387, 10-3400, 10-3417, 10-3426, 10-3486, 10-3528, 10-3545, 10-3625, 10-3635, 10-3684, 10-3694

Genotyping data: *RHCE*C* intron 2 absent, RHD/CE Hex03 present, *RHD* exon 3 present, *RHD* exon 4 602C, *RHD* exon 7 1048G.
- *RHD* haplotype 1: *RHD*DIIIa-CE(4-7)-D*
   *RHD* haplotype 2: Possibly *RHD*D*
   Predicted RHD phenotype: D?
   Serotype: D⁺
- *RHCE* haplotype 1: *RHCE*c*
   *RHCE* haplotype 2: *RHCE*c*
   Predicted RHCE C phenotype: C^{+W}
   Serotype: C⁺

The method determining the markers described herein predicted that the clinical phenotype could be *RHD*DIIIa-CE(4-7)-D,* but could not be *RHD*DIIIa* or *RHD*DIVa-2.* For the patients actually tested, the D antigen was present, and therefore the phenotype was not *RHD*DIIIa-CE(4-7)-D.* These data also show the serotype analysis incorrectly reported a C⁺ phenotype instead of C^{+W}; in the absence of *RHCE^{*}C* intron 2, the actual phenotype could not be C⁺.

### Group 3

### Number of Samples: 24

Sample IDs: 10-0085, 10-0177, 10-0443, 10-0656, 10-0715, 10-0847, 10-0853, 10-0900, 10-1374, 10-1455, 10-1532, 10-1577, 10-1588, 10-1649, 10-1661, 10-2220, 10-2238, 10-2335, 10-3392, 10-3427, 10-3461, 10-3561, 10-3718, 10-4060

Genotyping data: *RHCE*C* intron 2 absent, RHD/CE Hex03 present, *RHD* exon 3 absent, *RHD* exon 4 602 absent, *RHD* exon 7 1048 absent.
- *RHD* haplotype 1: *RHD*DIIIa-CE(4-7)-D*
   *RHD* haplotype 2: *RHD*DIIIa-CE(4-7)-D* or RHD*Ø
   Predicted RHD phenotype: D-
   Serotype: D-
- *RHCE* haplotype 1: *RHCE*c*
   *RHCE* haplotype 2: *RHCE*c*
   Predicted RHCE C phenotype: C^{+W}
   Serotype: C⁺

The method described herein predicted a *RHD*DIIIa-CE(4-7)-D* phenotype; however, the serotype analysis incorrectly reported a C⁺ antigen phenotype, due to the inability to distinguish C⁺ from C^{+W}.

### Group 4

### Number of Samples: 32

Sample IDs: 09-0275, 09-0300, 10-0041, 10-0074, 10-0107, 10-0425, 10-0481, 10-0523, 10-0579, 10-0590, 10-0628, 10-0642, 10-0669, 10-0717, 10-0770, 10-0842, 10-0942, 10-1233, 10-1413, 10-1458, 10-1468, 10-1574, 10-1658, 10-1683, 10-2215, 10-2391, 10-2433, 10-2435, 10-2456, 10-3546, 10-3574, 10-4080

Genotyping data: RHCE*C intron 2 absent, RHD/CE Hex03 present, *RHD* exon 3 present, *RHD* exon 4 602C/G (heterozygous), *RHD* exon 7 1048G.
- *RHD* haplotype 1: *RHD*DIIIa*
   *RHD* haplotype 2: Possibly *RHD*D*
   Predicted RHD phenotype: D?
   Serotype: D⁺
- *RHCE* haplotype 1: *RHCE*c*
   *RHCE* haplotype 2: *RHCE*c*
   Predicted RHCE C phenotype: C⁻
   Serotype: C-

The method predicted that the phenotype was not due to a *RHD*DIIIa-CE(4-7)-D* haplotype. Serotype data agreed with these results.

### Group 5

### Number of Samples: 8

Sample IDs: 10-0420, 10-0512, 10-0543, 10-0735, 10-1634, 10-2379, 10-2470, 10-3409

Genotyping data: *RHCE*C* intron 2 present, RHD/CE Hex03 present, *RHD* exon 3 present, *RHD* exon 4 602C/G (heterozygous), *RHD* exon 7 1048G.
- *RHD* haplotype 1: *RHD*DIIIa*
   *RHD* haplotype 2: Possibly *RHD*D*
   Predicted RHD phenotype: D?
   Serotype: D⁺
- *RHCE* haplotype 1: *RHCE*c*
   *RHCE* haplotype 2: *RHCE*C*
   Predicted RHCE C phenotype: C⁺
   Serotype: C⁺

The method correctly predicted that the clinical phenotype was not *RHD*DIIIa-CE(4-7)-D, RHD*DIIIa* or *RHD*DIVa-2.*

### Group 6

### Number of Samples: 10

Sample ID: 09-0032, 10-0187, 10-0281, 10-1379, 10-1621, 10-1628, 10-2142, 10-2506, 10-3051, 10-3097

Genotyping data: RHCE*C intron 2 absent, RHD/CE Hex03 present, *RHD* exon 3 absent, *RHD* exon 4 602G, *RHD* exon 7 1048G.
- *RHD* haplotype 1: *RHD*DIIIa*
   RHD haplotype 2: *RHD*DIIIa* or *RHD*DIIIa-CE(4-7)-D* or *RHD*Ø* ⁽¹⁾
   Predicted RHD phenotype: Partial D
- *RHCE* haplotype 1: *RHCE*c*
   *RHCE* haplotype 2: *RHCE*c*
   Predicted RHCE C phenotype: C⁻ or C^{+W}

No serotype data available for these samples.

### Group 7

### Number of Samples: 4

### Sample IDs: 09-0287, 09-0333, 10-0075, 10-0284

Genotyping data: *RHCE*C* intron 2 absent, RHD/CE Hex03 present, RHD exon 3 present, *RHD* exon 4 602G, *RHD* exon 71048G.
- *RHD* haplotype 1: *RHD*DIIIa* or *RHD*DIIIa-CE(4-7)-D*
   *RHD* haplotype 2: *RHD*weakD*
   Predicted RHD phenotype: Weak D orPartial D
   Serotype: D⁺
- *RHCE* haplotype 1: *RHCE*c*
   *RHCE* haplotype 2: *RHCE*c*
   Predicted RHCE C phenotype: C⁻ or C^{+W}
   Serotype: Data not available

The method correctly predicted that the clinical phenotype was not *RHD*DIIIa-CE(4-7)-D.*

### Group 8

### Number of Samples: 5

Sample IDs: 10-0052, 10-0638, 10-0723, 10-0876, 10-2144

Genotyping data: *RHCE*C* intron 2 absent, RHD/CE Hex03 present, *RHD* exon 3 present, *RHD* exon 4 602C, *RHD* exon 7 1048G/C (heterozygous).
- *RHD* haplotype 1: *RHD*DIVa-2*
   *RHD* haplotype 2: Possibly *RHD*D*
   Predicted RHD phenotype: D?
- *RHCE* haplotype 1: *RHCE*c*
   *RHCE* haplotype 2: *RHCE*c*
   Predicted RHCE C phenotype: C-

No serotype data available for these samples.

### Group 9

### Number of Samples: 3

Sample IDs: 10-0081, 10-0387, 10-0400

Genotyping data: *RHCE*C* intron 2 present, RHD/CE Hex03 present, RHD exon 3 present, *RHD* exon 4 602C, *RHD* exon 7 1048G/C.
- *RHD* haplotype 1: *RHD*DIVa-2*
   RHD haplotype 2: Possibly *RHD*D*
   Predicted RHD phenotype: D?
   Serotype: D⁺
- *RHCE* haplotype 1: *RHCE*c*
   *RHCE* haplotype 2: *RHCE*C*
   Predicted RHCE C phenotype: C⁺
   Serotype: C⁺

The method correctly predicted that the clinical phenotype was not *RHD*DIIIa-CE(4-7)-D, RHD*DIIIa* or *RHD*DIVa-2.*

*⁽¹⁾RHD*Ø:* No RHD gene.

The above results are summarized in Table 3.

**Table 3**

| | | No. Haplotypes | % Haplotypes |
|---|---|---|---|
| Hybrid Exon 3 Variant | *RHD-DIIIa-CE(4-7)-D* | 84 | 28.8 |
| | *RHD*DIIIa* | 50 | 17.1 |
| | *RHD*DIVa-2* | 8 | 2.7 |
| | Uncertain | 38 | 13.0 |
| Other | | 112 | 38.4 |
| Total | | 292 | 100.0 |
| | | | |

| | | No. Samples | % Samples |
|---|---|---|---|
| *RHD*DIIIa-CE(4-7)-D* Call | Present | 84 | 57.5 |
| | Absent | 48 | 32.9 |
| | Uncertain | 14 | 9.6 |
| | Total | 146 | 100.0 |
| | | | |

| | | No. Samples | % Samples |
|---|---|---|---|
| Predicted RhD Phenotype | D? | 108 | 74.0 |
| | Partial D | 10 | 6.9 |
| | D⁻ | 24 | 16.4 |
| | Uncertain | 4 | 2.7 |
| | Total | 146 | 100.0 |
| | | | |

| | | No. Samples | % Samples |
|---|---|---|---|
| Predicted RhC Phenotype | C⁺ | 22 | 15.1 |
| | C^{+W} | 73 | 50.0 |
| | C⁻ | 37 | 25.3 |
| | Uncertain | 14 | 9.6 |
| | Total | 146 | 100.0 |

These data show that using the markers described herein, it is possible to distinguish *RHD*DIIIa-CE(4-7)-D* from *RHD*DIIIa* or *RHD*DIVa-2.* Further, these data show that relying on serotype analysis can erroneously lead to the incorrect diagnosis of a C^{+W} antigen phenotype as C⁺.

### References

1. DIIIa and DIII Type 5 are encoded by the same allele and are associated with altered RHCE*ce alleles: clinical implications. Connie M. Westhoff, Sunitha Vege, Christine Halter-Hipsky, Trina Whorley, Kim Hue-Roye, Christine Lomas-Francis, and Marion E. Reid. Transfusion (2010) 50, pp. 1303-1311.
2. Heterogeneous molecular background of the weak C, VS+, hrB-, HrB- phenotype in black persons. Bach-Nga Pham, Thierry Peyrard, Genevieve Juszczak, Isabelle Dubeaux, Dominique Gien, Antoine Blancher, Jean-Pierre Cartron, Philippe Rouger, and Pierre-Yves Le Pennec. Transfusion (2009) 49, pp. 495-504.
3. RHC and RHc genotyping in different ethnic groups. Martine G.H.M. Tax, C. Ellen van der Schoot, Rene' van Doorn, Lotte Douglas-Berger, Dick J. van Rhenen, and Petra A. Maaskant-van Wijk. Transfusion (2002) 42, pp. 6234-644.
4. The Blood group antigen FactsBook, Second edition. 2004. M. E. Reid and C. Lomas-Francis. Elsevier Ltd.

## Claims

1. A method of discriminating the *RHD*DIIIa-CE(4-7)-D or RHD*DIIIa-CE(4-7)-*D-like blood type variants, which express the C^{+W} antigen and lack a D antigen, from *RHD*DIIIa, RHD*DIVa-2* and other blood type variants, of a subject, the method comprising:
determining at least 4 markers in a sample that has been obtained from the subject,
wherein the markers comprise:
(i) the presence or absence of an RHCE*C allele;
(ii) the presence or absence of an RHD/RHCE hybrid exon 3 (RHD/CE Hex03) allele;
(iii) the absence of, or a single nucleotide polymorphism (SNP) variant within, any one of RHD exon 4, *RHD* exon 5, or *RHD* exon 6; and
(iv) the absence of, or SNP variant within, RHD exon 7,
wherein:
absence of said RHCE*C allele; presence of said RHD/RHCE hybrid exon 3 allele;
absence of *RHD* exon 4, *RHD* exon 5 or *RHD* exon 6; and absence of *RHD* exon 7 in combination indicate that the sample contains said *RHD*DIIIa-CE(4-7)-D or said RHD*DIIIa-CE(4-7)-D-*like blood type variant.

2. A method according to claim 1, wherein:
a) the SNP variant within *RHD* exon 4 is at position 602 of the *RHD* coding sequence (rs1053355),
b) the SNP variant within *RHD* exon 5 is at position 667 of the *RHD* coding sequence (rs1053356),
c) the SNP variant within *RHD* exon 6 is at position 819 of the *RHD* coding sequence; and/or
d) the SNP variant within *RHD* exon 7 is at position 1048 of the *RHD* coding sequence (rs41307826),
wherein the *RHD* coding sequence is as set forth in SEQ ID NO: 1.

3. A method according to claim 1 or 2, wherein the markers further comprise:
(v) the presence or absence of an RHD exon 3 allele,
wherein the absence of said *RHD* exon 3 further indicates that the sample contains said *RHD*DIIIa-CE(4-7)-D or said RHD*DIIIa-CE(4-7-D-*like blood type variant.

4. A method according to any one of the preceding claims, wherein:
a) the method further comprises determining the RHD and RHC antigen phenotypes of the subject in accordance with Table 1; and/or
b) the method comprises detecting the presence or absence of a blood type variant selected from: *RHD*DIIIa; RHD*DIVa-2;* or *RHD*DIIIa-CE(4-7)-D* or *RHD*DIIIa-CE(4-7)-D)-*like blood type variants, e.g. wherein the method comprises detecting the presence or absence of *RHD*DIIIa-CE(4-7)-D* or *RHD*DIIIa-CE(4-7)-*D)-like blood type variants; and/or
c) marker (iii) is the SNP within RHD exon 4 at position 602 of the RHD coding sequence (rs1053355); and/or
d) the RHCE*C allele is determined by determining the presence or absence of RHCE*C intron 2, or any one of the following positions in the RHCE coding sequence: position 307 (exon 2), position 48 (exon 1), position 150 (exon 2), position 178 (exon 2), position 201 (exon 2) and/or position 203 (exon 2),
wherein the *RHD* coding sequence is as set forth in SEQ ID NO: 1 and wherein the *RCHE* coding sequence is as set forth in SEQ ID NO: 2.

5. A method according to any one of the preceding claims, wherein the sample comprises nucleic acid and the method comprises amplifying the nucleic acid or a portion thereof by PCR using primers, e.g. wherein:
a) the PCR primers for determining the RHCE*C allele are a forward PCR primer specific for RHCE*C, and a non-specific reverse PCR primer, e.g. wherein
(i) the non-specific reverse primer is shared with RHD, RHC*C and/or RHC*c; and/or
(ii) the PCR primers comprise:
Forward: 5'-GGCCACCACCATTTGAA-3' (SEQ ID NO: 3)
Reverse: 5'-CCATGAACATGCCACTTCAC-3', (SEQ ID NO: 4); and/or
b) the PCR primers for determining the RHD/CE Hex03 allele are forward and reverse PCR primers targeting sequences located in introns 2 and 3, or introns 3 and 2, respectively, e.g. wherein
(i) the PCR primers comprise:
Forward primer: 5'-TCCTGGCTCTCCCTCTCT-3' (SEQ ID NO: 9)
Reverse primer: 5'-TTTTCAAAACCCCGGAAG-3 (SEQ ID NO: 10);
and/or
c) the PCR primers for determining the SNP within RHD exon 4 at position 602 of the *RHD* coding sequence (rs1053355) are forward and reverse primers targeting sequences located in introns 3 and 4, or introns 4 and 3, respectively, e.g. wherein
(i) the PCR primers comprise:
Forward primer: 5'-GCTCTGAACTTTCTCCAAGGACT-3' (SEQ ID NO: 17)
Reverse primer: 5'-ATTCTGCTCAGCCCAAGTAG-3' (SEQ ID NO: 18); and/or
d) the PCR primers for determining the SNP within RHD exon 5 at position 667 of the *RHD* coding sequence (rs1053356) are forward and reverse primers targeting sequences located in introns 4 and 5, or introns 5 and 4, respectively, e.g. wherein
(i) the PCR primers comprise:
Forward primer: 5'-TTGAATTAAGCACTTCACAGAGCA-3' (SEQ ID NO: 19)
Reverse primer: 5'-CACCTTGCTGATCTTCCC-3' (SEQ ID NO: 20); and/or
e) the PCR primers for determining the SNP within RHD exon 6 at position 819 of the *RHD* coding sequence are forward and reverse primers targeting sequences located in introns 5 and 6, or introns 6 and 5, respectively, e.g. wherein
(i) the PCR primers comprise:
Forward primer: 5'-AGTAGTGAGCTGGCCCATCA-3' (SEQ ID NO: 21)
Reverse primer: 5'-CTTCAGCCAAAGCAGAGGAG-3' (SEQ ID NO: 22); and/or
f) the PCR primers for determining the SNP within RHD exon 7 at position 1048 of the *RHD* coding sequence (rs41307826) are forward and reverse primers targeting sequences located in introns 6 and 7, or introns 7 and 6, respectively, e.g. wherein
(i) the PCR primers comprise:
Forward primer: 5'-ACAAACTCCCCGATGATGTGAGTG-3' (SEQ ID NO: 35)
Reverse primer: 5'-GAGGCTGAGAAAGGTTAAGCCA-3' (SEQ ID NO: 36); and/or
g) as dependent from claim 3, the PCR primers for determining the RHD exon 3 allele are forward and reverse primers targeting sequences located in introns 2 and 3, or introns 3 and 2, respectively, e.g. wherein
(i) the PCR primers comprise:
Forward primer: 5'-TCCTGGCTCTCCCTCTCT-3' (SEQ ID NO: 15)
Reverse primer: 5'-GTTGTCTTTATTTTTCAAAACCCT-3' (SEQ ID NO:16);
wherein the RHD coding sequence is as set forth in SEQ ID NO: 1.

6. A method according to claim 5, wherein the amplified nucleic acid comprises a label, e.g. wherein
a) the label comprises a biotinylated nucleotide; and/or
b) the label comprises a fluorescent moiety.

7. A method according to any one of the preceding claims, wherein the sample comprises nucleic acid, and the method comprises amplifying the nucleic acid or a portion thereof by PCR using primers, fragmenting the amplified nucleic acid, and labelling the fragmented nucleic acid with biotinylated ddNTPS using a terminal deoxynucleotidyl transferase (TdT) enzyme.

8. A method according to any one of the preceding claims, wherein determining the presence, absence or SNP variant of a marker comprises contacting nucleic acid containing each marker with one or more probes, e.g. wherein:
a) as dependent from claim 3, the probes for determining the presence or absence of RHD/CE Hex03 or RHD exon 3 contact an SNP located in both RHD/CE Hex03 and RHD exon 3, wherein one SNP variant is specific for RHD/CE Hex03, and another SNP variant is specific for RHD exon 3, e.g. wherein
(i) the SNP is at position 410 of the coding sequence, located within both RHD/CE Hex03 and RHD exon 3; and/or
(ii) the probes comprise:
(1) 5'-TTTTACAGACGCCTGCTACCATG-3', (SEQ ID NO: 5)
(2) 5'-CATGGTAGCAGGCGTCTGTAAAA-3', (SEQ ID NO: 6)
(3) 5'-TTTTACAGACGTCTGCTACCATG-3', (SEQ ID NO: 7) and
(4) 5'-CATGGTAGCAGACGTCTGTAAAA-3', (SEQ ID NO: 8); and/or
b) the probes for determining the absence or SNP variant of the SNP at: position 602 of the *RHD* coding sequence located within exon 4 (rs1053355), position 667 of the *RHD* coding sequence located within exon 5 (rs1053356), or position 819 of the *RHD* coding sequence located within exon 6 comprise:
(i) RHD exon 4:
(1) 5'-ATAAAGATCAGACAGCAACGATACC-3' (SEQ ID NO: 23)
(2) 5'-TAAAGATCAGACAGCAACGATAC-3' (SEQ ID NO: 24)
(3) 5'-ATAAAGATCAGAGAGCAACGATACC-3' (SEQ ID NO: 25)
(4) 5'-TAAAGATCAGAGAGCAACGATAC-3' (SEQ ID NO: 26);
(ii) RHD exon 5:
(1) 5'-CTGGCCAAGTTTCAACTCTGC-3' (SEQ ID NO: 27)
(2) 5'-TGGCCAAGTTTCAACTCTG-3' (SEQ ID NO: 28)
(3) 5'-CTGGCCAAGTGTCAACTCTGC-3' (SEQ ID NO: 29)
(4) 5'-TGGCCAAGTGTCAACTCTG-3' (SEQ ID NO: 30);
(iii) RHD exon 6:
(1) 5'-GTGCACAGTGCGGTGTTGGCAGG-3' (SEQ ID NO: 31)
(2) 5'- TGCACAGTGCGGTGTTGGCAG -3' (SEQ ID NO: 32)
(3) 5'- GTGCACAGTGCAGTGTTGGCAGG -3' (SEQ ID NO: 33)
(4) 5'-TGCACAGTGCAGTGTTGGCAG-3' (SEQ ID NO: 34); and/or
c) the probes for determining the SNP variant of the SNP at position 1048 of the *RHD* coding sequence located within exon 7 (rs41307826)comprise:
(1) 5'-TGCTGGTGCTTGATACCGTCGGA-3' (SEQ ID NO: 37)
(2) 5'-GCTGGTGCTTGATACCGTCGG-3' (SEQ ID NO: 38)
(3) 5'-TGCTGGTGCTTCATACCGTCGGA-3' (SEQ ID NO: 39)
(4) 5'-GCTGGTGCT'fiCATACCGTCGG-3' (SEQ ID NO: 40);
wherein the *RHD* coding sequence is as set forth in SEQ ID NO:1.

9. A method according to claim 8, wherein
a) one or more of the probes comprise a label, e.g. wherein the label is a fluorescent moiety; and/or
b) one or more of the probes is attached to a solid support or conjugated to one or more particles.

10. Use of a set of primers in a method for discriminating the *RHD*DIIIa-CE(4-7)-D or RHD*DIIIa-CE(4-7_-D)-*like blood type variants, which express the C^{+W} antigen and lack a D antigen, from *RHD*DIIIa, RHD*DIVa-2* and other blood type variants, by amplifying nucleic acid comprising at least the four markers of claim 1, wherein the set of primers comprises at least three primer pairs selected from the primers set forth in:
(i) claim 5(a),
(ii) claim 5(b),
(iii) any one of claims 5(c), 5(d) or 5(e)
(iv) claim 5(f), and
(v) claim 5(g).

11. Use according to claim 10, wherein the set of primers comprises at least three primer pairs selected from the primers set forth in:
(i) claim 5(a)(ii),
(ii) claim 5(b)(i),
(iii) any one of claims 5(c)(i), 5(d)(i) or 5(e)(i),
(iv) claim 5(f)(i), and
(v) claim 5(g)(i).

12. Use according to claim 10 or claim 11, wherein at least 50% of the primers in the set are the primer pairs defined in claim 10 or 11.

13. Use of a set of probes in a method for discriminating the *RHD*DIIIa-CE(4-7)-D or RHD*DIIIa-CE(4-7_-D)-*like blood type variants, which express the C^{+W} antigen and lack a D antigen, from *RHD*DIIIa*, *RHD*DIVa-2* and other blood type variants, by determining the presence, absence or single nucleotide polymorphism (SNP) variant of at least the four markers of claim 1, wherein the set of probes comprises:
(i) the probes of SEQ ID NOs: 5, 6, 7 and 8;
(ii) the probes of SEQ ID NOs: 23, 24, 25 and 26;
(iii) the probes of SEQ ID NOs: 31, 32, 33 and 34
(iv) the probes of SEQ ID NOs: 27, 28, 29 and 30; and
(v) the probes of SEQ ID NOs: 37, 38, 39 and 40.

14. Use according to claim 13, wherein:
a) the probes are immobilised on a solid support or conjugated to one or more particles, e.g. wherein the solid support comprises one or more attached labels, e.g. wherein the label is a fluorochrome; and/or
b) one or more probes comprise a label, e.g wherein the label is a fluorescent moiety.

15. Use of a kit for genotyping a subject, the kit comprising a set of PCR primers as defined in any one of claims 10 to 12, and a set of probes as defined in any one of claims 13 to 14.

## Patentansprüche

1. Verfahren zur Unterscheidung von RHD*DIIIa-CE(4-7)-D- oder RHD*DIIIa-CE(4-7)-D-artigen Bluttypvarianten, die das C^{+W}-Antigen exprimieren und denen ein D-Antigen fehlt, von RHD*DIIIa-, RHD*DIVa-2- und sonstigen Bluttypvarianten eines Individuums, wobei das Verfahren Folgendes umfasst:
das Bestimmen von zumindest 4 Markern in einer Probe, die von dem Individuum gewonnen wurden, worin die Marker Folgendes umfassen:
(i) die Gegenwart oder Abwesenheit eines RHCE*C-Allels;
(ii) die Gegenwart oder Abwesenheit eines RHD/RHCE-Hybridexon-3-(RHD/CE-Hex03-) Allels;
(iii) die Abwesenheit einer oder eine Einzelnucleotidpolymorphismus-(SNP-) Variante innerhalb eines von RHD-Exon 4, RHD-Exon 5 und RHD-Exon 6; und
(iv) die Abwesenheit einer oder eine SNP-Variante innerhalb von RHD-Exon 7,
worin die Abwesenheit des RHCE*C-Allels; die Gegenwart des RHD/RHCE-Hybridexon-3-Allels; die Abwesenheit von RHD-Exon 4, RHD-Exon 5 oder RHD-Exon 6; und die Abwesenheit von RHD-Exon 7 in Kombination ein Indikator dafür sind, dass die Probe die RHD*DIIIa-CE(4-7)-D- oder die RHD*DIIIa-CE(4-7)-D-artige Bluttypvariante enthält.

2. Verfahren nach Anspruch 1, worin:
a) die SNP-Variante sich innerhalb von RHD-Exon 4 an Position 602 der für RHD kodierenden Sequenz befindet (rs1053355),
b) die SNP-Variante sich innerhalb von RHD-Exon 5 an Position 667 der für RHD kodierenden Sequenz befindet (rs1053356),
c) die SNP-Variante sich innerhalb von RHD-Exon 6 an Position 819 der für RHD kodierenden Sequenz befindet; und/oder
d) die SNP-Variante sich innerhalb von RHD-Exon 7 an Position 1048 der für RHD kodierenden Sequenz befindet (rs41307826),
worin die für RHD kodierende Sequenz in Seq.-ID Nr. 1 dargelegt ist.

3. Verfahren nach Anspruch 1 oder 2, worin die Marker weiters Folgendes umfassen:
(v) die Gegenwart oder Abwesenheit eines RHD-Exon-3-Allels,
worin die Abwesenheit von RHD-Exon 3 ein weiterer Indikator dafür ist, dass die Probe die RHD*DIIIa-CE(4-7)-D- oder die RHD*DIIIa-CE(4-7)-D-artige Bluttypvariante enthält.

4. Verfahren nach einem der vorangegangenen Ansprüche, worin:
a) das Verfahren weiters das Bestimmen der RHD- und RHC-Antigenphänotypen des Individuums in Übereinstimmung mit Tabelle 1 umfasst; und/oder
b) das Verfahren das Nachweisen der Gegenwart oder Abwesenheit einer aus RHD*DIIIa-; RHD*DIVa-2-, oder RHD*DIIIa-CE(4-7)-D- oder RHD*DIIIa-CE(4-7)-D-artigen Bluttypvarianten ausgewählten Bluttypvariante umfasst, z.B. worin das Verfahren das Nachweisen der Gegenwart oder Abwesenheit von RHD*DIIIa-CE(4-7)-D-oder RHD*DIIIa-CE(4-7)-D-artigen Bluttypvarianten umfasst; und/oder
c) Marker (iii) der SNP innerhalb von RHD-Exon 4 an Position 602 der für RHD kodierenden Sequenz ist (rs1053355); und/oder
d) das RHCE*C-Allel durch Bestimmen der Gegenwart oder Abwesenheit von RHCE*C-Intron 2 oder einer der folgenden Positionen in der für RHCE kodierenden Sequenz bestimmt wird: Position 307 (Exon 2), Position 48 (Exon 1), Position 150 (Exon 2), Position 178 (Exon 2), Position 201 (Exon 2) und/oder Position 203 (Exon 2), worin die für RHD kodierende Sequenz in Seq.-ID Nr. 1 dargelegt ist und worin die für RHCE kodierende Sequenz in Seq.-ID Nr. 2 dargelegt ist.

5. Verfahren nach einem der vorangegangenen Ansprüche, worin die Probe Nucleinsäure umfasst und das Verfahren das Amplifizieren der Nucleinsäure oder eines Teils davon mittels PCR unter Verwendung von Primern umfasst, worin z.B.:
a) die PCR-Primer zur Bestimmung des RHCE*C-Allels ein für RHCE*C spezifischer PCR-Vorwärtsprimer und ein unspezifischer PCR-Rückwärtsprimer sind, worin z.B.
(i) der unspezifische Rückwärtsprimer mit RHD, RHC*C und/oder RHC*c geteilt wird; und/oder
(ii) die PCR-Primer die folgenden umfassen:
Vorwärts: 5'-GGCCACCACCATTTGAA-3' (Seq.-ID Nr. 3)
Rückwärts: 5'-CCATGAACATGCCACTTCAC-3' (Seq.-ID Nr. 4); und/oder
b) die PCR-Primer zur Bestimmung des RHD/CE-Hex03-Allels PCR-Vorwärts-und -Rückwärtsprimer sind, die auf Sequenzen gerichtet sind, die in den Introns 2 und 3 bzw. in den Introns 3 und 2 liegen, worin z.B.:
(i) die PCR-Primer die folgenden umfassen:
Vorwärtsprimer: 5'-TCCTGGCTCTCCCTCTCT-3' (Seq.-ID Nr. 9)
Rückwärtsprimer: 5'-TTTTCAAAACCCCGGAAG-3' (Seq.-ID Nr. 10);
und/oder
c) die PCR-Primer zur Bestimmung des SNP innerhalb von RHD-Exon 4 an Position 602 der für RHD kodierenden Sequenz (rs1053355) Vorwärts- und Rück-wärtsprimer sind, die auf die in den Introns 3 und 4 bzw. in den Introns 4 und 3 liegenden Sequenzen gerichtet sind, worin z.B.:
(i) die PCR-Primer die folgenden umfassen:
Vorwärtsprimer: 5'-GCTCTGAACTTTCTCCAAGGACT-3' (Seq.-ID Nr. 17)
Rückwärtsprimer: 5'-ATTCTGCTCAGCCCAAGTAG-3' (Seq.-ID Nr. 18);
und/oder
d) die PCR-Primer zur Bestimmung des SNP innerhalb von RHD-Exon 5 an Position 667 der für RHD kodierenden Sequenz (rs1053356) Vorwärts- und Rück-wärtsprimer sind, die auf die in den Introns 4 und 5 bzw. in den Introns 5 und 4 liegenden Sequenzen gerichtet sind, worin z.B.:
(i) die PCR-Primer die folgenden umfassen:
Vorwärtsprimer: 5'-TTGAATTAAGCACTTCACAGAGCA-3' (Seq.-ID Nr. 19)
Rückwärtsprimer: 5'-CACCTTGCTGATCTTCCC-3' (Seq.-ID Nr. 20);
und/oder
e) die PCR-Primer zur Bestimmung des SNP innerhalb von RHD-Exon 6 an Position 819 der für RHD kodierenden Sequenz Vorwärts- und Rückwärtsprimer sind, die auf die in den Introns 5 und 6 bzw. in den Introns 6 und 5 liegenden Sequenzen gerichtet sind, worin z.B.:
(i) die PCR-Primer die folgenden umfassen:
Vorwärtsprimer: 5'-AGTAGTGAGCTGGCCCATCA-3' (Seq.-ID Nr. 21)
Rückwärtsprimer: 5'-CTTCAGCCAAAGCAGAGGAG-3' (Seq.-ID Nr. 22);
und/oder
f) die PCR-Primer zur Bestimmung des SNP innerhalb von RHD-Exon 7 an Position 1048 der für RHD kodierenden Sequenz (rs41307826) Vorwärts- und Rück-wärtsprimer sind, die auf die in den Introns 6 und 7 bzw. in den Introns 7 und 6 liegenden Sequenzen gerichtet sind, worin z.B.:
(i) die PCR-Primer die folgenden umfassen:
Vorwärtsprimer: 5'-ACAAACTCCCCGATGATGTGAGTG-3' (Seq.-ID Nr. 35)
Rückwärtsprimer: 5'-GAGGCTGAGAAAGGTTAAGCCA-3' (Seq.-ID Nr. 36);
und/oder
g) in Abhängigkeit von Anspruch 3 die PCR-Primer zur Bestimmung des RHD-Exon-3-Allels Vorwärts- und Rückwärtsprimer sind, die auf die in den Introns 2 und 3 bzw. in den Introns 3 und 2 liegenden Sequenzen gerichtet sind, worin z.B.:
(i) die PCR-Primer die folgenden umfassen:
Vorwärtsprimer: 5'-TCCTGGCTCTCCCTCTCT-3' (Seq.-ID Nr. 15)
Rückwärtsprimer: 5'-GTTGTCTTTATTTTTCAAAACCCT-3' (Seq.-ID Nr. 16);
worin die für RHD kodierende Sequenz in Seq.-ID Nr. 1 dargelegt ist.

6. Verfahren nach Anspruch 5, worin die amplifizierte Nucleinsäure eine Markierung umfasst, worin z.B.:
a) die Markierung ein biotinyliertes Nucleotid umfasst; und/oder
b) die Markierung eine fluoreszierende Gruppierung umfasst.

7. Verfahren nach einem der vorangegangenen Ansprüche, worin die Probe Nucleinsäure umfasst und das Verfahren das Amplifizieren der Nucleinsäure oder eines Teils davon mittels PCR unter Verwendung von Primern, das Fragmentieren der amplifizierten Nucleinsäure und das Markieren der fragmentierten Nucleinsäure mit biotinyliertem ddNTPS unter Verwendung eines terminalen Desoxynucleotidyltransferase- (TdT-) Enzyms umfasst.

8. Verfahren nach einem der vorangegangenen Ansprüche, worin das Bestimmen der Gegenwart, Abwesenheit oder SNP-Variante eines Markers das Kontaktieren von Nucleinsäure, die den jeweiligen Marker enthält, mit einer oder mehreren Sonden umfasst, worin z.B.:
a) in Abhängigkeit von Anspruch 3 die Sonden zur Bestimmung der Gegenwart oder Abwesenheit von RHD/CE-Hex03 oder RHD-Exon 3 einen SNP kontaktieren, der sowohl in RHD/CE-Hex03 als auch in RHD-Exon 3 liegt, worin eine SNP-Variante für RHD/CE-Hex03 spezifisch ist und eine andere SNP-Variante für RHD-Exon 3 spezifisch ist, worin z.B.:
(i) der SNP sich an Position 410 der kodierenden Sequenz sowohl innerhalb von RHD/CE-Hex03 als auch innerhalb von RHD-Exon 3 befindet; und/oder
(ii) die Sonden die folgenden umfassen:
(1) 5'-TTTTACAGACGCCTGCTACCATG-3' (Seq.-ID Nr. 5);
(2) 5'-CATGGTAGCAGGCGTCTGTAAAA-3' (Seq.-ID Nr. 6);
(3) 5'- TTTTACAGACGTCTGCTACCATG-3' (Seq.-ID Nr. 7); und
(4) 5'-CATGGTAGCAGACGTCTGTAAAA-3' (Seq.-ID Nr. 8);
und/oder
b) die Sonden zur Bestimmung der Abwesenheit oder SNP-Variante des SNP an Position 602 der für RHD kodierenden Sequenz innerhalb von Exon 4 (rs1053355), an Position 667 der für RHD kodierenden Sequenz innerhalb von Exon 5 (rs1053356) oder an Position 819 der für RHD kodierenden Sequenz innerhalb von Exon 6 jeweils die folgenden umfassen:
(i) RHD-Exon 4:
(1) 5'-ATAAAGATCAGACAGCAACGATACC-3' (Seq.-ID Nr. 23)
(2) 5'-TAAAGATCAGACAGCAACGATAC-3' (Seq.-ID Nr. 24)
(3) 5'-ATAAAGATCAGAGAGCAACGATACC-3' (Seq.-ID Nr. 25)
(4) 5'-TAAAGATCAGAGAGCAACGATAC-3' (Seq.-ID Nr. 26);
(ii) RHD-Exon 5:
(1) 5'-CTGGCCAAGTTTCAACTCTGC-3' (Seq.-ID Nr. 27)
(2) 5'-TGGCCAAGTTTCAACTCTG-3' (Seq.-ID Nr. 28)
(3) 5'-CTGGCCAAGTGTCAACTCTGC-3' (Seq.-ID Nr. 29)
(4) 5'-TGGCCAAGTGTCAACTCTG-3' (Seq.-ID Nr. 30);
(iii) RHD-Exon 6:
(1) 5'-GTGCACAGTGCGGTGTTGGCAGG-3' (Seq.-ID Nr. 31)
(2) 5'-TGCACAGTGCGGTGTTGGCAG-3' (Seq.-ID Nr. 32)
(3) 5'-GTGCACAGTGCAGTGTTGGCAGG-3' (Seq.-ID Nr. 33)
(4) 5'-TGCACAGTGCAGTGTTGGCAG-3' (Seq.-ID Nr. 34);
und/oder
c) die Sonden zur Bestimmung der SNP-Variante des SNP an Position 1048 der für RHD kodierenden Sequenz innerhalb von Exon 7 (rs41307826) die folgenden umfassen:
(1) 5'-TGCTGGTGCTTGATACCGTCGGA-3' (Seq.-ID Nr. 37)
(2) 5'-GCTGGTGCTTGATACCGTCGG-3' (Seq.-ID Nr. 38)
(3) 5'-TGCTGGTGCTTCATACCGTCGGA-3' (Seq.-ID Nr. 39)
(4) 5'-GCTGGTGCTTCATACCGTCGG-3' (Seq.-ID Nr. 40);
worin die für RHD kodierende Sequenz in Seq.-ID Nr. 1 dargelegt ist.

9. Verfahren nach Anspruch 8, worin
a) eine oder mehrere der Sonden eine Markierung umfassen, z.B. worin die Markierung eine fluoreszierende Gruppierung ist; und/oder
b) eine oder mehrere der Sonden an einen festen Träger gebunden oder an ein oder mehrere Partikel konjugiert sind.

10. Verwendung eines Primersatzes in einem Verfahren zur Unterscheidung von RHD*DIIIa-CE(4-7)-D- oder RHD*DIIIa-CE(4-7)-D-artigen Bluttypvarianten, die das C^{+W}-Antigen exprimieren und denen ein D-Antigen fehlt, von RHD*DIIIa-, RHD*DIVa-2- und sonstigen Bluttypvarianten durch das Amplifizieren von Nucleinsäure, die zumindest die vier Marker aus Anspruch 1 umfasst, worin der Primersatz zumindest drei aus den in:
(i) Anspruch 5(a),
(ii) Anspruch 5(b),
(iii) einem der Ansprüche 5(c), 5(d) oder 5(e),
(iv) Anspruch 5(f) und
(v) Anspruch 5(g)
dargelegten Primersätzen ausgewählte Primerpaare umfasst.

11. Verwendung nach Anspruch 10, worin der Primersatz zumindest drei aus den in:
(i) Anspruch 5(a)(ii),
(ii) Anspruch 5(b)(i),
(iii) einem der Ansprüche 5(c)(i), 5(d)(i) oder 5(e)(i),
(iv) Anspruch 5(f)(i) und
(v) Anspruch 5(g)(i)
dargelegten Primersätzen ausgewählte Primerpaare umfasst.

12. Verwendung nach Anspruch 10 oder Anspruch 11, worin zumindest 50 % der Primer in dem Satz die in Anspruch 10 oder Anspruch 11 definierten Primerpaare sind.

13. Verwendung eines Satzes an Sonden in einem Verfahren zur Unterscheidung von RHD*DIIIa-CE(4-7)-D- oder RHD*DIIIa-CE(4-7)-D-artigen Bluttypvarianten, die das C^{+W}-Antigen exprimieren und denen ein D-Antigen fehlt, von RHD*DIIIa-, RHD*DIVa-2- und sonstigen Bluttypvarianten durch das Bestimmen der Gegenwart, Abwesenheit oder Einzelnucleotidpolymorphismus- (SNP-) Variante der zumindest vier Marker aus Anspruch 1, worin der Sondensatz Folgendes umfasst:
(i) die Sonden mit Seq.-ID Nr. 5, 6, 7, und 8;
(ii) die Sonden mit Seq.-ID Nr. 23, 24, 25 und 26;
(iii) die Sonden mit Seq.-ID Nr. 31, 32, 33 und 34;
(iv) die Sonden mit Seq.-ID Nr. 27, 28, 29 und 30; und
(v) die Sonden mit Seq.-ID Nr. 37, 38, 39 und 40.

14. Verwendung nach Anspruch 13, worin:
a) die Sonden auf einem festen Träger immobilisiert oder an ein oder mehrere Teilchen konjugiert sind, worin z.B. der feste Träger eine oder mehrere angehaftete Markierungen umfasst, z.B. worin die Markierung ein Fluorochrom ist; und/oder
b) eine oder mehrere Sonden eine Markierung umfassen, worin z.B. die Markierung eine fluoreszierende Gruppierung ist.

15. Verwendung eines Sets zur Genotypisierung eines Individuums, wobei das Set einen Satz an PCR-Primern wie in einem der Ansprüche 10 bis 12 definiert und einen Satz an Sonden wie in einem der Ansprüche 13 und 14 definiert umfasst.

## Revendications

1. Procédé pour différencier les variants de groupe sanguin *RHD*DIIIa-CE (4-7)-D* ou analogues à *RHD*DIIIa-CE(4-7)-D,* qui expriment l'antigène C^{+W} et sont dépourvus d'un antigène D, de *RHD*DIIIa, RHD*DIVa-2* et d'autres variants de groupe sanguin d'un sujet, le procédé consistant à :
déterminer au moins 4 marqueurs dans un échantillon qui a été obtenu à partir du sujet, où les marqueurs comprennent :
(i) la présence ou l'absence d'un allèle RHCE*C ;
(ii) la présence ou l'absence d'un allèle de l'exon 3 hybride RHD/RHCE (RHD/CE Hex03) ;
(iii) l'absence de, ou un variant de polymorphisme de nucléotide unique (SNP) au sein de, l'un quelconque de l'exon 4 de *RHD,* l'exon 5 de *RHD,* ou l'exon 6 de *RHD* ; et
(iv) l'absence de, ou un variant de SNP au sein de, l'exon 7 de *RHD,*
où :
l'absence dudit allèle RHCE*C ; la présence dudit allèle de l'exon 3 hybride RHD/RHCE ; l'absence de l'exon 4 de *RHD,* l'exon 5 de *RHD* ou l'exon 6 de *RHD* ; et l'absence de l'exon 7 de *RHD* en combinaison indique que l'échantillon contient ledit variant de groupe sanguin *RHD*DIIIa-CE(4-7)-D ou* ledit analogue à *RHD*DIIIa-CE (4-7)-D*.

2. Procédé selon la revendication 1, dans lequel :
a) le variant de SNP au sein de l'exon 4 de *RHD* est à la position 602 de la séquence codant pour *RHD* (rs1053355),
b) le variant de SNP au sein de l'exon 5 de *RHD* est à la position 667 de la séquence codant pour *RHD* (rs1053356),
c) le variant de SNP au sein de l'exon 6 de *RHD* est à la position 819 de la séquence codant pour *RHD* ; et/ou
d) le variant de SNP au sein de l'exon 7 de *RHD* est à la position 1048 de la séquence codant pour *RHD* (rs41307826), où la séquence codant pour *RHD* est telle que décrite dans SEQ ID NO: 1.

3. Procédé selon la revendication 1 ou 2, dans lequel les marqueurs comprennent en outre :
(v) la présence ou l'absence d'un allèle de l'exon 3 de *RHD,*
où l'absence dudit exon 3 de *RHD* indique en outre que l'échantillon contient ledit variant de groupe sanguin *RHD*DIIIa-CE (4-7)-D ou* ledit analogue à *RHD*DIIIa-CE (4-7) -* D.

4. Procédé selon l'une quelconque des revendications précédentes, dans lequel :
a) le procédé consiste en outre à déterminer les phénotypes antigéniques de RHD et RHC du sujet conformément au Tableau 1 ; et/ou
b) le procédé consiste à détecter la présence ou l'absence d'un variant de groupe sanguin sélectionné parmi : *RHD*DIIIa ; RHD*DIVa-2 ;* ou des variants de groupe sanguin *RHD*DIIIa-CE (4-7) -D ou* analogues à *RHD*DIIIa-CE (4-*7)-D, par exemple où le procédé consiste à détecter la présence ou l'absence de variants de groupe sanguin *RHD*DIIIa-CE (4-7) -D ou* analogues à *RHD*DIIIa-CE (4-7) -D* ; et/ou
c) le marqueur (iii) est le SNP au sein de l'exon 4 de RHD à la position 602 de la séquence codant pour RHD (rs1053355) ; et/ou
d) l'allèle RHCE*C est déterminé en déterminant la présence ou l'absence de l'intron 2 de RHCE*C, ou l'une quelconque des positions suivantes dans la séquence codant pour RHCE : la position 307 (exon 2), la position 48 (exon 1), la position 150 (exon 2), la position 178 (exon 2), la position 201 (exon 2) et/ou la position 203 (exon 2),
où la séquence codant pour *RHD* est telle que décrite dans SEQ ID NO: 1 et où la séquence codant pour *RHCE* est telle que décrite dans SEQ ID NO: 2.

5. Procédé selon l'une quelconque des revendications précédentes, dans lequel l'échantillon comprend un acide nucléique et le procédé consiste à amplifier l'acide nucléique ou une partie de celui-ci par PCR en utilisant des amorces, par exemple où :
a) les amorces de PCR pour déterminer l'allèle RHCE*C sont une amorce de PCR sens spécifique pour RHCE*C, et une amorce de PCR antisens non spécifique, par exemple où
(i) l'amorce antisens non spécifique est partagée avec RHD, RHC*C et/ou RHC*c ; et/ou
(ii) les amorces de PCR comprennent :
Sens : 5'-GGCCACCACCATTTGAA-3' (SEQ ID NO: 3)
Antisens : 5'-CCATGAACATGCCACTTCAC-3' (SEQ ID NO: 4) ; et/ou
b) les amorces de PCR pour déterminer l'allèle RHD/CE Hex03 sont des amorces de PCR sens et antisens ciblant des séquences localisées dans les introns 2 et 3, ou les introns 3 et 2, respectivement, par exemple où :
(i) les amorces de PCR comprennent :
Amorce sens : 5'-TCCTGGCTCTCCCTCTCT-3' (SEQ ID NO: 9)
Amorce antisens : 5'-TTTTCAAAACCCCGGAAG-3' (SEQ ID NO: 10) ;
et/ou
c) les amorces de PCR pour déterminer le SNP au sein de l'exon 4 de RHD à la position 602 de la séquence codant pour *RHD* (rs1053355) sont des amorces sens et antisens ciblant des séquences localisées dans les introns 3 et 4, ou les introns 4 et 3, respectivement, par exemple où :
(i) les amorces de PCR comprennent :
Amorce sens : 5'-GCTCTGAACTTTCTCCAAGGACT-3' (SEQ ID NO: 17)
Amorce antisens : 5'-ATTCTGCTCAGCCCAAGTAG-3' (SEQ ID NO: 18) ; et/ou
d) les amorces de PCR pour déterminer le SNP au sein de l'exon 5 de RHD à la position 667 de la séquence codant pour *RHD* (rs1053356) sont des amorces sens et antisens ciblant des séquences localisées dans les introns 4 et 5, ou les introns 5 et 4, respectivement, par exemple où :
(i) les amorces de PCR comprennent :
Amorce sens : 5'-TTGAATTAAGCACTTCACAGAGCA-3' (SEQ ID NO: 19)
Amorce antisens : 5'-CACCTTGCTGATCTTCCC-3' (SEQ ID NO: 20) ; et/ou
e) les amorces de PCR pour déterminer le SNP au sein de l'exon 6 de RHD à la position 819 de la séquence codant pour *RHD* sont des amorces sens et antisens ciblant des séquences localisées dans les introns 5 et 6, ou les introns 6 et 5, respectivement, par exemple où :
(i) les amorces de PCR comprennent :
Amorce sens : 5'-AGTAGTGAGCTGGCCCATCA-3' (SEQ ID NO: 21)
Amorce antisens : 5'-CTTCAGCCAAAGCAGAGGAG-3' (SEQ ID NO: 22) ; et/ou
f) les amorces de PCR pour déterminer le SNP au sein de l'exon 7 de RHD à la position 1048 de la séquence codant pour *RHD* (rs41307826) sont des amorces sens et antisens ciblant des séquences localisées dans les introns 6 et 7, ou les introns 7 et 6, respectivement, par exemple où :
(i) les amorces de PCR comprennent :
Amorce sens : 5'-ACAAACTCCCCGATGATGTGAGTG-3' (SEQ ID NO: 35)
Amorce antisens : 5'-GAGGCTGAGAAAGGTTAAGCCA-3' (SEQ ID NO: 36) ; et/ou
g) tel que dépendant de la revendication 3, les amorces de PCR pour déterminer l'allèle de l'exon 3 de RHD sont des amorces sens et antisens ciblant des séquences localisées dans les introns 2 et 3, ou les introns 3 et 2, respectivement, par exemple où :
(i) les amorces de PCR comprennent :
Amorce sens : 5'-TCCTGGCTCTCCCTCTCT-3' (SEQ ID NO: 15) Amorce antigens 5'-GTTGTCTTTATTTTTCAAAACCCT-3' (SEQ ID NO: 16) ;
où la séquence codant pour *RHD* est telle que décrite dans SEQ ID NO: 1.

6. Procédé selon la revendication 5, dans lequel l'acide nucléique amplifié comprend un marqueur, par exemple où
a) le marqueur comprend un nucléotide biotinylé ; et/ou
b) le marqueur comprend un fragment fluorescent.

7. Procédé selon l'une quelconque des revendications précédentes, dans lequel l'échantillon comprend un acide nucléique, et le procédé consiste à amplifier l'acide nucléique ou une partie de celui-ci par PCR en utilisant des amorces, à fragmenter l'acide nucléique amplifié, et à marquer l'acide nucléique fragmenté avec des ddNTP biotinylés en utilisant une enzyme désoxynucléotidyl transférase terminale (TdT).

8. Procédé selon l'une quelconque des revendications précédentes, dans lequel la détermination de la présence, de l'absence ou d'un variant de SNP d'un marqueur consiste à mettre en contact un acide nucléique contenant chaque marqueur avec une ou plusieurs sondes, par exemple où :
a) tel que dépendant de la revendication 3, les sondes pour déterminer la présence ou l'absence de RHD/CE Hex03 ou de l'exon 3 de RHD sont en contact avec un SNP localisé à la fois dans RHD/CE Hex03 et l'exon 3 de RHD, où un variant de SNP est spécifique pour RHD/CE Hex03 et un autre variant de SNP est spécifique pour l'exon 3 de RHD, par exemple où (i) le SNP est à la position 410 de la séquence codante, localisée à la fois au sein de RHD/CE Hex03 et de l'exon 3 de RHD ; et/ou
(ii) les sondes comprennent :
(1) 5'-TTTTACAGACGCCTGCTACCATG-3', (SEQ ID NO: 5)
(2) 5'-CATGGTAGCAGGCGTCTGTAAAA-3', (SEQ ID NO: 6)
(3) 5'-TTTTACAGACGTCTGCTACCATG-3', (SEQ ID NO: 7) et
(4) 5'-CATGGTAGCAGACGTCTGTAAAA-3', (SEQ ID NO: 8) ; et/ou
b) les sondes pour déterminer l'absence ou un variant de SNP du SNP à : la position 602 de la séquence codant pour *RHD* localisée au sein de l'exon 4 (rs1053355), la position 667 de la séquence codant pour RHD localisée au sein de l'exon 5 (rs1053356), ou la position 819 de la séquence codant pour RHD localisée au sein de l'exon 6 comprennent :
(i) l'exon 4 de RHD :
(1) 5'-ATAAAGATCAGACAGCAACGATACC-3' (SEQ ID NO: 23)
(2) 5'-TAAAGATCAGACAGCAACGATAC-3' (SEQ ID NO: 24)
(3) 5'-ATAAAGATCAGAGAGCAACGATACC-3' (SEQ ID NO: 25)
(4) 5'-TAAAGATCAGAGAGCAACGATAC3-3' (SEQ ID NO: 26) ;
(ii) l'exon 5 de RHD :
(1) 5'-CTGGCCAAGTTTCAACTCTGC-3' (SEQ ID NO: 27)
(2) 5'-TGGCCAAGTTTCAACTCTG-3' (SEQ ID NO: 28)
(3) 5'-CTGGCCAAGTGTCAACTCTGC-3' (SEQ ID NO: 29)
(4) 5'-TGGCCAAGTGTCAACTCTG-3' (SEQ ID NO: 30)
(iii) l'exon 6 de RHD :
(1) 5'-GTGCACAGTGCGGTGTTGGCAGG-3' (SEQ ID NO: 31)
(2) 5'-TGCACAGTGCGGTGTTGGCAG-3' (SEQ ID NO: 32)
(3) 5'-GTGCACAGTGCAGTGTTGGCAGG-3' (SEQ ID NO: 33)
(4) 5'-TGCACAGTGCAGTGTTGGCAG-3' (SEQ ID NO: 34) ; et/ou
c) les sondes pour déterminer le variant de SNP du SNP à la position 1048 de la séquence codant pour *RHD* localisée au sein de l'exon 7 (rs41307826) comprennent :
(1) 5'-TGCTGGTGCTTGATACCGTCGGA-3' (SEQ ID NO: 37)
(2) 5'-GCTGGTGCTTGATACCGTCGG-3' (SEQ ID NO: 38)
(3) 5'-TGCTGGTGCTTCATACCGTCGGA-3' (SEQ ID NO: 39)
(4) 5'-GCTGGTGCTTCATACCGTCGG-3' (SEQ ID NO: 40) ;
où la séquence codant pour *RHD* est telle que décrite dans SEQ ID NO: 1.

9. Procédé selon la revendication 8, dans lequel
a) une ou plusieurs des sondes comprennent un marqueur, par exemple où le marqueur est un fragment fluorescent ; et/ou
b) une ou plusieurs des sondes sont attachées à un support solide ou sont conjuguées à une ou plusieurs particules.

10. Utilisation d'un jeu d'amorces dans un procédé pour différencier les variants de groupe sanguin *RHD*DIIIa-CE (4-7) -D ou* analogues à *RHD*DIIIa-CE (4-7) -D*, qui expriment l'antigène C^{+W} et sont dépourvus d'un antigène D, de *RHD*DIIIa, RHD*DIVa-2* et d'autres variants de groupe sanguin, en amplifiant un acide nucléique comprenant au moins les quatre marqueurs selon la revendication 1, où le jeu d'amorces comprend au moins trois paires d'amorces sélectionnées à partir du jeu d'amorces décrit dans :
(i) la revendication 5(a),
(ii) la revendication 5(b),
(iii) l'une quelconque des revendications 5(c), 5(d) ou 5 (e)
(iv) la revendication 5(f), et
(v) la revendication 5(g).

11. Utilisation selon la revendication 10, dans laquelle le jeu d'amorces comprend au moins trois paires d'amorces sélectionnées à partir du jeu d'amorces décrit dans :
(i) la revendication 5(a)(ii),
(ii) la revendication 5(b)(i),
(iii) l'une quelconque des revendications 5(c)(i), 5 (d) (i) ou 5 (e) (i),
(iv) la revendication 5(f)(i), et
(v) la revendication 5(g)(i).

12. Utilisation selon la revendication 10 ou la revendication 11, dans laquelle au moins 50 % des amorces dans le jeu sont les paires d'amorces définies dans la revendication 10 ou 11.

13. Utilisation d'un jeu de sondes dans un procédé pour différencier les variants de groupe sanguin *RHD*DIIIa-CE (4-7) -D ou* analogues à *RHD*DIIIa-CE (4-7)-D*, qui expriment l'antigène C^{+W} et sont dépourvus d'un antigène D, de *RHD*DIIIa, RHD*DIVa-2* et d'autres variants de groupe sanguin, en déterminant la présence, l'absence ou un variant de polymorphisme de nucléotide unique (SNP) d'au moins les quatre marqueurs selon la revendication 1, où le jeu de sondes comprend :
(i) les sondes de SEQ ID NO: 5, 6, 7 et 8 ;
(ii) les sondes de SEQ ID NO: 23, 24, 25 et 26 ;
(iii) les sondes de SEQ ID NO: 31, 32, 33 et 34
(iv) les sondes de SEQ ID NO: 27, 28, 29 et 30 ; et
(v) les sondes de SEQ ID NO: 37, 38, 39 et 40.

14. Utilisation selon la revendication 13, dans laquelle :
a) les sondes sont immobilisées sur un support solide ou sont conjuguées à une ou plusieurs particules, par exemple où le support solide comprend un ou plusieurs marqueurs attachés, par exemple où le marqueur est un fluorochrome ; et/ou
b) une ou plusieurs sondes comprennent un marqueur, par exemple où le marqueur est un fragment fluorescent.

15. Utilisation d'un kit pour établir le génotype d'un sujet, le kit comprenant un jeu d'amorces de PCR tel que défini dans l'une quelconque des revendications 10 à 12, et un jeu de sondes tel que défini dans l'une quelconque des revendications 13 à 14.
